# EUROPEAN PATENT APPLICATION

(11) **EP 4 620 954 A1**
(43) Date of publication of application: **24.09.2025**
(21) Application number: 23890884.2
(22) Date of filing: 17.11.2023
(51) Int. Cl.: C07D 401/12, C07D 231/38, A61K 31/395, A61K 31/454, A61K 31/4439, A61P 35/00

(54) **CDK2 INHIBITOR, PREPARATION METHOD THEREFOR, AND USE THEREOF**

(30) Priority: 17.11.2022 CN 202211443043; 17.01.2023 CN 202310055539; 30.05.2023 CN 202310624531
(71) Applicant: Shandong Luye Pharmaceutical Co., Ltd., Yantai, Shandong 264670 (CN)
(72) Inventor: TIAN, Jingwei, Yantai, Shandong 264670 (CN); YE, Liang, Yantai, Shandong 264670 (CN); YANG, Yifei, Yantai, Shandong 264670 (CN); WANG, Yunjie, Yantai, Shandong 264670 (CN); YU, Dawei, Yantai, Shandong 264670 (CN); ZHANG, Wenjing, Yantai, Shandong 264670 (CN); ZHANG, Jianzhao, Yantai, Shandong 264670 (CN); WANG, Wenyan, Yantai, Shandong 264670 (CN); MA, Mingxu, Yantai, Shandong 264670 (CN)
(74) Representative: karo IP
(86) International application number: PCT/CN2023/132228
(87) International publication number: WO 2024/104455

(57) **Abstract**

A CDK2 inhibitor and a preparation method therefor, and a pharmaceutical composition. The present invention further relates to a use of the compound or the pharmaceutical composition in preparation of drugs for preventing or treating related diseases mediated by CDK2, wherein the diseases comprise abnormal cell growth.

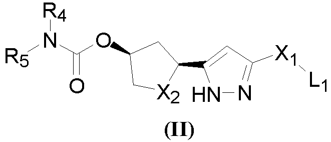

## Description

### TECHNICAL FIELD

The present disclosure relates to the technical field of pharmaceuticals, and specifically, to a CDK2 inhibitor compound and a method for preparing same, and use thereof in a medicament for a tumor-related disease mediated by CDK2.

### BACKGROUND

Since their discovery, tumors have remained the most significant threat to human health. From the discovery of the first anti-tumor drug, nitrogen mustard, in the 1940s to the discovery of the first tumor-targeting drug in the 1990s, the survival rates for patients with most types of tumors have been continuously increasing.

Cyclin-dependent kinases (CDKs), members of the serine/threonine protein kinase family, can regulate the cell cycle progression and gene transcription through phosphorylation, ensuring the orderly progression of various cell cycle phases. Currently, cyclin-dependent kinases are primarily divided into two categories: one is the cell cycle-related kinases, which regulate various stages of the cell cycle, including CDK1, CDK2, CDK3, CDK4, and CDK6; the other is the transcription-related kinases, which regulate the gene transcription processes, including CDK7, CDK8, CDK9, CDK11, CDK12, and CDK13. CDKs possess catalytic activity only after forming a heterodimer (CDK-cyclin) by binding to a regulatory subunit, i.e., a cyclin, and their activity is further regulated positively or negatively by cyclin and cyclin-dependent-kinase inhibitors (CKIs).

The loss of cell cycle regulation mechanism is one of the characteristics of tumorigenesis, and the functional abnormalities of CDKs are frequently observed in malignant tumors. Therefore, the treatment of malignant tumors by the inhibition of CDKs has always been a hot spot among anti-tumor therapies. Over the past decade or so, significant progress has been made in the research and development of CDK inhibitors. Selective dual inhibitors against CDK4/6 have demonstrated potent clinical activity and controllable toxicity. Currently, several CDK4/6 inhibitors have been successfully developed and approved for the treatment of breast cancer. They also exhibited good clinical efficacy and controllable safety in lung cancer, prostate cancer, and ovarian cancer. CDK2, along with CDK4/6, is a cell cycle-related kinase and is a critical core cell cycle regulator. It is active from the late G1 phase through the entire S phase and is one of the current hot spots in the research and development of CDKs.

Clinical studies have demonstrated that three CDK4/6 inhibitors palbociclib, ribociclib, and abemaciclib can significantly prolong the progression-free survival in patients. The CDK4/6 inhibitors have become the standard treatments for endocrine-resistant breast cancer. However, at present, the treatment of patients with resistance to CDK4/6 inhibitors has become a major challenge in the clinical treatment of breast cancer. Phase III clinical trials of palbociclib revealed that 70% to 80% of patients developed resistance within 12 to 36 months after the CDK4/6 inhibitor treatment. Investigations into the causes of resistance indicated that the amplification of CCNE1 or the downregulation of p27Kip1 upregulates the CDK2 activity. Subsequently, CDK2 can increase the phosphorylation of Rb, enabling the G1/S transition and promoting cell proliferation. Additionally, 10% to 20% of breast cancer patients exhibited insensitivity to the CDK4/6 inhibitors from the outset of the treatment, and studies on these patients have found significantly increased CCNE1 expression in them. Therefore, inhibiting CDK2 may potentially reverse the resistance to CDK4/6 inhibitor treatment.

The CCNE1 gene encodes cyclin E1 and is amplified in various tumors, with the amplification ranging from 0.4% to 40.4% (34.53% in breast cancer patients, 7.09% in breast cancer patients, 15.51% in gastric cancer patients, 17.33% in lung cancer patients, etc.). The cyclin encoded by this gene forms a complex with CDK2 and acts as its regulatory subunit, with its activity being essential for the G1/S transition during the cell cycle. This protein accumulates at the G1-S boundary and is degraded as cells enter the S phase. Studies have found that the CCNE1 amplification is more common in primary platinum-refractory epithelial ovarian cancer and is associated with the poor prognoses of ovarian cancer, gastric cancer, endometrial cancer, triple-negative breast cancer, etc. For patients with CCNE1 amplification, CDK2 inhibitors may be able to effectively inhibit tumor proliferation. Meanwhile, preclinical studies suggest that CDK2 inhibitors offer comparable efficacy and higher safety compared to CDK2/4/6 inhibitors. Individual CDK2 inhibitors may be used in combination with other CDK inhibitors, demonstrating greater flexibility and safety in clinical applications.

CDK1, which binds to cyclin A and cyclin B, controls the progression from the S phase to the G2 and M phases in the cell cycle. Its functionality cannot be replaced by other CDK family members, and inhibiting CDK1 kinase activity results in significant toxicity.

On the basis of the above factors, there is an urgent need to develop highly active and specific CDK2 inhibitors for the treatment of various tumors, which hold great market potential.

### SUMMARY

In one aspect, the present disclosure provides a compound of formula (I) or a pharmaceutically acceptable salt, a stereoisomer, or a deuteride thereof: 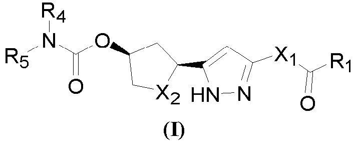 wherein,
X₁ is selected from the group consisting of NH or O;
X₂ is selected from the group consisting of CH₂ or O;
R₁ is selected from the group consisting of 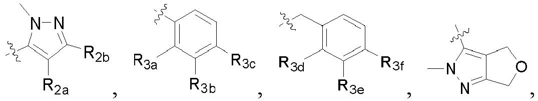 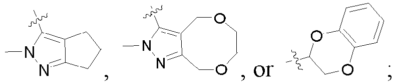
   R₂ₐ and R_{2b} are each independently selected from the group consisting of H or C₁₋₆ alkyl, wherein the C₁₋₆ alkyl is optionally substituted with C₁₋₆ alkoxy, R_{2c}-S(O)₂-, or R_{2c}-S(O)(NH)-, and R_{2c} is selected from the group consisting of H, C₁₋₆ alkyl, C₃₋₆ cycloalkyl, or amino;
   R₃ₐ, R_{3b}, R_{3c}, R₃ₐ, R₃ₑ, and R_{3f} are each independently selected from the group consisting of H, CH₃-S(O)₂-CH₂-, (CH₃)₂-P(O)-, or 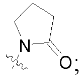
      R₄ and R₅ are independently selected from the group consisting of H, C₁₋₆ alkyl, C₂₋₆ alkenyl, C₂₋₆ alkynyl, C₁₋₆ fluoroalkyl, C₂₋₆ fluoroalkenyl, C₂₋₆ fluoroalkynyl, or C₃₋₁₀ cycloalkyl, wherein the terminal C atom of each C₁₋₆ alkyl is optionally substituted with cyano, and each C₃₋₁₀ cycloalkyl is optionally substituted with difluoromethylene;
      or R₄ and R₅, together with the N atom to which they are linked, form 4- to 6-membered heterocyclyl or 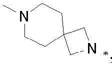 , wherein the 4- to 6-membered heterocyclyl is optionally substituted with 1-2 methyl.

In some embodiments of the compound of formula (I):
X₁ is selected from the group consisting of NH;
R₁ is selected from the group consisting of 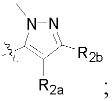
   R₂ₐ and R_{2b} are each independently selected from the group consisting of H or C₁₋₆ alkyl, wherein the C₁₋₆ alkyl is optionally substituted with C₁₋₆ alkoxy, R_{2c}-S(O)₂-, or R_{2c}-S(O)(NH)-, and R_{2c} is selected from the group consisting of H, C₁₋₆ alkyl, C₃₋₆ cycloalkyl, or amino;
   R₄ and R₅ are independently selected from the group consisting of H, C₁₋₆ alkyl, C₂₋₆ alkenyl, C₂₋₆ alkynyl, C₁₋₆ fluoroalkyl, C₂₋₆ fluoroalkenyl, C₂₋₆ fluoroalkynyl, or C₃₋₁₀ cycloalkyl, wherein the terminal C atom of each C₁₋₆ alkyl is optionally substituted with cyano, and each C₃₋₁₀ cycloalkyl is optionally substituted with difluoromethylene;
   or R₄ and R₅, together with the N atom to which they are linked, form 4- to 6-membered heterocyclyl or 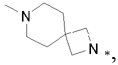 wherein the 4- to 6-membered heterocyclyl is optionally substituted with 1-2 methyl.

In some embodiments of the compound of formula (I):
X₁ is selected from the group consisting of NH;
R₁ is selected from the group consisting of 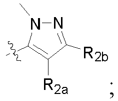
R₂ₐ and R_{2b} are each independently selected from the group consisting of H or methyl, wherein the methyl is optionally substituted with methoxy, ethoxy, n-propoxy, isopropoxy, CH₃-S(O)₂-, CH₃CH₂-S(O)₂-, cyclopropyl-S(O)₂-, NH₂-S(O)₂-, or CH₃-S(O)(NH)-;
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, n-but-1-yn-4-yl, cyclohexyl, difluoromethylenecyclohexyl, or adamantyl;
   or R₄ and R₅, together with the N atom to which they are linked, form azetidinyl, 2,2-dimethylazetidinyl, or

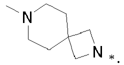

In some embodiments of the compound of formula (I):
X₁ is selected from the group consisting of NH;
R₁ is selected from the group consisting of 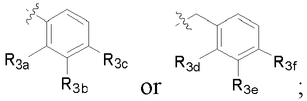
R₃ₐ, R_{3b}, R_{3c}, R₃ₐ, R₃ₑ, and R_{3f} are each independently selected from the group consisting of H, CH₃-S(O)₂-CH₂-, (CH₃)₂-P(O)-, or 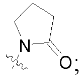
R₄ and R₅ are independently selected from the group consisting of H, C₁₋₆ alkyl, C₂₋₆ alkenyl, C₂₋₆ alkynyl, C₁₋₆ fluoroalkyl, C₂₋₆ fluoroalkenyl, C₂₋₆ fluoroalkynyl, or C₃₋₁₀ cycloalkyl, wherein the terminal C atom of each C₁₋₆ alkyl is optionally substituted with cyano, and each C₃₋₁₀ cycloalkyl is optionally substituted with difluoromethylene;
or R₄ and R₅, together with the N atom to which they are linked, form 4- to 6-membered heterocyclyl or 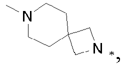 wherein the 4- to 6-membered heterocyclyl is optionally substituted with 1-2 methyl.

In some embodiments of the compound of formula (I):
X₁ is selected from the group consisting of NH;
R₁ is selected from the group consisting of 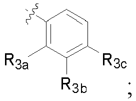
   R₃ₐ and R_{3c} are each independently selected from the group consisting of H, and R_{3b} is independently selected from the group consisting of CH₃-S(O)₂-CH₂-;
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, n-but-1-yn-4-yl, cyclohexyl, difluoromethylenecyclohexyl, or adamantyl;
   or R₄ and R₅, together with the N atom to which they are linked, form azetidinyl, 2,2-dimethylazetidinyl, or

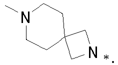

In some embodiments of the compound of formula (I):
X₁ is selected from the group consisting of NH;
R₁ is selected from the group consisting of 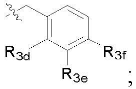 when R_{3d} and R_{3f} are each independently selected from the group consisting of H, R₃. is independently selected from the group consisting of CH₃-S(O)₂-CH₂- or (CH₃)₂-P(O)-; or when R_{3d} and R₃ₑ are each independently selected from the group consisting of H, R_{3f} is independently selected from the group consisting of 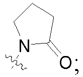
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, n-but-1-yn-4-yl, cyclohexyl, difluoromethylenecyclohexyl, or adamantyl;
   or R₄ and R₅, together with the N atom to which they are linked, form azetidinyl, 2,2-dimethylazetidinyl, or

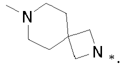

In some embodiments of the compound of formula (I):
X₁ is selected from the group consisting of NH;
R₁ is selected from the group consisting of 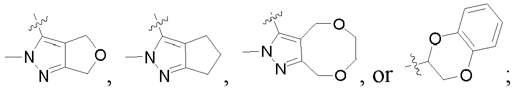
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, n-but-1-yn-4-yl, cyclohexyl, difluoromethylenecyclohexyl, or adamantyl;
   or R₄ and R₅, together with the N atom to which they are linked, form azetidinyl, 2,2-dimethylazetidinyl, or

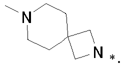

In another aspect, the present disclosure provides a compound of formula (II) or a pharmaceutically acceptable salt, a stereoisomer, or a deuteride thereof: 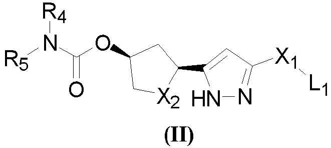 wherein,
X₁ is selected from the group consisting of NH or O;
X₂ is selected from the group consisting of CH₂ or O;
L₁ is selected from the group consisting of 6- to 10-membered aryl, 6- to 10-membered heteroaryl, or 6- to 10-membered heterocyclyl, wherein the 6- to 10-membered aryl, 6- to 10-membered heteroaryl, or 6- to 10-membered heterocyclyl is optionally substituted with 1, 2, or 3 R₆;
R₄ and R₅ are independently selected from the group consisting of H, C₁₋₆ alkyl, cyano-substituted C₁₋₆ alkyl, C₂₋₆ alkenyl, C₂₋₆ fluoroalkenyl, or C₂₋₆ alkynyl;
or R₄ and R₅, together with the N atom to which they are linked, form 4- to 6-membered heterocyclyl or 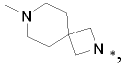 wherein the 4- to 6-membered heterocyclyl is optionally substituted with 1-2 methyl; each R₆ is independently selected from the group consisting of H, halogen, C₁₋₆ alkyl, (CH₃)₂-P(O)-, CH₃-S(O)₂-, -CH₂R₇, -NHR₇, or 6- to 10-membered heterocyclyl, wherein the C₁₋₆ alkyl is optionally substituted with C₁₋₆ alkoxy, and the 6- to 10-membered heterocyclyl is optionally substituted with 1, 2, 3, 4, or 5 halogens, C₁₋₆ alkyl, or C₁₋₆ haloalkyl;
R₇ is independently selected from the group consisting of 6- to 10-membered heterocyclyl, wherein the 6- to 10-membered heterocyclyl is optionally substituted with 1, 2, 3, 4, or 5 halogens, C₁₋₆ alkyl, or C₁₋₆ haloalkyl;
the "N atom marked with *" refers to the N atom linked to R₄ and R₅ in the general formula (II).

In some embodiments of the compound of formula (II):
X₁ is selected from the group consisting of NH;
L₁ is selected from the group consisting of phenyl, pyridinyl, pyrazolo[1,5-a]pyrazinyl, 2,3-dihydropyrrolo[3,4-c]pyridin-1-onyl, or piperidinyl, wherein the phenyl, pyridinyl, pyrazolo[1,5-a]pyrazinyl, 2,3-dihydropyrrolo[3,4-c]pyridin-1-onyl, or piperidinyl is optionally substituted with 1, 2, or 3 R₆;
R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl;
or R₄ and R₅, together with the N atom to which they are linked, form azetidinyl, 2,2-dimethylazetidinyl, or 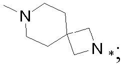
each R₆ is independently selected from the group consisting of H, F, Cl, Br, I, methyl, ethyl, n-propyl, isopropyl, n-butyl, isobutyl, tert-butyl, (CH₃)₂-P(O)-, CH₃-S(O)₂-, -CH₂R₇, -NHR₇, piperidinyl, or piperazinyl, wherein the methyl, ethyl, n-propyl, isopropyl, n-butyl, isobutyl, or tert-butyl is optionally substituted with methoxy, ethoxy, n-propoxy, or isopropoxy; the piperidinyl or piperazinyl is optionally substituted with 1, 2, 3, 4, or 5 F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl;
R₇ is independently selected from the group consisting of piperidinyl or piperazinyl, wherein the piperidinyl or piperazinyl is optionally substituted with 1, 2, 3, 4, or 5 F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

In some embodiments of the compound of formula (II):
X₁ is selected from the group consisting of NH or O;
X₂ is selected from the group consisting of CH₂ or O;
L₁ is selected from the group consisting of 6- to 10-membered aryl, 6- to 10-membered heteroaryl, or 6- to 10-membered heterocyclyl, wherein the 6- to 10-membered aryl, 6- to 10-membered heteroaryl, or 6- to 10-membered heterocyclyl is optionally substituted with 1, 2, or 3 R₆;
R₄ and R₅ are independently selected from the group consisting of H, C₁₋₆ alkyl, C₂₋₆ alkenyl, C₂₋₆ alkynyl, or C₂₋₆ fluoroalkenyl;
or R₄ and R₅, together with the N atom to which they are linked, form 4- to 6-membered heterocyclyl or 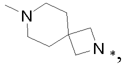 wherein the 4- to 6-membered heterocyclyl is optionally substituted with 1-2 methyl; each R₆ is independently selected from the group consisting of H, C₁₋₆ alkyl, (CH₃)₂-P(O)-, CH₃-S(O)₂-, or 6- to 10-membered heterocyclyl, wherein the C₁₋₆ alkyl is optionally substituted with C₁₋₆ alkoxy or 6- to 10-membered heterocyclyl substituted with C₁₋₆ alkyl.

In some embodiments of the compound of formula (II):
X₁ is selected from the group consisting of NH;
L₁ is selected from the group consisting of phenyl, pyridinyl, pyrazolo[1,5-a]pyrazinyl, 2,3-dihydropyrrolo[3,4-c]pyridin-1-onyl, or piperidinyl, wherein the phenyl, pyridinyl, pyrazolo[1,5-a]pyrazinyl, 2,3-dihydropyrrolo[3,4-c]pyridin-1-onyl, or piperidinyl is optionally substituted with 1, 2, or 3 R₆;
R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl;
or R₄ and R₅, together with the N atom to which they are linked, form azetidinyl, 2,2-dimethylazetidinyl, or 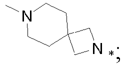
each R₆ is independently selected from the group consisting of H, methyl, ethyl, n-propyl, isopropyl, n-butyl, isobutyl, tert-butyl, (CH₃)₂-P(O)-, CH₃-S(O)₂-, piperidinyl, or piperazinyl, wherein the methyl, ethyl, n-propyl, isopropyl, n-butyl, isobutyl, or tert-butyl is optionally substituted with methoxy, ethoxy, n-propoxy, isopropoxy, piperidinyl, N-methylpiperidinyl, N-ethylpiperidinyl, piperazinyl, N-methylpiperazinyl, or N-ethylpiperazinyl.

In some embodiments of the compound of formula (II):
X₁ is selected from the group consisting of NH;
L₁ is selected from the group consisting of 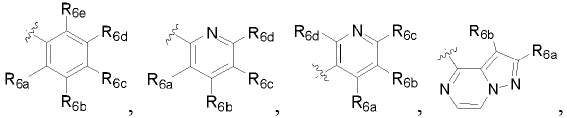 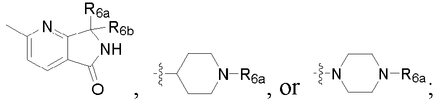
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl;
   or R₄ and R₅, together with the N atom to which they are linked, form azetidinyl, 2,2-dimethylazetidinyl, or 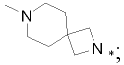 R₆ₐ, R_{6b}, R_{6c}, R_{6d}, and R₆ₑ are each independently selected from the group consisting of H, F, Cl, Br, I, methyl, ethyl, n-propyl, isopropyl, n-butyl, isobutyl, tert-butyl, (CH₃)₂-P(O)-, CH₃-S(O)₂-, -CH₂R₇, -NHR₇, piperidinyl, or piperazinyl, wherein the methyl, ethyl, n-propyl, isopropyl, n-butyl, isobutyl, or tert-butyl is optionally substituted with methoxy, ethoxy, n-propoxy, or isopropoxy; the piperidinyl or piperazinyl is optionally substituted with 1, 2, 3, 4, or 5 F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl;
   R₇ is independently selected from the group consisting of piperidinyl or piperazinyl, wherein the piperidinyl or piperazinyl is optionally substituted with 1, 2, 3, 4, or 5 F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

In some embodiments of the compound of formula (II):
X₁ is selected from the group consisting of NH;
L₁ is selected from the group consisting of 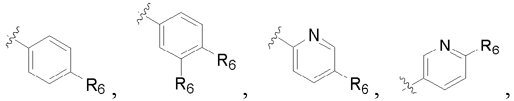 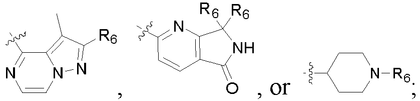
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl;
   or R₄ and R₅, together with the N atom to which they are linked, form azetidinyl, 2,2-dimethylazetidinyl, or 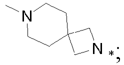
   each R₆ is independently selected from the group consisting of H, methyl, ethyl, n-propyl, isopropyl, n-butyl, isobutyl, tert-butyl, (CH₃)₂-P(O)-, CH₃-S(O)₂-, piperidinyl, or piperazinyl, wherein the methyl, ethyl, n-propyl, isopropyl, n-butyl, isobutyl, or tert-butyl is optionally substituted with methoxy, ethoxy, n-propoxy, isopropoxy, piperidinyl, N-methylpiperidinyl, N-ethylpiperidinyl, piperazinyl, N-methylpiperazinyl, or N-ethylpiperazinyl.

In some embodiments of the compound of formula (II):
X₁ is selected from the group consisting of NH;
L₁ is selected from the group consisting of 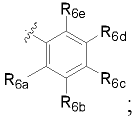
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of isopropyl;
   R₆ₐ, R_{6b}, R_{6c}, R_{6d}, and R₆ₑ are each independently selected from the group consisting of H, F, Cl, Br, I, methyl, ethyl, n-propyl, isopropyl, n-butyl, isobutyl, tert-butyl, (CH₃)₂-P(O)-, CH₃-S(O)₂-, -CH₂R₇, -NHR₇, 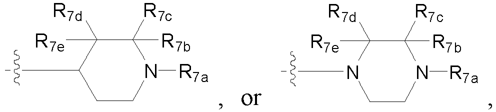 wherein the methyl, ethyl, n-propyl, isopropyl, n-butyl, isobutyl, or tert-butyl is optionally substituted with methoxy, ethoxy, n-propoxy, or isopropoxy;
   R₇ is independently selected from the group consisting of 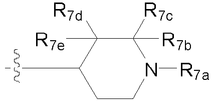 or 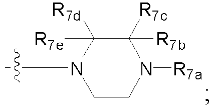
   R₇ₐ, R_{7b}, R_{7c}, R_{7d}, and R₇ₑ are independently selected from the group consisting of H, F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

In some embodiments of the compound of formula (II):
X₁ is selected from the group consisting of NH;
L₁ is selected from the group consisting of 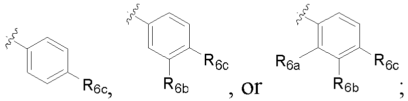
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of isopropyl;
   R₆ₐ and R_{6b} are each independently selected from the group consisting of H, F, Cl, Br, I, methyl, ethyl, n-propyl, isopropyl, n-butyl, isobutyl, or tert-butyl, wherein the methyl, ethyl, n-propyl, isopropyl, n-butyl, isobutyl, or tert-butyl is optionally substituted with methoxy, ethoxy, n-propoxy, or isopropoxy; R_{6c} is independently selected from the group consisting of (CH₃)₂-P(O)-, CH₃-S(O)₂-, -CH₂R₇, - NHR₇, 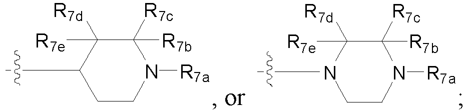
   R₇ is independently selected from the group consisting of 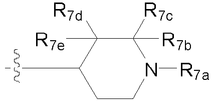 or 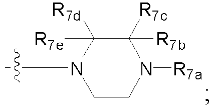
   R₇ₐ, R_{7b}, R_{7c}, R_{7d}, and R₇ₑ are independently selected from the group consisting of H, F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

In some embodiments of the compound of formula (II):
X₁ is selected from the group consisting of NH;
L₁ is selected from the group consisting of 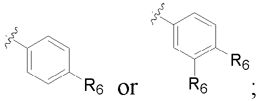
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of isopropyl;
   each R₆ is independently selected from the group consisting of (CH₃)₂-P(O)-, 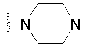 or

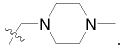

In some embodiments of the compound of formula (II):
X₁ is selected from the group consisting of NH;
L₁ is selected from the group consisting of 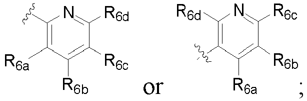
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of isopropyl;
   R₆ₐ, R_{6b}, R_{6c}, and R_{6d} are each independently selected from the group consisting of H, F, Cl, Br, I, methyl, ethyl, n-propyl, isopropyl, n-butyl, isobutyl, tert-butyl, (CH₃)₂-P(O)-, CH₃-S(O)₂-, -CH₂R₇, - NHR₇, 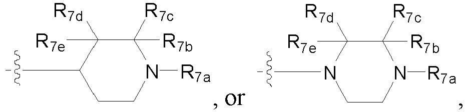 wherein the methyl, ethyl, n-propyl, isopropyl, n-butyl, isobutyl, or tert-butyl is optionally substituted with methoxy, ethoxy, n-propoxy, or isopropoxy;
   R₇ is independently selected from the group consisting of 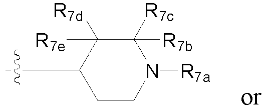 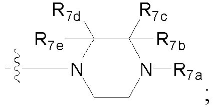
   R₇ₐ, R_{7b}, R_{7c}, R_{7d}, and R₇ₑ are independently selected from the group consisting of H, F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

In some embodiments of the compound of formula (II):
X₁ is selected from the group consisting of NH;
L₁ is selected from the group consisting of 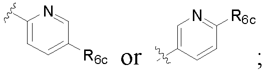
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of isopropyl;
   R_{6c} is independently selected from the group consisting of

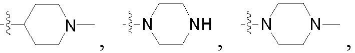

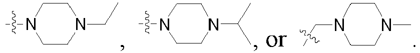

In some embodiments of the compound of formula (II):
X₁ is selected from the group consisting of NH;
L₁ is selected from the group consisting of 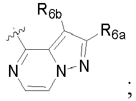
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of 3,3-difluoro-2-propenyl or n-but-1-yn-3-yl;
   R₆ₐ is selected from the group consisting of methoxymethyl;
   R_{6b} is selected from the group consisting of methyl.

In some embodiments of the compound of formula (II):
X₁ is selected from the group consisting of NH;
L₁ is selected from the group consisting of 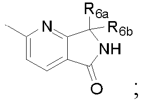
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of 3,3-difluoro-2-propenyl or 2-propynyl;
   R₆ₐ and R_{6b} are each independently selected from the group consisting of methyl.

In some embodiments of the compound of formula (II):
X₁ is selected from the group consisting of NH;
L₁ is selected from the group consisting of 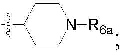
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl;
   R₆ₐ is independently selected from the group consisting of CH₃-S(O)₂-.

In some embodiments of the compound of formula (II):
X₁ is selected from the group consisting of NH;
L₁ is selected from the group consisting of 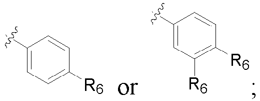
   R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of isopropyl;
   each R₆ is independently selected from the group consisting of (CH₃)₂-P(O)-, 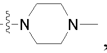 or

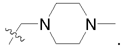

In another aspect, the present disclosure provides a compound of formula (IIA) or a pharmaceutically acceptable salt, a stereoisomer, or a deuteride thereof: 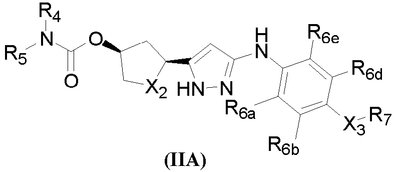 wherein,
X₂ is selected from the group consisting of CH₂ or O;
X₃ is selected from the group consisting of a bond, CH₂, NH, or O;
R₄ and R₅ are independently selected from the group consisting of H, C₁₋₆ alkyl, cyano-substituted C₁₋₆ alkyl, C₂₋₆ alkenyl, C₂₋₆ fluoroalkenyl, or C₂₋₆ alkynyl;
or R₄ and R₅, together with the N atom to which they are linked, form 4- to 6-membered heterocyclyl or 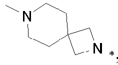 , wherein the 4- to 6-membered heterocyclyl is optionally substituted with 1-2 methyl;
   R₆ₐ, R_{6b}, R_{6d}, and R₆ₑ are each independently selected from the group consisting of H, halogen, C₁₋₆ alkyl, (CH₃)₂-P(O)-, or CH₃-S(O)₂-, wherein the C₁₋₆ alkyl is optionally substituted with C₁₋₆ alkoxy; R₇ is independently selected from the group consisting of 6- to 10-membered heterocyclyl, wherein the 6- to 10-membered heterocyclyl is optionally substituted with 1, 2, 3, 4, or 5 halogens, C₁₋₆ alkyl, or C₁₋₆ haloalkyl;
   the "N atom marked with *" refers to the N atom linked to R₄ and R₅ in the general formula (II).

In some embodiments of the compound of formula (IIA):
R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl;
or R₄ and R₅, together with the N atom to which they are linked, form azetidinyl, 2,2-dimethylazetidinyl, or

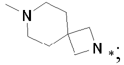

In some embodiments of the compound of formula (IIA):
R₆ₐ, R_{6b}, R_{6d}, and R₆ₑ are each independently selected from the group consisting of H, F, Cl, Br, I, methyl, ethyl, n-propyl, isopropyl, n-butyl, isobutyl, tert-butyl, (CH₃)₂-P(O)-, or CH₃-S(O)₂-, wherein the methyl, ethyl, n-propyl, isopropyl, n-butyl, isobutyl, or tert-butyl is optionally substituted with methoxy, ethoxy, n-propoxy, or isopropoxy.

In some embodiments of the compound of formula (IIA):
R₇ is independently selected from the group consisting of piperidinyl or piperazinyl, wherein the piperidinyl or piperazinyl is optionally substituted with 1, 2, 3, 4, or 5 F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

In some embodiments of the compound of formula (IIA):
X₂ is selected from the group consisting of CH₂ or O;
X₃ is selected from the group consisting of a bond, CH₂, NH, or O;
R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl; R₄ is preferably selected from the group consisting of H, and R₅ is preferably selected from the group consisting of isopropyl;
R₆ₐ, R_{6b}, R_{6d}, and R₆ₑ are each independently selected from the group consisting of H, F, Cl, Br, I, methyl, ethyl, n-propyl, isopropyl, n-butyl, isobutyl, tert-butyl, (CH₃)₂-P(O)-, or CH₃-S(O)₂-, wherein the methyl, ethyl, n-propyl, isopropyl, n-butyl, isobutyl, or tert-butyl is optionally substituted with methoxy, ethoxy, n-propoxy, or isopropoxy;
R₇ is independently selected from the group consisting of 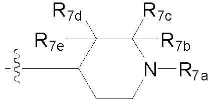 or 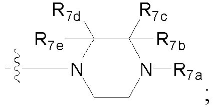
R₇ₐ, R_{7b}, R_{7c}, R_{7d}, and R₇ₑ are independently selected from the group consisting of H, F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

In some embodiments of the compound of formula (IIA):
X₂ is selected from the group consisting of CH₂; X₃ is selected from the group consisting of CH₂;
R₄ is selected from the group consisting of H; R₅ is selected from the group consisting of C₁₋₆ alkyl, preferably isopropyl;
R₆ₐ, R_{6b}, R_{6d}, and R₆ₑ are each independently selected from the group consisting of H or halogen;
R₇ is independently selected from the group consisting of 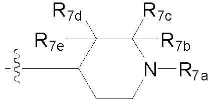 or 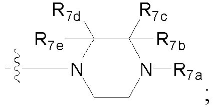
R₇ₐ, R_{7b}, R_{7c}, R_{7d}, and R₇ₑ are independently selected from the group consisting of H, F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

In another aspect, the present disclosure provides a compound as shown below or a pharmaceutically acceptable salt, a stereoisomer, or a deuteride thereof, wherein the compound is selected from the group consisting of:

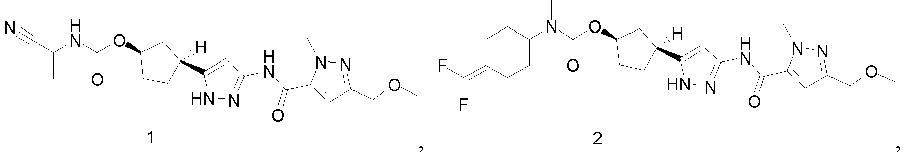

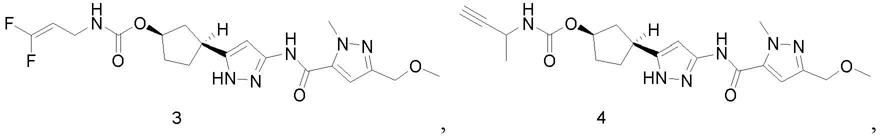

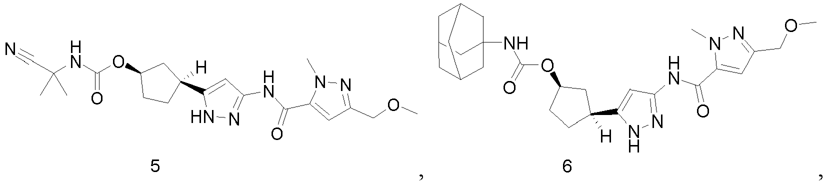

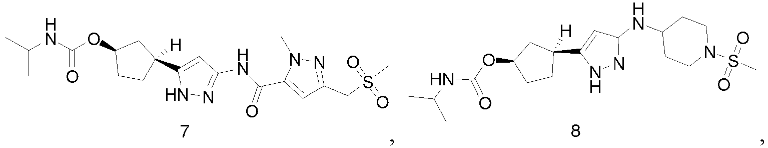

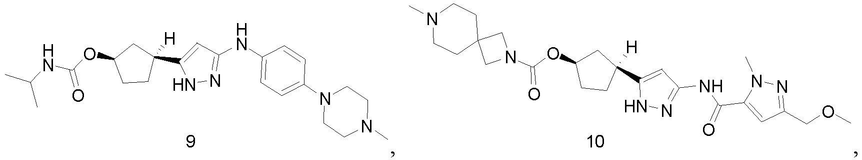

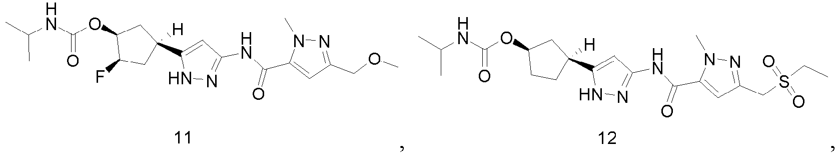

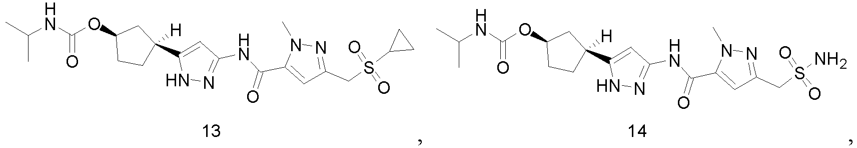

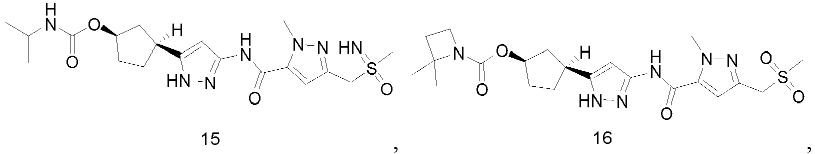

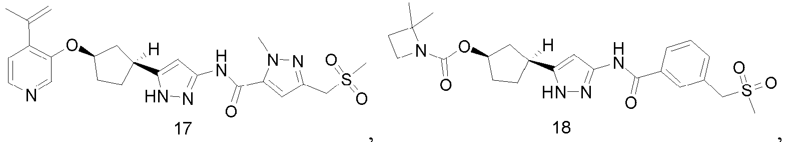

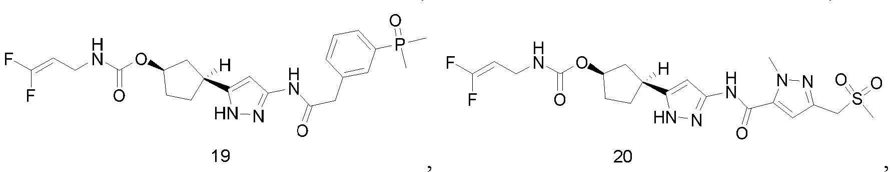

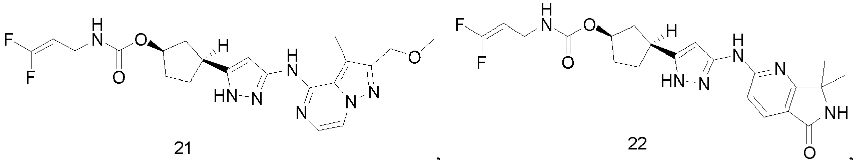

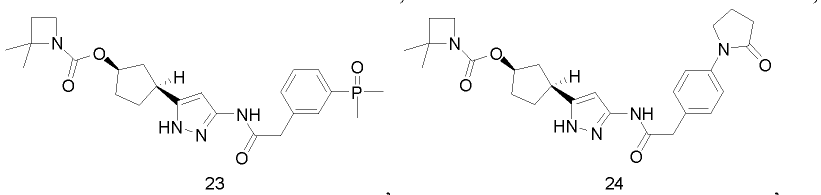

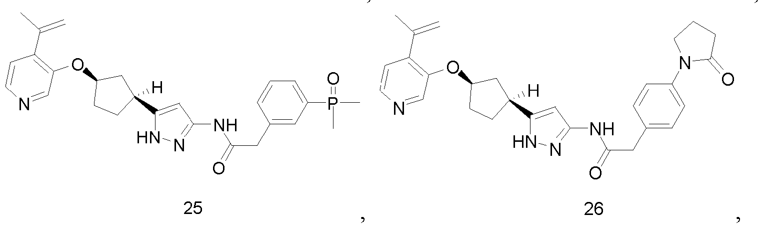

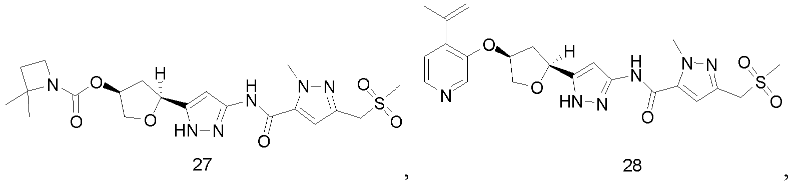

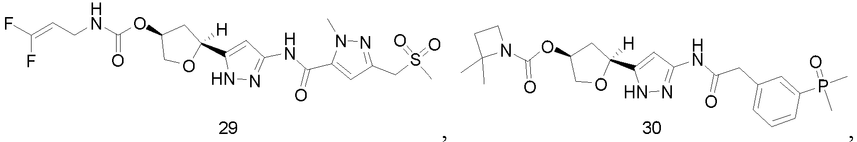

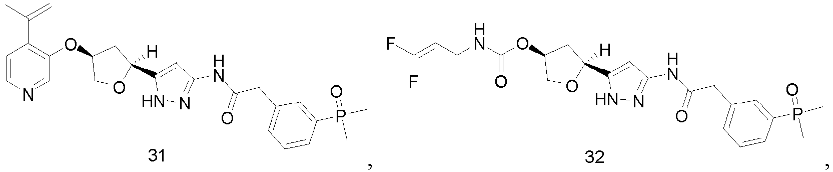

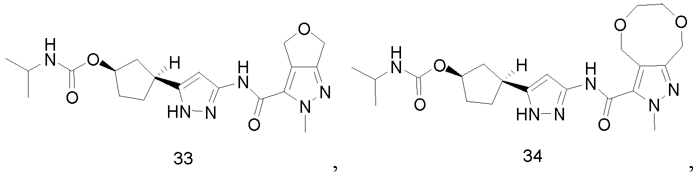

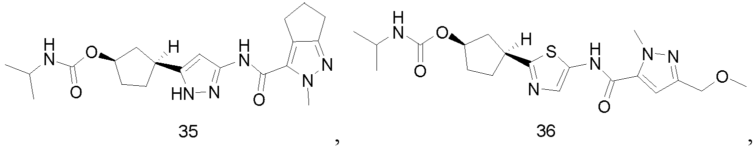

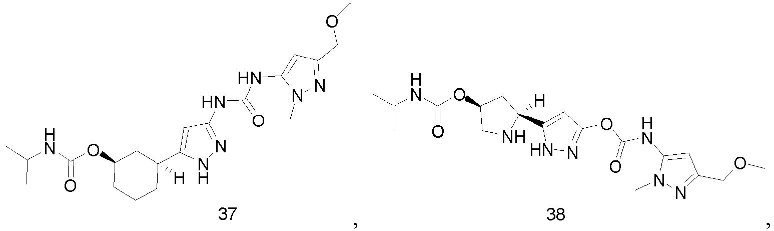

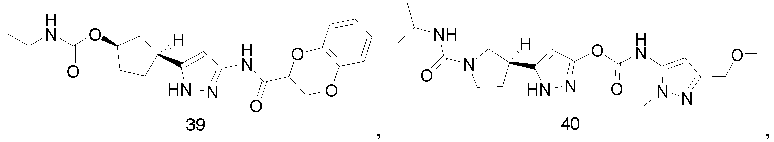

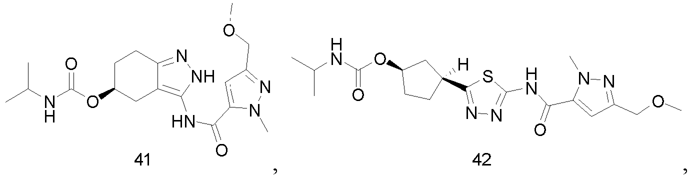

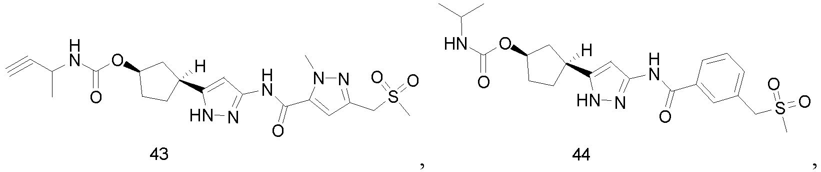

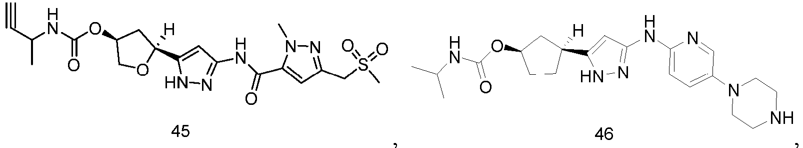

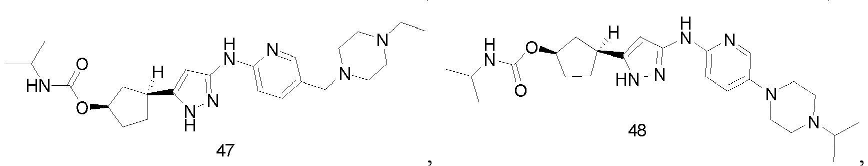

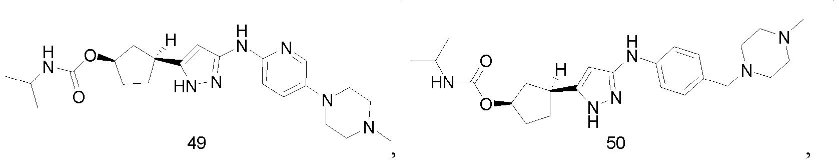

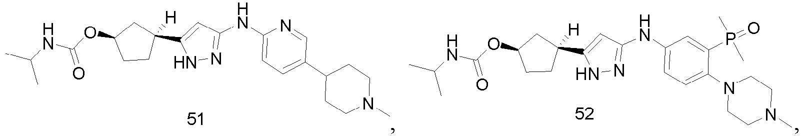

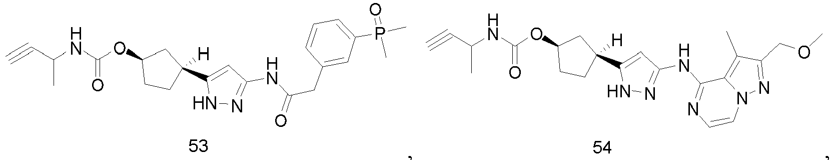

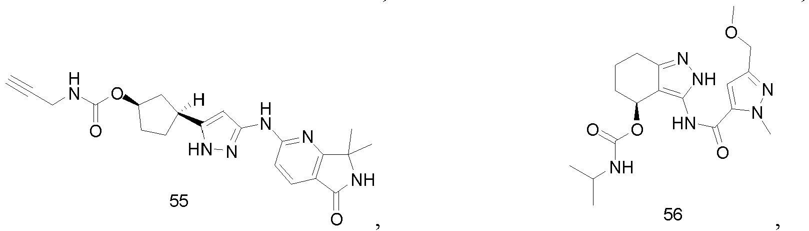

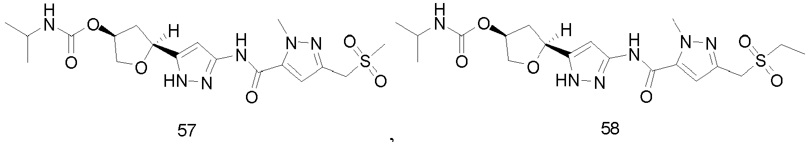

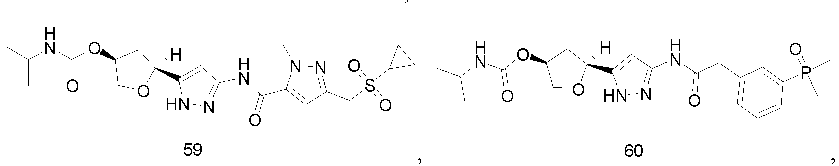

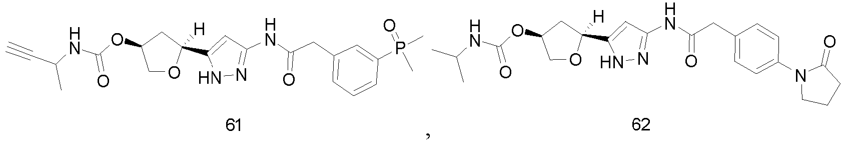

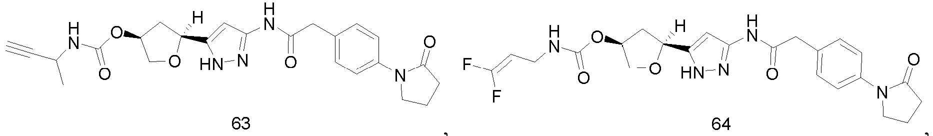

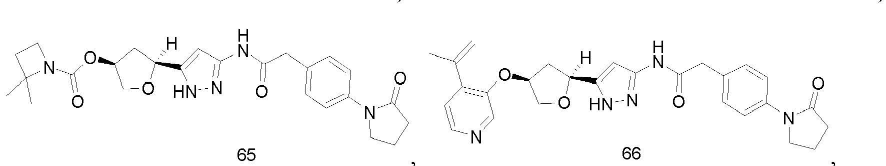

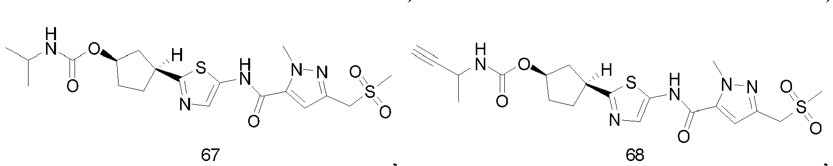

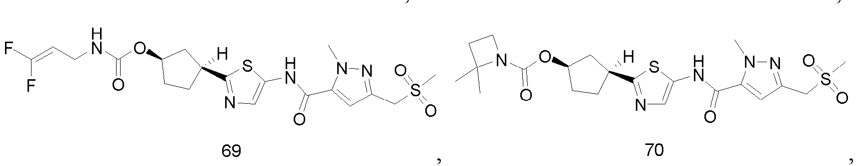

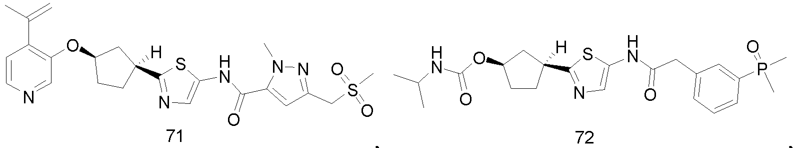

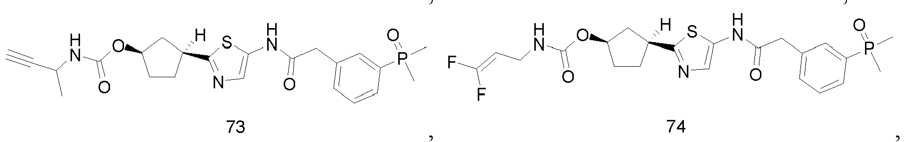

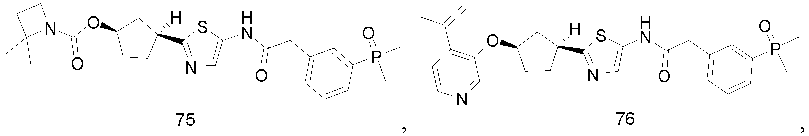

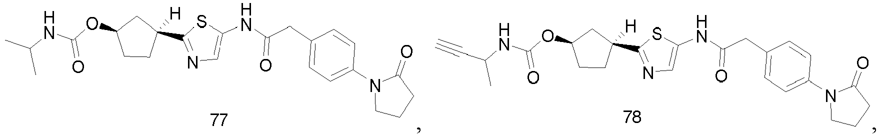

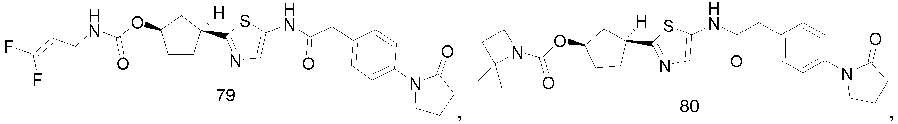

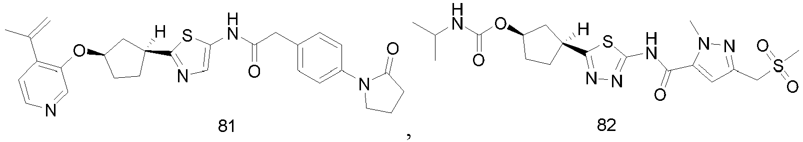

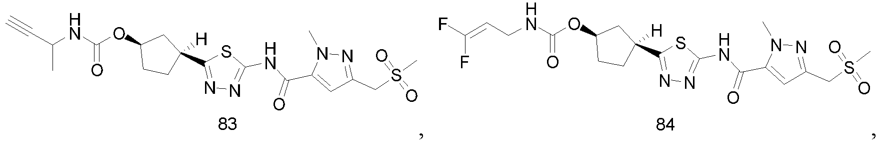

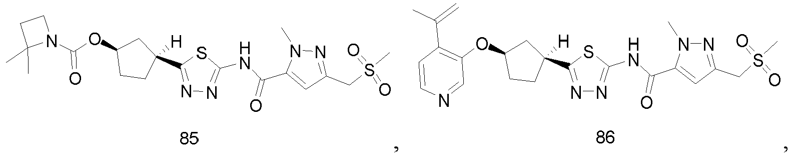

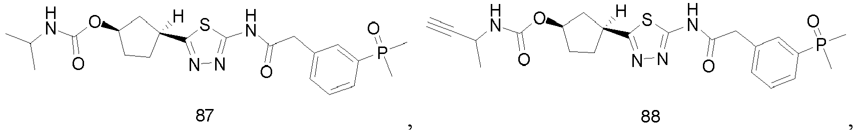

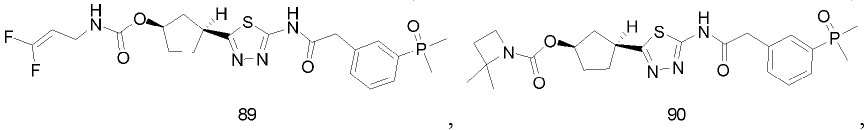

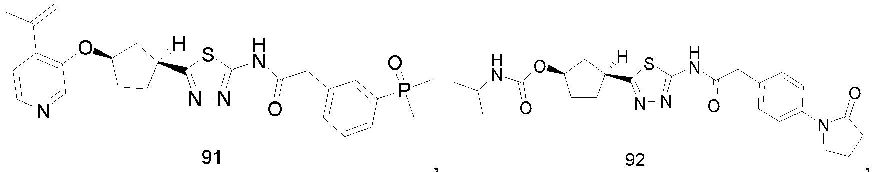

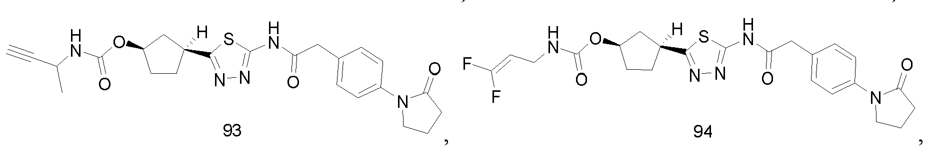

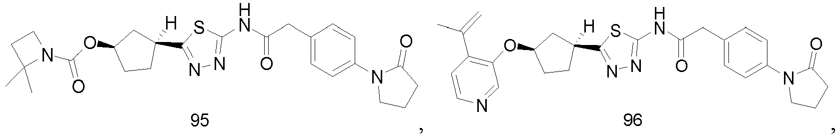

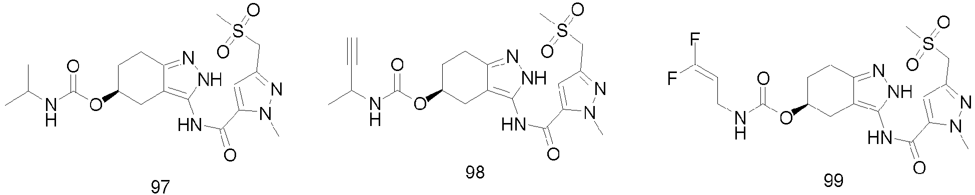

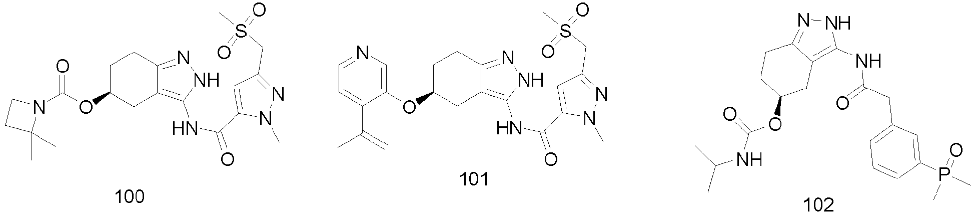

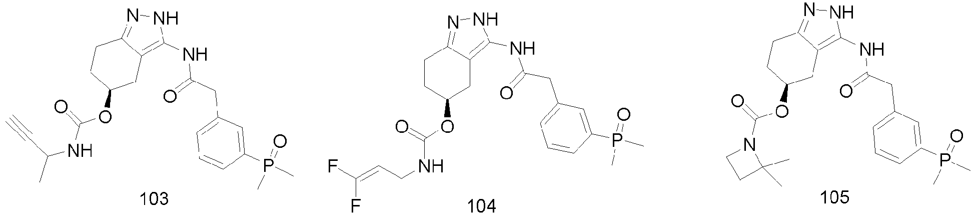

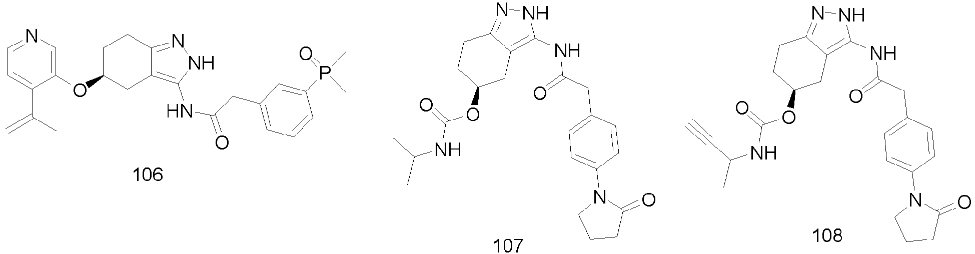

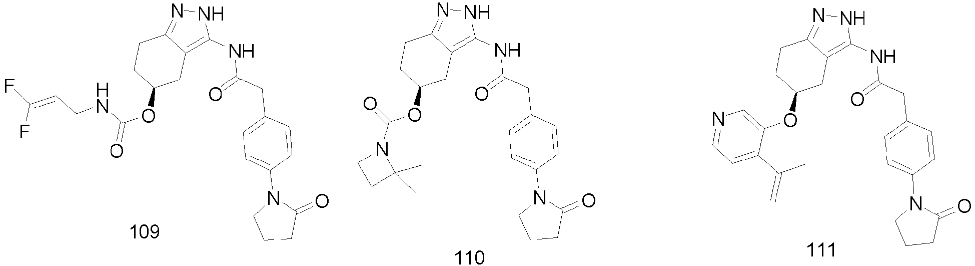

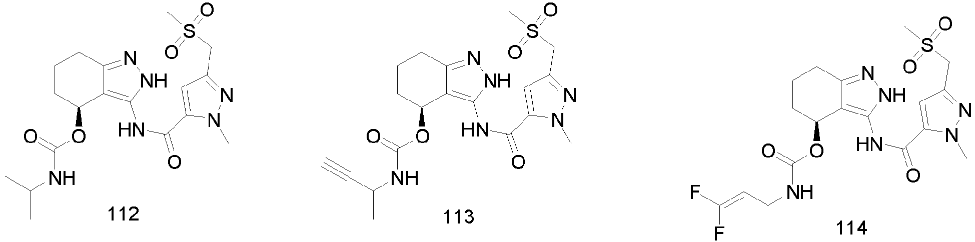

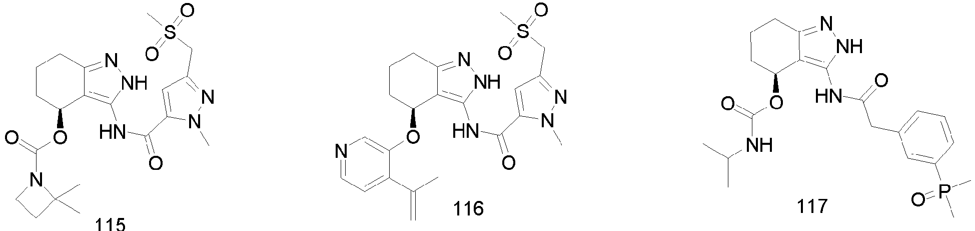

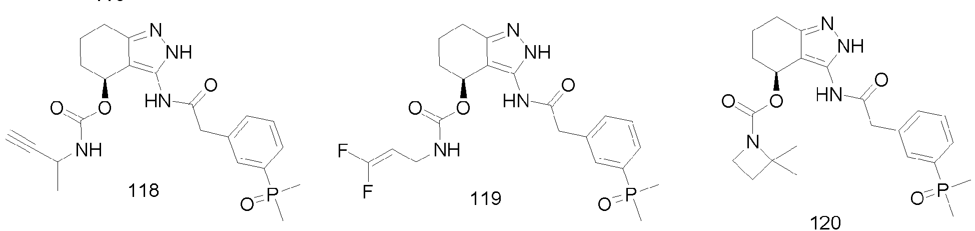

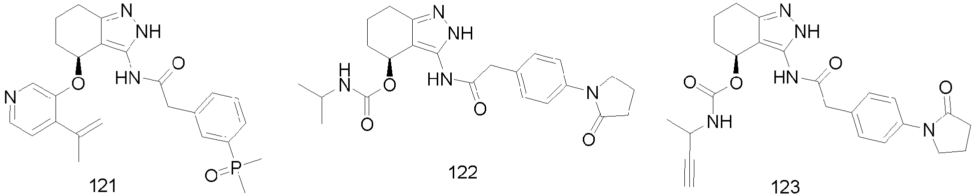

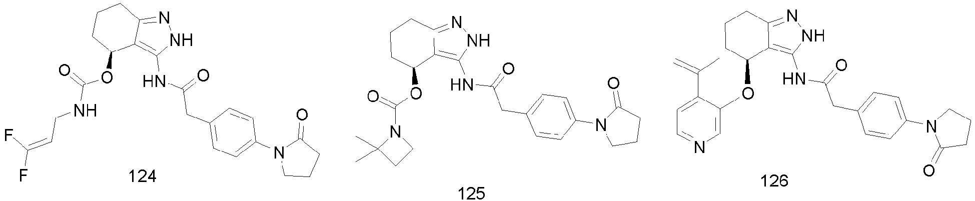

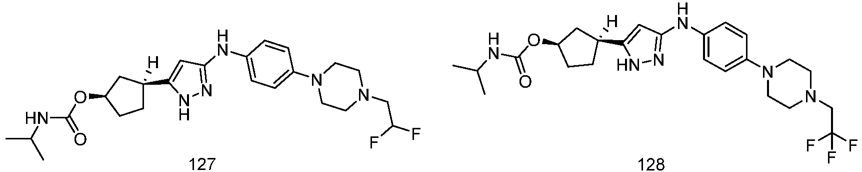

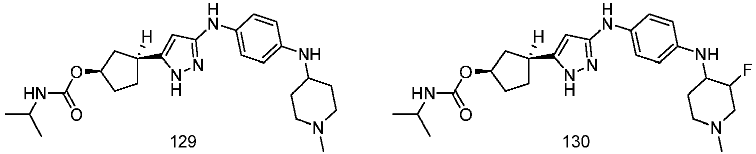

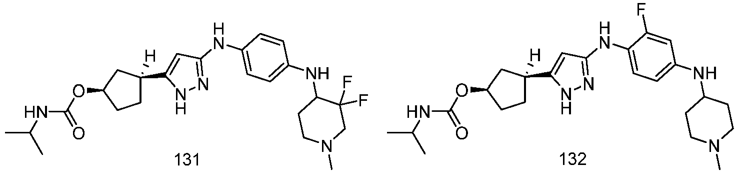

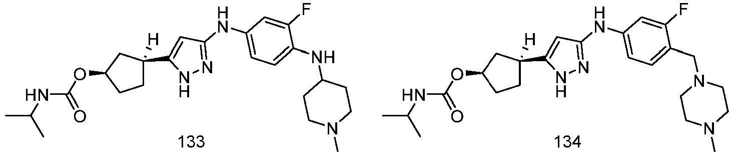

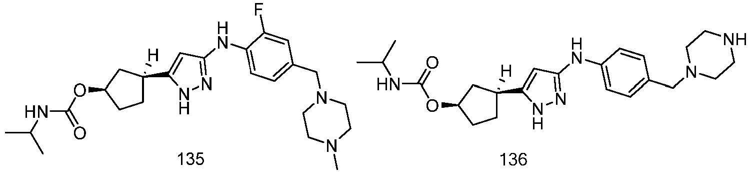

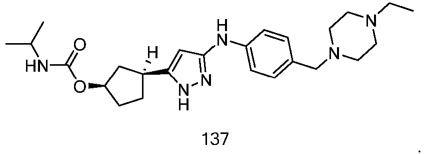

The present disclosure further provides a pharmaceutical composition comprising a therapeutically effective amount of the compound or the pharmaceutically acceptable salt, the stereoisomer, or the deuteride thereof according to any one of the above and a pharmaceutically acceptable carrier. The carrier includes conventional auxiliary ingredients in the art, such as fillers, binders, diluents, disintegrants, lubricants, colorants, flavoring agents, antioxidants, or wetting agents.

The pharmaceutical composition may be prepared into various pharmaceutically acceptable dosage forms, such as tablets, capsules, oral liquids, suspensions, granules, powders, microgranules, pills, mini-tablets, fast-dissolving films, nasal sprays, transdermal patches, injections, or various sustained-release and controlled-release preparations. The pharmaceutical composition may be administered via oral, transmucosal, rectal, or parenteral routes (including intravascular, intravenous, intraperitoneal, subcutaneous, intramuscular, and intrasternal administration). The dose may be appropriately adjusted depending on the age, sex, and disease of the patient.

For oral administration, the pharmaceutical composition may be in the form of, for example, a tablet, a capsule, a liquid capsule, a suspension, or a liquid. The pharmaceutical composition is preferably prepared in a dosage unit form containing a specific amount of the active ingredient. For example, the pharmaceutical composition may be provided in a tablet or capsule containing the active ingredient in an amount ranging from about 0.1 to 1000 mg, preferably from about 0.25 to 250 mg, and more preferably from about 0.5 to 100 mg. Suitable daily doses for humans or other mammals may vary widely depending on the condition of the patient and other factors, but can be determined using conventional methods.

The present disclosure further provides a method/use of the compound, the pharmaceutically acceptable salt thereof, the stereoisomer thereof, or the pharmaceutical composition thereof as a medicament, and specifically, a method and use of the compound, the pharmaceutically acceptable salt thereof, the stereoisomer thereof, or the pharmaceutical composition thereof for preventing, treating, or ameliorating pathology and/or a symptom of a disease in an animal or a human.

In certain aspects and embodiments of the compound, the pharmaceutically acceptable salt thereof, the stereoisomer thereof, or the pharmaceutical composition thereof, and the method and use thereof described herein, the compound, the pharmaceutically acceptable salt thereof, the stereoisomer thereof, or the pharmaceutical composition thereof of the present disclosure exhibit higher selectivity for CDK2 than for other CDKs, particularly CDK1.

In one aspect, the present disclosure provides use/method of the compound, the pharmaceutically acceptable salt thereof, the stereoisomer thereof, or the pharmaceutical composition thereof according to any one of the above for preventing/treating a related disease mediated by CDK2. The related disease mediated by CDK2 comprises abnormal cell growth in a subject, wherein the abnormal cell growth is cancer. In some certain embodiments, the cancer is selected from the group consisting of breast cancer, triple-negative breast cancer (TNBC), ovarian cancer, bladder cancer, uterine cancer, cervical cancer, prostate cancer, lung cancer (including NSCLC, SCLC, squamous-cell carcinoma, or adenocarcinoma), esophageal cancer, head and neck cancer, colorectal cancer, renal cancer (including RCC), liver cancer (including HCC), pancreatic cancer, gastric cancer, thyroid cancer, skin cancer, lymphoma, sarcoma, multiple myeloma, or a solid tumor.

### Definitions and Description

Unless otherwise specified, the following terms and phrases used herein are intended to have the following meanings. A particular term or phrase, unless otherwise specifically defined, should not be considered indefinite or unclear, but construed according to its common meaning. When referring to a trade name, it is intended to refer to its corresponding commercial product or its active ingredient. The term "pharmaceutically acceptable" is used herein for those compounds, materials, compositions, and/or dosage forms that are, within the scope of sound medical judgment, suitable for use in contact with the tissues of humans and animals without excessive toxicity, irritation, allergic response, or other problems or complications, and commensurate with a reasonable benefit/risk ratio.

The term "pharmaceutically acceptable salt" refers to a salt of the compound of the present disclosure, which is prepared from the compound having particular substituents disclosed herein and a relatively nontoxic acid or base. When the compound of the present disclosure contains a relatively acidic functional group, a base addition salt can be obtained by contacting the neutral form of such a compound with a sufficient amount of a base in a pure solution or a suitable inert solvent. Examples of the pharmaceutically acceptable acid addition salt include inorganic acid salts and organic acid salts, and also include salts of amino acids (e.g., arginine) and salts of organic acids (e.g., glucuronic acid). Certain specific compounds of the present disclosure contain both basic and acidic functional groups that allow the compounds to be converted into either base or acid addition salt.

The pharmaceutically acceptable salt of the present disclosure can be synthesized from the parent compound containing an acid radical or a basic group by conventional chemical methods. In general, the salt is prepared by reacting the free acid or base form of the compound with a stoichiometric amount of an appropriate base or acid in water or an organic solvent or a mixture thereof.

Certain compounds of the present disclosure may have asymmetric carbon atoms (optical centers) or double bonds. Racemates, diastereoisomers, geometric isomers, and individual isomers are all included within the scope of the present disclosure.

The compounds of the present disclosure may be in the form of a particular geometric isomer or stereoisomer. All such compounds are contemplated herein, including cis and trans isomers, (-)- and (+)-enantiomers, (*R*)- and (*S*)-enantiomers, diastereoisomers, (*D*)-isomers, (*L*)-isomers, and racemic mixtures and other mixtures thereof, such as enantiomerically or diastereoisomerically enriched mixtures, all of which fall within the scope of the present disclosure. Additional asymmetric carbon atoms may be present in substituents such as alkyl. All these isomers and mixtures thereof are encompassed within the scope of the present disclosure.

Optically active (*R*)- and (*S*)-isomers as well as *D*- and *L*-isomers can be prepared by chiral synthesis or chiral reagents or other conventional techniques. If one enantiomer of a certain compound of the present disclosure is desired, the desired pure enantiomer can be prepared by asymmetric synthesis or derivatization using a chiral auxiliary, where the resulting diastereoisomeric mixture is separated and the auxiliary group is cleaved. Alternatively, when the molecule contains a basic functional group (e.g., amino) or an acidic functional group (e.g., carboxyl), a diastereoisomeric salt is formed with an appropriate optically active acid or base, followed by diastereoisomeric resolution through conventional methods well known in the art, and the pure enantiomer is subsequently recovered. Furthermore, the separation of enantiomers and diastereoisomers is generally accomplished by chromatography using a chiral stationary phase, optionally in combination with chemical derivatization (e.g., carbamate formation from amines).

The term "pharmaceutically acceptable carrier" refers to any preparation or carrier medium capable of delivering an effective amount of the active substance of the present disclosure, without interfering with the biological activity of the active substance or causing adverse effects in the host or patient; representative carriers include, but are not limited to: binders, fillers, lubricants, disintegrants, wetting agents, dispersing agents, solubilizers, suspending agents, and the like.

For a drug or pharmacologically active agent, the term "effective amount" or "therapeutically effective amount" refers to an amount of the drug or medicament that is sufficient to achieve the desired effect but is non-toxic. For oral dosage forms of the present disclosure, an "effective amount" of one active substance in the composition refers to the amount required to achieve the desired effect when the active substance is combined with another active substance in the composition. The determination of the effective amount varies from person to person. It depends on the age and general condition of the recipient, as well as the specific active substance used. The appropriate effective amount in a case may be determined by those skilled in the art in the light of routine tests.

The present disclosure is intended to include all isotopes of atoms present in the compounds of the present disclosure. Isotopes include those atoms having the same atomic number but different mass numbers. As a general and non-limiting example, isotopes of hydrogen include deuterium and tritium. Isotopes of carbon include ¹³C and ¹⁴C. Isotopically labeled compounds of the present disclosure can generally be prepared by conventional techniques known to those skilled in the art or by methods similar to those described herein using an appropriate isotopically labeled reagent in place of the non-labeled reagent otherwise used.

The term "deuterated analog" refers to an analog resulting from replacement of one or more hydrogen atoms in a compound with a deuterium atom. The term "optional" or "optionally" means that the subsequently described event or circumstance may, but not necessarily, occur, and the description includes instances where the event or circumstance occurs and instances where it does not. For example, "optionally substituted with one or more deuterium atoms" means that the group may be unsubstituted or substituted with one or more deuterium atoms; that is, it includes instances where the group is not deuterated, partially deuterated, and/or fully deuterated.

The term "optional", "optionally", or "optionally substituted with..." means that the subsequently described event or circumstance may, but not necessarily, occur, and the description includes instances where the event or circumstance occurs and instances where it does not. For example, "optionally substituted with..." means that a substituent may or may not be present, and that one, two, three, or more substituents are included.

The term "substituted" means that any one or more hydrogen atoms on a specific atom are substituted with substituents that may include deuterium and hydrogen variants, as long as the valence of the specific atom is normal and the compound after substitution is stable. When the substituent is keto (i.e., =O), it means that two hydrogen atoms are substituted. Substitution with keto does not occur on aromatic groups.

Unless otherwise specified, the term "alkyl" is used to refer to a linear or branched saturated hydrocarbon group, which may be monovalent (e.g., methyl), divalent (e.g., methylene), or polyvalent (e.g., methine). For example, C₁-C₁₀ refers to 1 to 10 carbon atoms, where C₁₋₁₀ is selected from the group consisting of C₁, C₂, C₃, C₄, C₅, C₆, C₇, C₈, C₉, and C₁₀. Examples of the alkyl include, but are not limited to, methyl (Me), ethyl (Et), propyl (e.g., n-propyl and isopropyl), butyl (e.g., n-butyl, isobutyl, s-butyl, and t-butyl), pentyl (e.g., n-pentyl, isopentyl, neopentyl, and 1-ethylpropyl), hexyl (e.g., n-hexyl, isohexyl, 1,1-dimethylbutyl, 2,2-dimethylbutyl, 3,3-dimethylbutyl, and 2-ethylbutyl), heptyl, octyl, nonyl, decyl, and the like. It will be appreciated that the term "alkylene" refers to a residue derived from an "alkyl" group by the removal of one additional hydrogen atom. Examples of C₁-C₆ alkylene include, but are not limited to, -CH₂-, -(CH₂)₂-, -(CH₂)₃-, -(CH₂)₄-, -CH₂CH(CH₃)-CH₂, and the like. It should be noted that alkylene present at the terminal of a linear or branched chain or at the terminal of a substituent (e.g., methylene) includes the case of "CH₂=", where a hydrogen atom may be substituted with 1 or 2 halogen atoms (e.g., fluorine atoms).

Unless otherwise specified, the term "alkenyl" refers to a linear or branched hydrocarbon group consisting of carbon and hydrogen atoms. It has at least one carbon-carbon double bond that may be in the (E)- or (Z)-configuration, has 2 to 6 carbon atoms, and is linked to the rest of the molecule via a single bond. For example, C₂₋₆ refers to 2 to 6 carbon atoms, and C₂₋₆ is selected from the group consisting of C₂, C₃, C₄, C₅, and C₆. Examples of alkenyl include, but are not limited to, ethenyl, 1-propenyl, 2-propenyl (allyl), 1-methyl-ethenyl, 1-butenyl, 2-butenyl, 3-butenyl, 1-methyl-1-propenyl, 2-methyl-1-propenyl, 1-methyl-2-propenyl, and 2-methyl-2-propenyl.

Unless otherwise specified, the term "alkynyl" refers to a linear or branched hydrocarbon group consisting of carbon and hydrogen atoms. It has at least one carbon-carbon triple bond and 2 to 6 carbon atoms and is linked to the rest of the molecule via a single bond. For example, C₂₋₆ refers to 2 to 6 carbon atoms, and C₂₋₆ is selected from the group consisting of C₂, C₃, C₄, C₅, and C₆. In one embodiment of alkynyl, the number of triple bonds is 1. Examples of alkynyl include, but are not limited to, ethynyl, prop-1-yn-1-yl, prop-2-yn-1-yl, n-but-1-yn-1-yl, n-but-1-yn-3-yl, n-but-1-yn-4-yl, and n-but-2-yn-1-yl.

Unless otherwise specified, the term "halo" or "halogen" by itself or as part of another substituent refers to a fluorine, chlorine, bromine, or iodine atom.

Unless otherwise specified, the term "fluoroalkyl" is intended to include linear or branched monofluoroalkyl and polyfluoroalkyl. For example, the term "C₁₋₆ fluoroalkyl" is intended to include, but not be limited to, fluoromethyl, difluoromethyl, trifluoromethyl, 2-fluoroethyl, 2,2-difluoroethyl, 2,2,2-trifluoroethyl, tetrafluoroethyl, pentafluoroethyl, 3-fluoropropyl, 3,3-difluoropropyl, 2,2'-difluoroisopropyl, 3,3,3-trifluoropropyl, 4-fluorobutyl, 4,4-difluorobutyl, 4,4,4-trifluorobutyl, 2-fluoro-2-methylpropyl, 5,5,5-trifluoropentyl, and 6,6,6-trifluorohexyl.

Unless otherwise specified, the term "fluoroalkenyl" is intended to include linear or branched monofluoroalkenyl and polyfluoroalkenyl. For example, examples of "C₂₋₆ fluoroalkenyl" include 2-fluoroethenyl, 2,2-difluoroethenyl, 3-fluoro-1-propenyl, 3,3-difluoro-1-propenyl, 3-fluoro-2-propenyl, and 3,3-difluoro-2-propenyl.

Unless otherwise specified, the term "cycloalkyl" is intended to include any stable cyclic or polycyclic hydrocarbon group with any carbon atom being saturated, which may be monosubstituted or polysubstituted and may be monovalent, divalent, or polyvalent. For example, C₃₋₁₀ refers to 3 to 10 carbon atoms, and C₃₋₁₀ is selected from the group consisting of C₃, C₄, C₅, C₆, C₇, C₈, C₉, or C₁₀. Examples of such cycloalkyl groups include, but are not limited to, cyclopropyl, cyclobutyl, cyclopentyl, cyclohexyl, cycloheptyl, cyclooctyl, norbornyl, [2.2.2]bicyclooctyl, [4.4.0]bicyclodecyl, tricyclo[3.3.1.1^{3,7}]decyl (adamantyl), and the like.

Unless otherwise specified, the term "alkoxy" refers to alkyl-O-, where alkyl is as defined above. Examples of C₁₋₆ alkoxy include, but are not limited to, methoxy, ethoxypropyl, n-propoxy, isopropoxy, n-butoxy, OCH(CH₃)-C₂H₅, OCH₂-CH(CH₃)₂, OC(CH₃)₃, and the like.

Unless otherwise specified, the term "heterocyclyl" refers to a fully saturated or unsaturated cyclic group, such as a 3- to 7-membered monocyclic, 7- to 11-membered bicyclic, or 10- to 15-membered tricyclic ring system, which has one or more oxygen, sulfur, or nitrogen heteroatoms, preferably 1 to 4 or 1 to 3 heteroatoms, in the ring. The nitrogen and sulfur heteroatoms may optionally be oxidized, and the nitrogen heteroatom may optionally be quaternized. Monocyclic heterocyclyl groups include, but are not limited to, aziridinyl, azetidinyl, oxetanyl, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furanyl, tetrahydrofuranyl, thienyl, oxadiazolyl, piperidinyl, and piperazinyl.

Unless otherwise specified, the term "aryl" refers to a monovalent aromatic carbocyclic group of 6 to 10 carbon atoms, having a single ring or multiple fused rings. Examples of aryl include, but are not limited to, phenyl and naphthyl.

Unless otherwise specified, the term "heteroaryl" refers to a monovalent aromatic group of 5 to 10 ring atoms, having one or more oxygen, nitrogen, and sulfur heteroatoms, preferably 1 to 4 or 1 to 3 heteroatoms, in the ring. The nitrogen and sulfur heteroatoms may optionally be oxidized. The heteroaryl group may have a single ring (e.g., pyridinyl or furanyl) or multiple fused rings, provided that the point of attachment is a heteroaryl ring atom. Monocyclic heteroaryl generally includes 5- or 6-membered aromatic rings, and examples of monocyclic heteroaryl include pyridinyl, pyridazinyl, pyrimidinyl, pyrazinyl, triazinyl, pyrrolyl, indolyl, quinolinyl, isoquinolinyl, quinazolinyl, quinoxalinyl, furanyl, thienyl, furanyl, pyrrolyl, imidazolyl, oxazolyl, isoxazolyl, isothiazolyl, and pyrazolyl. Examples of fused-ring heteroaryl include benzofuranyl, benzothienyl, indolyl, benzimidazolyl, indazolyl, pyrazolo[1,5-a]pyrazinyl, quinolinyl, benzopyranyl, indolizinyl, and the like.

Compounds are named either manually or by ChemDraw^{®} software, and supplier's catalog names are given for commercially available compounds.

### DETAILED DESCRIPTION

The present disclosure will be further illustrated with reference to the following specific examples and test examples, but the scope of the present disclosure is not limited in any way.

### Example 1

### Preparation of fragment 1:

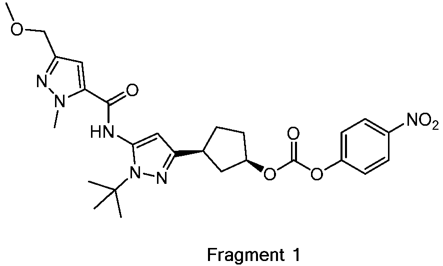

### Synthetic route:

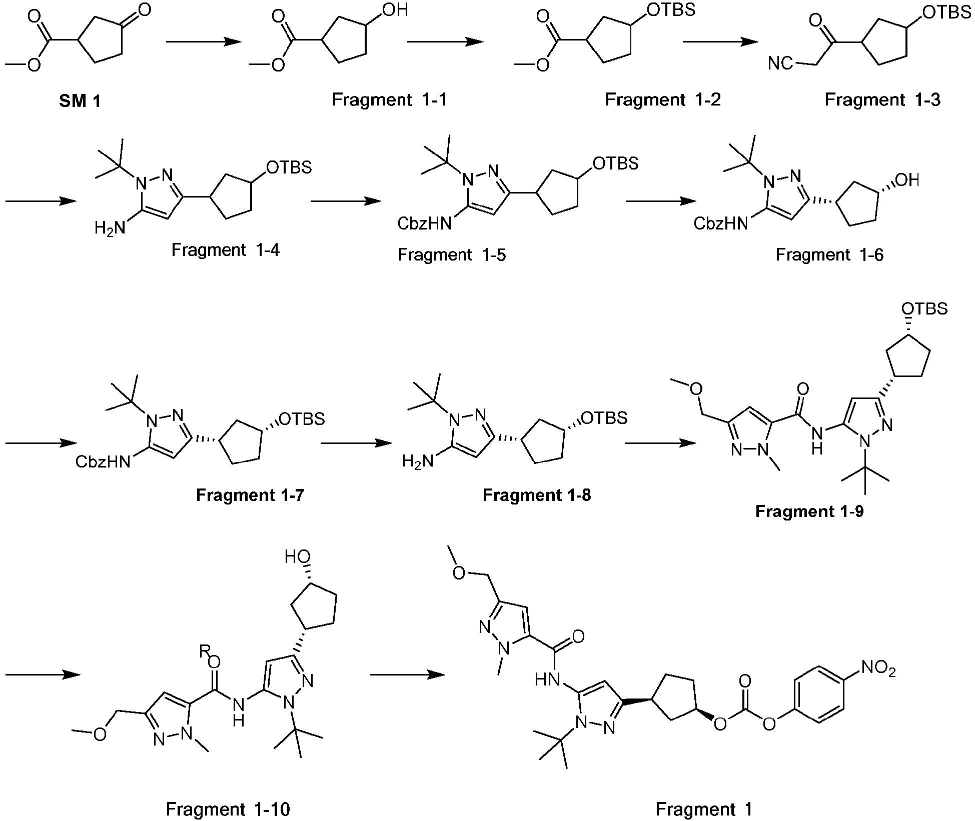

### Step 1: synthesis of fragment 1-1

Methanol (1500 mL) and SM 1 (150 g, 1.00 eq) were added into a 3000 mL three-necked flask in sequence, and the mixture was cooled to 0 °C. Sodium borohydride (41.4 g, 1.04 eq) was added to the reaction solution in 40 portions. The reaction system was reacted at 0 °C for 0.5 h in a nitrogen atmosphere. Acetic acid (40 mL) was added dropwise to the reaction solution, and the mixture was concentrated to dryness to remove methanol, so as to give a crude product. The crude product was extracted with ethyl acetate (1200 mL × 2). The organic phases were combined, washed with saturated brine (1200 mL), dried, and concentrated to dryness to give a crude product. The crude product was purified by column chromatography to give fragment 1-1 (white liquid, 147 g, 52.0% yield, crude).

### Step 2: synthesis of fragment 1-2

Anhydrous tetrahydrofuran (1750 mL) and fragment 1-1 (140 g, 1.00 eq) were added into a 5000 mL three-necked flask in sequence, and tert-butyldimethylchlorosilane (292 g, 2.00 eq) and imidazole (132 g, 2.00 eq) were added at room temperature. The reaction system was stirred at room temperature for 12 h. Water (700 mL) was added to the reaction solution, and the mixture was extracted with ethyl acetate (1000 mL × 3). The organic phases were combined, washed with saturated brine (1000 mL), dried, and concentrated to dryness to give a crude product. The crude product was purified by column chromatography to give fragment 1-2 (white liquid, 190 g, 55.8% yield, crude). MS m/z (ESI): 259 [M+H]⁺; 1H NMR (400 MHz CDCl₃), δ = 4.22 (quin, J = 5.4 Hz, 1H), 3.67 (s, 3H), 2.73 (t, J = 8.3 Hz, 1H), 2.16 - 2.02 (m, 2H), 1.96 - 1.76 (m, 3H), 1.73 - 1.54 (m, 2H), 0.88 - 0.85 (m, 9H), 0.08 - 0.02 (m, 6H).

### Step 3: synthesis of fragment 1-3

Anhydrous tetrahydrofuran (645 mL) and n-butyllithium (2.50 M, 495 mL, 2.00 eq) were added into a 3000 mL three-necked flask in sequence. The temperature was lowered to -65 °C, and acetonitrile (50.8 g, 65.2 mL, 2.00 eq) was slowly and dropwise added to the reaction solution. The reaction system was reacted at -65 °C for 1 h in a nitrogen atmosphere. Fragment 1-2 (160 g, 1.00 eq) was dissolved in anhydrous tetrahydrofuran (160 mL), and the compound 1-2 solution was added dropwise to the reaction solution at -65 °C. The system was reacted at -65 °C for 2 h in a nitrogen atmosphere. The reaction solution was warmed to 0 °C, and water (120 mL) was added dropwise to the reaction solution. The reaction solution was adjusted to pH 7 with a HCl solution (1 M, 1338 mL), and the mixture was extracted with ethyl acetate (1000 mL × 3). The organic phases were combined, washed with saturated brine (1200 mL), dried, and concentrated to dryness to give a crude product. The crude product was purified by column chromatography to give fragment 1-3 (pale yellow liquid, 121 g, 57.7% yield, 80.0% purity). MS m/z (ESI): 268 [M+H] ⁺; 1H NMR (400 MHz CDCl₃), δ= 4.39 - 4.26 (m, 1H), 3.60 - 3.49 (m, 2H), 3.02 (tt, J = 6.5, 8.9 Hz, 1H), 2.19 - 2.03 (m, 2H), 1.96 - 1.83 (m, 2H), 1.81 - 1.64 (m, 2H), 0.89 - 0.81 (m, 8H), 0.05 (s, 6H).

### Step 4: synthesis of fragment 1-4

Absolute ethanol (1073 mL) and sodium hydroxide (21.7 g, 1.20 eq) were added into a 3000 mL three-necked flask, and tert-butylhydrazine hydrochloride (67.6 g, 1.20 eq) was added at room temperature. The reaction solution was stirred at room temperature for 1 h. Fragment 1-3 (121 g, 1.00 eq) was dissolved in absolute ethanol (207 mL), and the compound 1-3 solution was added dropwise to a solution of tert-butylhydrazine hydrochloride at room temperature. The mixed solution was warmed to 85 °C and stirred for 12 h. The reaction solution was filtered, and the filtrate was concentrated to give fragment 1-4 (orange liquid, 147 g, 84.7% yield, 87.6% purity). MS m/z (ESI): 338[M+H]⁺.

### Step 5: synthesis of fragment 1-5

Acetonitrile (1590 mL) and fragment 1-4 (160 g, 1.00 eq) were added into a 3000 mL three-necked flask in sequence at 50 °C, and benzyl chloroformate (161 g, 2.00 eq) was added at room temperature. The system was reacted at room temperature for 16 h. The reaction solution was concentrated to dryness by rotary evaporation to give a crude product, and water (1000 mL) was added. The resultant mixture was extracted with ethyl acetate (1000 mL × 3). The organic phases were combined, washed with saturated brine (1000 mL), dried, filtered, and concentrated to dryness to give a crude product. The crude product was purified by column chromatography to give fragment 1-5 (orange liquid, 183 g, 71.5% yield). MS m/z (ESI): 472[M+H]⁺; 1H NMR (400 MHz CDCl₃), δ= 7.38 (br s, 5H), 6.30 - 5.91 (m, 2H), 5.20 (s, 2H), 4.42 - 4.25 (m, 1H), 3.00 (br t, J = 8.9 Hz, 1H), 2.34 - 2.11 (m, 1H), 2.01 - 1.64 (m, 6H), 1.58 (s, 9H), 0.89 (s, 10H), 0.07 - 0.02 (m, 6H).

### Step 6: synthesis of fragment 1-6

Absolute methanol (1630 mL) and fragment 1-5 (163 g, 1.00 eq) were added into a 3000 mL three-necked flask, and hydrochloric acid (1 M, 1.64 L, 4.72 eq) was added at room temperature. The system was reacted at room temperature for 1 h. Water (1000 mL) was added to the reaction solution, and the mixture was extracted with ethyl acetate (1000 mL × 3). The organic phases were combined, dried, and concentrated to dryness to give a crude product. The crude product was firstly purified by column chromatography to give a fragment 1-6 racemate (orange liquid, 77.1 g), which was then resolved by SFC to give fragment 1-6 (white solid, 35.7 g, 49.1% yield, 51.3% purity) in absolute configuration. SFC resolution conditions: column: Chiralpak AD 50 mm × 4.6 mm × 3 µm; mobile phase: A: CO₂, B: methanol (0.05% DEA); gradient: from 5% to 40% of B in 2.5 min and hold 40% for 0.5 min, then 5% of B for 1 min; flow rate: 4 mL/min; column temp.: 35 °C; ABPR: 1500 psi. MS m/z (ESI): 358[M+H]⁺; 1H NMR (400 MHz CDCl₃), δ = 7.37 (br s, 4H), 6.49 (br s, 1H), 6.06 (br s, 1H), 5.19 (s, 2H), 4.34 (br s, 1H), 3.27 - 3.17 (m, 1H), 2.13 - 1.76 (m, 6H), 1.55 (s, 9H).

### Step 7: synthesis of fragment 1-7

Anhydrous tetrahydrofuran (125 mL) and fragment 1-6 (10.0 g, 1.00 eq) in absolute configuration were added into a 100 mL single-necked flask, and tert-butyldimethylchlorosilane (8.43 g, 2.00 eq) and imidazole (3.81 g, 2.00 eq) were added to the reaction solution in sequence. The system was reacted at room temperature for 12 h. Water (100 mL) was added to the reaction solution, and the mixture was extracted with ethyl acetate (120 mL × 3). The organic phases were combined, washed with saturated brine (150 mL), dried, and concentrated to dryness to give a crude product. The crude product was purified by column chromatography to give fragment 1-7 (pink solid, 20.4 g, crude). MS m/z (ESI): 472 [M+H]⁺. 1H NMR (400 MHz CDCl₃), δ= 7.38 (br s, 4H), 6.26 - 6.05 (m, 2H), 5.20 (s, 2H), 4.30 (dd, J = 5.1, 6.0 Hz, 1H), 3.00 (br t, J = 9.0 Hz, 1H), 2.37 - 2.25 (m, 1H), 2.03 - 1.89 (m, 1H), 1.89 - 1.78 (m, 2H), 1.71 - 1.64 (m, 2H), 0.96 - 0.85 (m, 11H), 0.14 - 0.00 (m, 7H).

### Step 8: synthesis of fragment 1-8

Methanol (392 mL), fragment 1-7 (20.4 g, 1.00 eq), and wet Pd/C (7.84 g) were added into a 1000 mL hydrogenation flask in sequence. The system was reacted at 50 °C for 12 h in a hydrogen atmosphere (40 Psi). The reaction solution was filtered to give fragment 1-8 (pink liquid, 14.0 g, 87.5% yield, 91.3% purity). MS m/z (ESI): 338 [M+H]⁺. 1H NMR (400 MHz CDCl₃), δ = 5.47 (s, 1H), 4.28 (dd, J = 4.7, 6.2 Hz, 1H), 3.49 (br s, 2H), 3.00 - 2.87 (m, 1H), 2.34 - 2.22 (m, 1H), 1.99 - 1.63 (m, 5H), 1.61 (s, 9H), 0.93 - 0.85 (m, 9H), 0.12 - 0.03 (m, 6H).

### Step 9: synthesis of fragment 1-9

N,N-Dimethylformamide (5 mL), 3-(methoxymethyl)-1-methyl-1H-pyrazole-5-carboxylic acid (459 mg, 1.00 eq), N,N-diisopropylethylamine (697 mg, 2.00 eq), and O-(7-azabenzotriazol-1-yl)-N,N,N',N'-tetramethyluronium hexafluorophosphate (1.23 g, 1.20 eq) were added into a 50 mL single-necked flask at room temperature in sequence. The system was stirred at room temperature for 1 h. Fragment 1-8 (1.00 g, 1.10 eq) was added to the reaction solution, and the system was stirred at room temperature for 16 h (in two portions). Water (100 mL) was added to the reaction solution, and the mixture was extracted with ethyl acetate (50 mL × 4). The organic phases were combined, washed with saturated brine (240 mL), dried, and concentrated to dryness to give a crude product. The crude product was purified by column chromatography to give fragment 1-9 (brown liquid, 1.40 g, 34.4% yield, 65.0% purity). MS m/z (ESI): 490 [M+H]⁺. 1H NMR (400 MHz CDCl₃), δ = 8.01 (s, 1H), 7.56 (br s, 1H), 6.64 (br s, 1H), 6.27 (s, 1H), 4.48 (s, 2H), 4.30 (dd, J = 4.9, 6.2 Hz, 1H), 4.19 (s, 3H), 3.49 (s, 10H), 3.03 (s, 1H), 2.96 (s, 2H), 2.88 (s, 2H), 2.80 (s, 4H), 2.30 (dd, J = 5.9, 13.2 Hz, 1H), 2.09 - 1.76 (m, 5H), 1.66 - 1.60 (m, 9H), 0.89 (s, 9H), 0.05 (d, J = 1.9 Hz, 6H).

### Step 10: synthesis of fragment 1-10

Methanol (26.7 mL), fragment 1-9 (2.67 g, 1.00 eq), and hydrochloric acid (1 M, 26.7 mL, 4.90 eq) were added into a 100 mL single-necked flask in sequence. The mixture was stirred at room temperature for 1 h. Water (20 mL) was added to the reaction solution, and the mixture was extracted with ethyl acetate (30 mL × 10). The organic phases were combined, dried, and concentrated to dryness to give fragment 1-10 (brown liquid, 2.00 g, 67.2% yield, 68.8% purity). MS m/z (ESI): 376 [M+H]⁺. 1H NMR (400 MHz CDCl₃), δ = 7.94 (br s, 1H), 6.72 (br s, 1H), 6.20 (s, 1H), 4.47 (s, 2H), 4.39 - 4.33 (m, 1H), 4.18 (s, 3H), 3.46 - 3.37 (m, 3H), 3.33 - 3.23 (m, 1H), 2.03 - 1.74 (m, 5H), 1.61 (s, 8H), 0.94 - 0.85 (m, 3H).

### Step 11: synthesis of fragment 1

Anhydrous tetrahydrofuran (2.4 mL), fragment 1-10 (200 mg, 1.00 eq), and p-nitrophenyl chloroformate (214 mg, 2.00 eq) were added into a 50 mL three-necked flask at room temperature in sequence, followed by the addition of pyridine (126 mg, 3.00 eq) and 4-dimethylaminopyridine (6.51 mg, 0.10 eq) in sequence. The system was stirred at 50 °C for 28 h to give fragment 1, which was used directly in the next step.

### Example 2

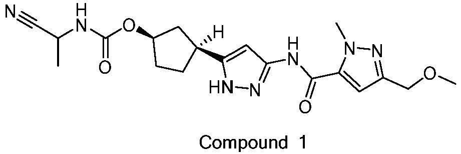

### Synthetic route:

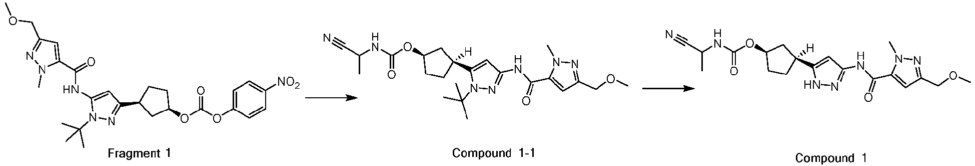

### Step 1: synthesis of compound 1-1

Anhydrous tetrahydrofuran (2.3 mL), N,N-diisopropylethylamine (205 mg, 10.00 eq), and 2-aminopropionitrile hydrochloride (169 mg, 10.0 eq) were added into a 50 mL three-necked flask at room temperature in sequence. The system was stirred at room temperature for 1 h. The reaction solution of 2-aminopropionitrile hydrochloride was added to a reaction solution of fragment 1 at a temperature of 50 °C. The resulting system was stirred at 50 °C for 12 h and then at 70 °C for 14 h. The reaction solution was adjusted to pH 5 with hydrochloric acid (1 M), and the mixture was extracted with dichloromethane (6 mL × 3). The organic phase was collected, adjusted to pH 8 with an aqueous Na₂CO₃ solution, washed with a saturated aqueous sodium chloride solution (10 mL × 3), dried, and concentrated to dryness to give a crude product. The crude product was purified by preparative thin-layer chromatography to give compound 1-1 (brown solid, 68.0 mg, 70.6% yield, 78.0% purity). MS m/z (ESI): 472 [M+H]⁺. 1H NMR (400 MHz CDCl₃), δ = 7.76 - 7.62 (m, 1H), 6.69 (br s, 1H), 6.23 (br d, J = 4.4 Hz, 1H), 5.61 (br d, J = 8.9 Hz, 1H), 4.65 (br d, J = 5.4 Hz, 1H), 4.19 (d, J = 1.1 Hz, 3H), 3.44 (s, 3H), 3.28 - 3.18 (m, 1H), 2.39 - 2.24 (m, 1H), 2.02 - 1.77 (m, 5H), 1.63 (d, J = 1.0 Hz, 9H), 1.53 (dd, J = 4.4, 7.3 Hz, 3H).

### Step 2: synthesis of compound 1

Formic acid (1.95 mL) and compound 1-1 (39.1 mg, 1.00 eq) were added into a 10 mL single-necked flask at room temperature in sequence. The system was stirred at 80 °C for 16 h. The reaction solution was directly concentrated to dryness by rotary evaporation to give a crude product. The crude product was purified by preparative chromatography to give compound 1 (white solid, 21.8 mg, 61.3% yield, 96.9% purity). MS m/z (ESI): 416 [M+H]⁺. 1H NMR (400 MHz CDCl₃), δ = 7.76 - 7.62 (m, 1H), 6.69 (br s, 1H), 6.23 (br d, J = 4.4 Hz, 1H), 5.61 (br d, J = 8.9 Hz, 1H), 4.65 (br d, J = 5.4 Hz, 1H), 4.19 (d, J = 1.1 Hz, 3H), 3.44 (s, 3H), 3.28 - 3.18 (m, 1H), 2.39 - 2.24 (m, 1H), 2.02 - 1.77 (m, 5H), 1.63 (d, J = 1.0 Hz, 9H), 1.53 (dd, J = 4.4, 7.3 Hz, 3H).

Referring to the synthesis methods of step 1 and step 2 in Example 2 (compound 1), compounds listed in the following table were synthesized:

| Compound No. | Structure | MS m/z | Nuclear magnetic resonance |
|---|---|---|---|
| Compound 2 | 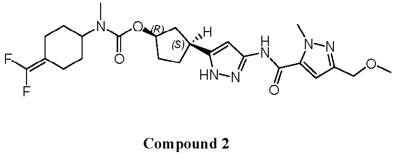 | 507.2 [M+H]⁺ | Hydrogen spectrum: |
| | | | ¹H NMR (400 MHz CDCl₃), δ =10.0 (br s, 1H), 6.88 (s, 1H), 6.73 (s, 1H), 5.21 (br s, 2H), 4.50 (s, 2H), 4.16 (s, 3H), 3.47 (s, 3H), 3.20 - 3.14 (m, 1H), 2.71 (br s, 3H), 2.58 - 2.54 (m, 3H), 2.16 - 2.10 (m, 1H), 1.98 - 1.93 (m, 8H), 1.90 - 1.89 (m, 2H), 1.46 - 1.43 (m, 2H). |
| | | | Fluorine spectrum: |
| | | | ¹⁹F NMR (400 MHz CDCl₃), δ = -97.74 - -97.80 |
| Compound 3 | 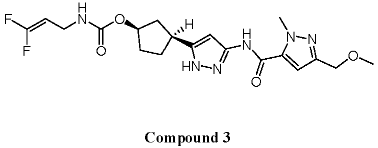 | 439.2 [M+H]⁺ | Hydrogen spectrum: |
| | | | ¹H NMR (400 MHz CDCl₃), δ = 10.7 (br s, 1H), 6.90 (s, 1H), 6.71 (s, 1H), 5.19 (br s, 2H). 4.51 (s, 2H). 4.45 - 4.35 (m, 1H). 4.13 (s, 3H). 3.77 (br s, 2H). 3.48 (s, 3H). 3.13 - 3.09 (m, 1H), 2.47 - 2.44 (m, 1H), 2.11 - 2.09 (m, 1H), 1.95 - 1.85 (m, 4H). |
| | | | Fluorine spectrum: |
| | | | ¹⁹F NMR (400 MHz CDCl₃), δ = -86.12 - -86.23. |
| | | | δ = - 88.06 - -88.16 |
| Compound 4 | 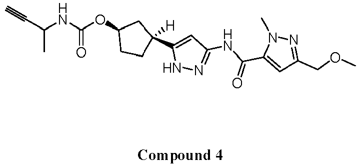 | 415 [M+H]⁺ | Hydrogen spectrum: |
| | | | ¹H NMR (400 MHz CD₃OD), δ = 6.92 (s, 1H), 6.48 (br s, 1H), 5.13 (br s, 1H), 4.44 (s, 2H), 4.41 (br s, 1H), 4.13 (s, 3H), 3.38 (s, 3H), 3.20 (br t, *J* = 7.1 Hz, 1H), 2.65 - 2.48 (m, 2H), 2.13 (br d, *J =* 2.3 Hz, 1H), 1.96 (br d, *J* = 6.1 Hz, 5H), 1.37 (d, *J =* 6.9 Hz, 3H) |
| Compound 5 | 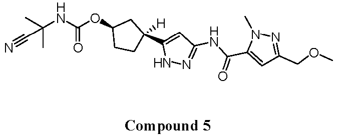 | 430 [M+H]⁺ | Hydrogen spectrum: |
| | | | ¹H NMR (400 MHz CD₃OD), δ = 7.05 (s, 1H), 6.41 (br s, 1H), 5.19 (br s, 1H), 4.46 (s, 2H), 4.16 (s, 3H), 3.39 (s, 3H), 2.72 - 2.60 (m, 1H), 2.22 (br d, *J* = 6.8 Hz, 1H), 2.10 - 1.82 (m, 4H), 1.63 (s, 6H) |
| Compound 6 | 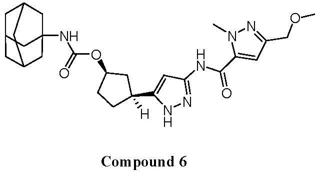 | 497 [M+H]⁺ | Hydrogen spectrum: |
| | | | ¹H NMR (400 MHz CDCl₃), δ = 10.63 (br s, 1H), 6.90 (s, 1H), 6.74 (s, 1H), 5.12 (br s, 1H), 4.68 (br s, 1H), 4.52 (s, 2H), 4.15 (s, 3H), 3.49 (s, 3H), 3.07 (br t, J = 8.3 Hz, 1H), 2.55 - 2.40 (m, 1H), 2.07 (br s, 4H), 1.98 - 1.77 (m, 11H), 1.65 (br s, 6H) |

### Example 3

### Preparation of fragment 2:

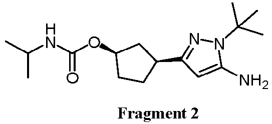

### Synthetic route:

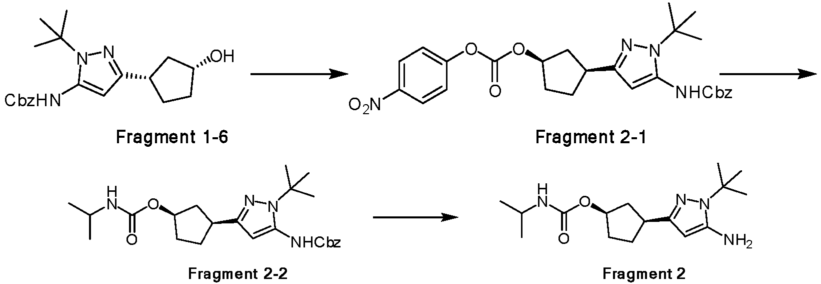

### Step 1: synthesis of fragment 2-1

Dichloromethane (120 mL), fragment 1-6 (10.0 g, 1.00 eq), p-nitrophenyl chloroformate (8.46 g, 1.50 eq), pyridine (6.64 g, 3.00 eq), and DMAP (341 mg, 0.10 eq) were added into a 250 mL three-necked flask in sequence. The system was reacted at 25 °C for 12 h in a nitrogen atmosphere to give fragment 2-1, which was used directly in the next step.

### Step 2: synthesis of fragment 2-2

Compound isopropylamine (4.96 g, 3.00 eq) and DIEA (10.85 g, 3.00 eq) were added to the reaction solution of fragment 2-1, and the system was stirred at 30 °C for 4 h. Hydrochloric acid (1 M, 200 mL) was added to the reaction solution, and the mixture was extracted with dichloromethane (200 mL × 2). The phases were separated, and the organic phase was adjusted to pH 8-9. The phases were separated again, and the organic phase was washed with brine, dried, and concentrated to dryness to give a crude product. The crude product was separated and purified by preparative chromatography to give fragment 2-2 (yellow oil, 9.2 g, 33.05% yield, 80% purity). MS m/z (ESI): 443 [M+H]⁺. 1H NMR (400 MHz CDCl₃), δ = 7.38 (br s, 4H), 6.34 - 6.04 (m, 2H), 5.15 (br s, 1H), 4.65 - 4.43 (m, 1H), 3.88 - 3.73 (m, 1H), 3.50 (d, J = 4.0 Hz, 1H), 3.08 (quin, J = 8.4 Hz, 1H), 2.53 - 2.38 (m, 1H), 2.09 - 1.98 (m, 1H), 1.96 - 1.76 (m, 4H), 1.58 (s, 9H), 1.17 - 1.11 (m, 6H).

### Step 3: synthesis of fragment 2

Methanol (140 mL), fragment 2-2 (7.12 g, 1.00 eq), and Pd/C (3.5 g, 10% purity) were added into a 500 mL hydrogenation flask in sequence. The system was reacted at 50 °C for 12 h in a H₂ atmosphere (40 Psi). The reaction solution was filtered and concentrated to dryness by rotary evaporation to give fragment 2 (pink oil, 5.06 g, crude). MS m/z (ESI): 309[M+H]⁺. 1H NMR (400 MHz CDCl₃), δ = 5.12 (br s, 1H), 4.45 (br s, 1H), 3.81 (br d, J = 4.8 Hz, 1H), 3.49 (br d, J = 4.4 Hz, 3H), 3.07 - 2.93 (m, 1H), 2.55 - 2.40 (m, 1H), 2.07 - 1.95 (m, 1H), 1.95 - 1.86 (m, 1H), 1.84 - 1.73 (m, 2H), 1.61 (s, 9H), 1.15 (d, J = 6.5 Hz, 6H).

### Example 4

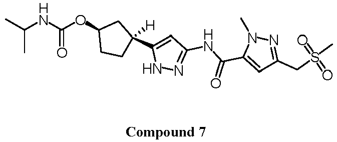

### Synthetic route:

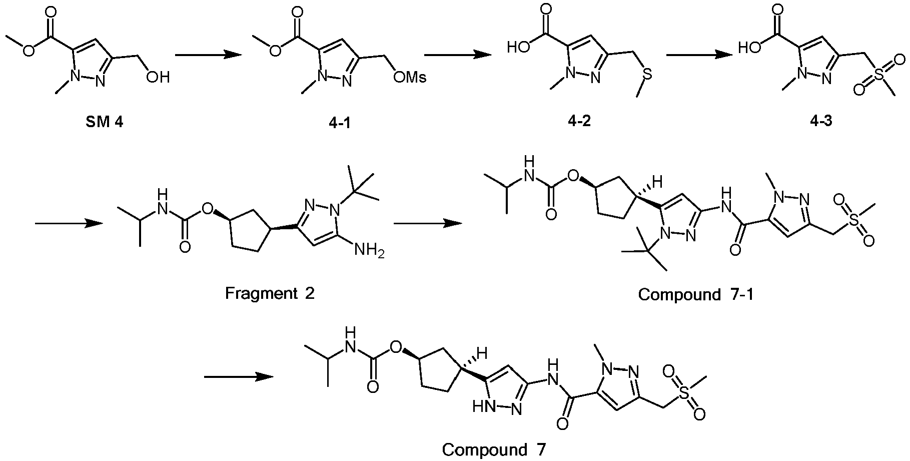

### Step 1: synthesis of 4-1

Dichloromethane (180 mL), compound SM 4 (18.0 g, 1.00 eq), potassium iodide (668 mg, 0.50 eq), and triethylamine (16.0 g, 1.50 eq) were added to a 500 mL three-necked flask in sequence, followed by the addition of methanesulfonyl chloride (13.7 g, 1.14 eq). The system was reacted at room temperature for 16 h. Water (100 mL) was added to the reaction solution, and the mixture was stirred for 10 min. The organic layer was separated, washed with water (100 mL), dried over Na₂SO₄, and filtered. The filtrate was concentrated in vacuum to give a crude product. The crude product was subjected to column chromatography to give compound 4-1 (yellow liquid, 10.5 g, crude).

### Step 2: synthesis of compound 4-2

N,N-Dimethylformamide (120 mL), compound 4-1 (2.00 g, 1.00 eq), potassium iodide (668 mg, 0.50 eq), and sodium thiomethoxide (1.69 g, 3.00 eq) were added into a 250 mL three-necked flask in sequence. The system was reacted at room temperature for 16 h in a N₂ atmosphere. The reaction solution was adjusted to pH 3 with hydrochloric acid (1 M), and the mixture was extracted with ethyl acetate (150 mL × 4). The organic phase was washed with brine, dried, and concentrated to dryness to give a crude product. The crude product (5 mL) was added dropwise to water (40 mL), and a solid was precipitated in the mixed solution. The mixture was filtered to give compound 4-2 (brown solid, 1.15 g, 67.9% yield, 89.0% purity). MS m/z (ESI): 187[M+H]⁺. 1H NMR (400 MHz DMSO-d6), δ = 6.70 (s, 1H), 4.01 (s, 3H), 3.61 (s, 2H), 2.01 (s, 3H).

### Step 3: synthesis of compound 4-3

Dichloromethane (11.4 mL), acetonitrile (11.4 mL), compound 4-2 (1.14 g, 1.00 eq), and m-chloroperoxybenzoic acid (3.73 g, 3.00 eq) were added to a 100 mL three-necked flask in sequence. The system was reacted at room temperature for 12 h in a N₂ atmosphere. Dichloromethane (20 mL) was added to the reaction solution. The mixture was filtered, and the filtrate was concentrated to give a crude product. The crude product was added to water (100 mL), and the mixture was extracted with ethyl acetate (40 mL × 15). The organic phase was dried and concentrated to dryness to give another crude product. The crude product was subjected to column chromatography to give compound 4-3 (pink solid, 182 mg, 19.4% yield, 93.5% purity) (white solid, 482 mg, 32.8% yield, 91.0% purity). MS m/z (ESI): 187[M+H]⁺. 1H NMR (400 MHz DMSO-d6), δ = 6.85 (s, 1H), 4.47 (s, 2H), 4.07 (s, 3H), 2.96 (s, 3H).

### Step 4: synthesis of compound 7-1

N,N-Dimethylformamide (5.3 mL), fragment 2 (530 mg, 1.00 eq), and compound 4-3 (375 mg, 1.00 eq) were added into a 50 mL three-necked flask in sequence, followed by the addition of O-(7-azabenzotriazol-1-yl)-N,N,N',N'-tetramethyluronium hexafluorophosphate (526 mg, 1.20 eq), and the system was stirred at room temperature for 2.5 h. Finally, N,N-diisopropylethylamine (666 mg, 3.00 eq) was added, and the resultant system was stirred at 70 °C for 2 h. Water (34 mL) was added to the reaction solution, and the mixture was extracted with ethyl acetate (15 mL × 4). The organic phase was washed with brine, dried, and concentrated to dryness to give a crude product. The crude product was subjected to column chromatography to give compound 7-1 (pink solid, 182 mg, 19.4% yield, 93.5% purity). MS m/z (ESI): 509[M+H]⁺. 1H NMR (400 MHz CDCl₃), δ = 7.98 (br d, J = 19.9 Hz, 1H), 6.92 (br s, 1H), 6.19 (s, 1H), 5.12 (br d, J = 4.1 Hz, 1H), 4.59 (br d, J = 2.9 Hz, 1H), 4.31 (s, 2H), 4.21 (s, 3H), 3.78 (br d, J = 5.1 Hz, 1H), 3.18 - 3.04 (m, 1H), 2.92 - 2.86 (m, 3H), 2.50 - 2.35 (m, 1H), 1.99 - 1.74 (m, 5H), 1.63 (s, 9H), 1.14 (dd, J = 1.9, 6.4 Hz, 6H).

### Step 5: synthesis of compound 7

Compound 7-1 (152 mg, 1.00 eq) was dissolved in formic acid (7.6 mL). The system was incubated for reaction at 80 °C for 16 h. The reaction solution was concentrated to dryness to give a crude product. The crude product was separated and purified by preparative chromatography to give compound 7 (white solid, 75.0 mg, 53.9% yield, 97.3% purity). MS m/z (ESI): 453 [M+H]⁺. 1H NMR (400 MHz CD₃OD), δ = 12.24 (s, 1H), 10.87 (s, 1H), 7.22 (s, 1H), 6.95 (br d, J = 7.3 Hz, 1H), 6.43 (br s, 1H), 5.01 (br s, 1H), 4.47 (s, 2H), 4.08 (s, 3H), 3.66 - 3.49 (m, 1H), 3.08 (br s, 1H), 3.00 (s, 3H), 2.47 (br s, 1H), 2.03 (br d, J = 7.3 Hz, 1H), 1.90 (br d, J = 3.0 Hz, 1H), 1.80 - 1.68 (m, 2H), 1.61 (br s, 1H), 1.03 (d, J = 6.5 Hz, 6H).

Referring to the synthesis methods of steps 1-5 in Example 4 (compound 7), compounds listed in the following table were synthesized:

| Compound No. | Structure | MS m/z | Nuclear magnetic resonance |
|---|---|---|---|
| Compound 12 | 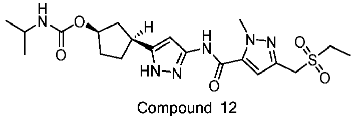 | 467.1 [M+H]⁺ | ¹H NMR (400 MHz, CDCl₃) δ 11.34 (br s, 1H), 10.75 - 9.69 (m, 1H), 7.06 (s, 1H), 6.76 (s, 1H), 5.16 (br s, 1H), 4.73 (br s, 1H), 4.34 - 4.25 (m, 2H), 4.22 (s, 3H), 3.86 - 3.72 (m, 1H), 3.25 (q, *J =* 8.2 Hz, 1H), 3.13 (q, *J* = 7.4 Hz, 2H), 2.53 - 2.42 (m, 1H), 2.15 - 2.04 (m, 1H), 1.89 (br s, 2H), 1.87 - 1.76 (m, 2H), 1.46 (t, *J* = 7.4 Hz, 3H), 1.17 - 1.12 (m, 6H). |
| Compound 13 | | 479.3 [M+H]⁺ | ¹H NMR (400 MHz, CDCl₃) δ 11.27 (br s, 1H), 7.09 (s, 1H), 6.76 (br s, 1H),5.17 (br s, 1H), 4.76 (br s, 1H), 4.38 (s, 2H), 4.22 (s, 3H), 3.85 - 3.75 (m, 1H), 3.31 - 3.19 (m, 1H), 2.59 - 2.50 (m, 1H), 2.50 - 2.41 (m, 1H), 2.16 - 2.05 (m, 1H), 1.90 (br, 2H), 1.88 - 1.77 (m, 2H), 1.30 - 1.24 (m, 2H), 1.17 (m, 1H), 1.14 (m, 5H), 1.12 - 1.10 (m, 1H). |
| Compound 16 | | 479.2 [M+H]⁺ | ¹H NMR (400 MHz, CDCl₃) δ 10.94 (br s, 0.5 H), 10.83 (br s, 0.5 H), 7.05 (s, 1H), 6.75 (s, 1 H), 5.19 -5.16 (m, 1 H), 4.37 - 4.28 (m, 2 H), 4,23 (s, 3 H), 3.88 - 3.77 (m, 2 H), 3.28 - 3.19 (m, 1 H), 3.00 (s, 3 H), 2.60 - 2.42 (m, 1 H), 2.18 - 2.06 (m, 1 H), 2.00 (t, *J* = 7.2 Hz, 2 H), 1.93 - 1.90 (m, 2 H), 1.78 - 1.68 (m, 2 H), 1.51 - 1.49 (m, 1 H), 1.48 (s, 3 H), 1.41 (d, *J =* 5.2 Hz, 3 H). |
| Compound 33 | | 403.1 [M+H]⁺ | ¹H NMR (400 MHz, DMSO-*d₆*) δ 12.25 (br s, 1H), 10.52 (br s, 1H), 6.96 (br d, *J =* 7.1 Hz, 1H), 6.39 (br s, 1H), 4.99 (br s, 3H), 4.72 (s, 2H), 3.98 (s, 3H), 3.68 - 3.46 (m, 1H), 3.19 - 2.94 (m, 1H), 2.12 - 1.82 (m, 2H), 1.81 - 1.52 (m, 2H), 1.60 (br s, 1H), 1.11 - 0.95 (m, 6H). |

### Example 5

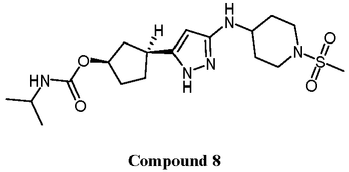

### Synthetic route:

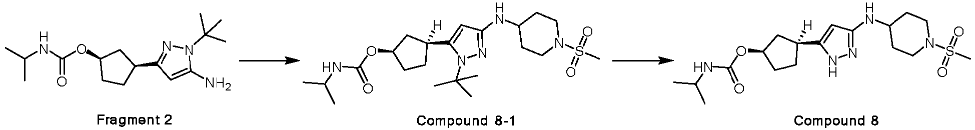

### Step 1: synthesis of compound 8-1

Dichloroethane (20 mL), compound 1-(methylsulfonyl)piperidin-4-one (344 mg, 1.00 eq), fragment 2 (600 mg, 1.00 eq), and zinc chloride (318 mg, 1.20 eq) were added into a 100 mL three-necked flask in sequence. The system was incubated for reaction at 85 °C for 12 h. After the formation of the intermediate state was detected, sodium triacetoxyborohydride (659 mg, 1.60 eq) was added at room temperature, and the mixture was incubated for reaction at 60 °C for 14 h. Water (20 mL) was added, and the mixture was extracted with dichloromethane (20 mL × 2). The organic phase was washed with brine, dried, and concentrated to dryness to give a crude product. The crude product was separated and purified by preparative chromatography to give compound 8-1 (yellow solid, 475 mg, 44.19% yield, 85% purity). MS m/z (ESI): 470 [M+H]⁺.

### Step 2: synthesis of compound 8

Compound 8-1 (475 mg, 1.00 eq) was dissolved in concentrated hydrochloric acid (12 M, 23 mL). The system was incubated for reaction at room temperature for 12 h. The reaction solution was concentrated to dryness to give a crude product. The crude product was separated and purified by preparative chromatography and then separated by SFC to give compound 8 (white solid, 91 mg, 21.54% yield, 99% purity). MS m/z (ESI): 414[M+H]⁺. 1H NMR (400 MHz CD₃OD), δ= 5.46 (br s, 1H), 5.06 (br s, 1H), 4.59 (s, 5H), 3.74 - 3.60 (m, 3H), 3.40 - 3.33 (m, 1H), 3.12 - 2.99 (m, 1H), 2.97 - 2.86 (m, 2H), 2.84 (s, 3H), 2.55 - 2.43 (m, 1H), 2.14 - 2.00 (m, 3H), 1.98 - 1.66 (m, 2H), 1.57 - 1.44 (m, 2H), 1.12 (d, J = 6.6 Hz, 6H).

### Example 6

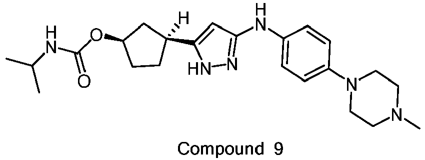

### Synthetic route:

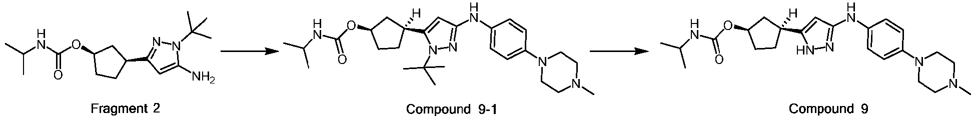

### Step 1: synthesis of compound 9-1

Toluene (10 mL), 1-(4-iodophenyl)-4-methylpiperazine (1.02 g, 1.00 eq), fragment 2 (1.00 g, 1.00 eq), sodium tert-butoxide (477 mg, 1.50 eq), tri-tert-butylphosphine (401 mg, 10% purity, 0.06 eq), and tris(dibenzylideneacetone)dipalladium (90 mg, 0.03 eq) were added into a 25 mL microwave tube in sequence. The system was reacted in microwaves at 100 °C for 1 h. The reaction solution was filtered, and extracted with dichloromethane (20 mL × 2). The organic phase was washed with brine, dried, and concentrated to dryness to give a crude product. The crude product was separated and purified by preparative chromatography to give compound 9-1 (yellow solid, 927 mg, 49.90% yield, 86% purity). MS m/z (ESI): 483[M+H]⁺. ¹H NMR (400 MHz CD₃OD), δ = 6.85 (d, J = 8.9 Hz, 2H), 6.69 (d, J = 8.9 Hz, 2H), 5.87 (s, 1H), 5.31 (s, 1H), 5.13 (br s, 1H), 4.87 (s, 1H), 4.55 - 4.32 (m, 1H), 3.79 (br d, J = 3.9 Hz, 1H), 3.25 (br t, J = 4.8 Hz, 4H), 3.10 - 3.01 (m, 1H), 2.94 (br s, 4H), 2.60 (s, 3H), 2.55 - 2.46 (m, 1H), 1.96 - 1.71 (m, 4H), 1.61 (s, 9H), 1.17 - 1.08 (m, 6H).

### Step 2: synthesis of compound 9

Compound 9-1 (726 mg, 1.00 eq) was dissolved in formic acid (366 mL). The system was incubated for reaction at 80 °C for 12 h. The reaction solution was concentrated to dryness to give a crude product. The crude product was separated and purified by preparative chromatography to give compound 9 (white solid, 90 mg, 13.98% yield, 99.63% purity). MS m/z (ESI): 427[M+H]⁺. ¹H NMR (400 MHz CD₃OD), δ = 7.04 (br d, J = 8.4 Hz, 2H), 6.87 (br d, J = 8.8 Hz, 2H), 5.68 (s, 1H), 5.04 (br s, 1H), 4.56 (br s, 3H), 3.71 - 3.57 (m, 1H), 3.29 - 3.24 (m, 4H), 3.06 (br d, J = 4.4 Hz, 5H), 2.68 - 2.56 (m, 4H), 2.53 - 2.41 (m, 1H), 2.33 (s, 3H), 2.05 (br d, J = 7.5 Hz, 1H), 1.95 - 1.65 (m, 4H), 1.33 - 1.21 (m, 1H), 1.12 - 1.00 (m, 6H).

### Example 7

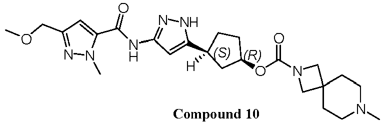

### Synthetic route:

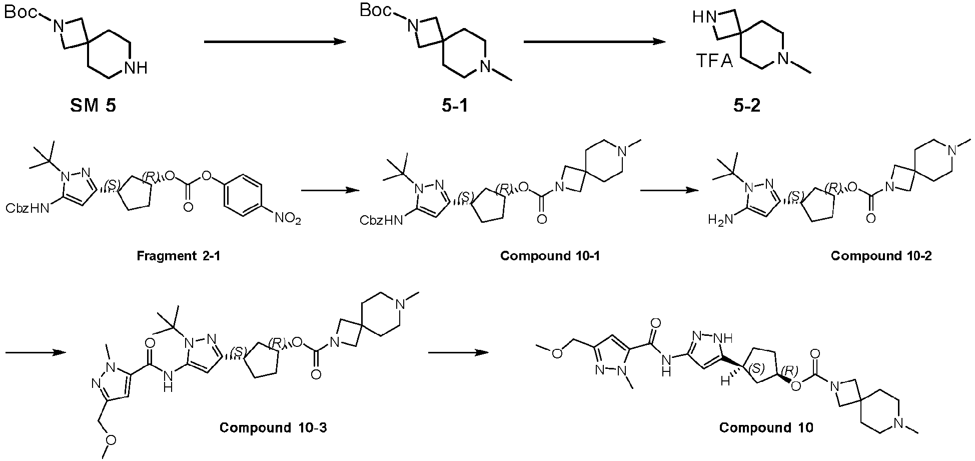

### Step 1: synthesis of compound 5-1

Tetrahydrofuran (80 mL), CH₂O (584 mg, 1.10 eq), and SM 5 (4.00 g, 1.00 eq) were added to a 250 mL three-necked flask in sequence, and the system was stirred at 25 °C for 30 min. NaBH(OAc)₃ (50.0 mg, 3.00 eq) was added, and the resultant system was stirred at 25 °C for 12 h. The reaction solution was concentrated to dryness, and water (80 mL) was added. The mixture was extracted with dichloromethane (200 mL × 3). The organic phases were combined, washed with a saturated sodium bicarbonate solution (1 M, 800 mL), dried, and concentrated to dryness to give compound 5-1 (yellow oil, 3.00 g, crude).

### Step 2: synthesis of compound 5-2

Dichloromethane (100 mL) and compound 5-1 (3.00 g, 1.00 eq) were added to a 250 mL single-necked flask, and trifluoroacetic acid (30.4 g, 21.4 eq) was added to the reaction solution at 0 °C. The system was stirred at 25 °C for 12 h. The reaction solution was concentrated to dryness to give compound 5-2 (yellow oil, 8.90 g, TFA salt, crude). 1H NMR (400 MHz DMSO-d6), δ = 3.80 - 3.62 (m, 9H), 3.34 - 3.21 (m, 2H), 2.92 - 2.78 (m, 2H), 2.68 - 2.56 (m, 3H), 2.13 - 2.03 (m, 2H), 1.89 - 1.82 (m, 4H), 1.80 - 1.66 (m, 2H).

### Step 3: synthesis of compound 10-1

Tetrahydrofuran (50 mL), fragment 2-1 (2.70 g, 1.00 eq), compound 5-2 (4.19 g, 1.10 eq), and DIEA (2.07 mg, 3.10 eq) were added to a 10 mL single-necked flask in sequence. The system was stirred at 0 °C for 30 min. The reaction solution was concentrated to dryness to give a crude product. The crude product was purified by column chromatography and preparative chromatography to give compound 10-1 (yellow solid, 900 mg, 33.2% yield). MS m/z (ESI): 524 [M+H]⁺. ¹H NMR (400 MHz CDCl₃), δ = 7.38 (br s, 5H), 6.48 (s, 1H), 6.13 (br s, 1H), 5.14 (br s, 1H), 3.77 - 3.72 (m, 4H), 3.57 - 3.45 (m, 2H), 3.23 - 3.05 (m, 7H), 2.75 (br s, 2H), 2.65 (br d, J = 1.8 Hz, 1H), 2.54 - 2.21 (m, 3H), 1.91 - 1.79 (m, 4H), 1.60 (s, 9H).

### Step 4: synthesis of compound 10-2

Tetrahydrofuran (10 mL), compound 10-1 (900 mg, 1.00 eq), and dry Pd/C (100 mg, 0.10 eq) were added into a 75 mL hydrogenation flask. The system was stirred at 50 °C for 12 h in a hydrogen atmosphere (45 Psi). After the reaction solution was filtered, the filtrate was concentrated to dryness to give crude compound 10-2 (yellow oil, 500 mg, 67.9% yield). MS m/z (ESI): 390[M+H]⁺. ¹H NMR (400 MHz CDCl₃), δ = 5.43 - 5.37 (m, 1H), 5.16 - 5.04 (m, 1H), 3.79 - 3.70 (m, 6H), 3.60 - 3.50 (m, 1H), 3.12 (br s, 1H), 3.04 - 2.93 (m, 1H), 2.78 (s, 3H), 2.45 (td, J = 7.3, 14.2 Hz, 1H), 2.13 - 1.98 (m, 4H), 1.96 - 1.82 (m, 4H), 1.81 - 1.68 (m, 3H), 1.62 (s, 9H).

### Step 5: synthesis of compound 10-3

Toluene (9 mL), triethylamine (321.9 mg, 2.00 eq), compound 10-2 (600 mg, 1.90 e-1 eq), and 3-(methoxymethyl)-1-methyl-1H-pyrazole-5-carbonyl chloride (900.0 mg, 1.00 eq) were added into a 100 mL single-necked flask. The system was stirred at 120 °C for 12 h. The reaction solution was concentrated to dryness and purified by preparative thin-layer chromatography to give a mixture of compound 10-3 (yellow solid, 320 mg, crude) and compound 10 (pale yellow solid, 60 mg, crude).

### Step 6: synthesis of compound 10

HCOOH (15 mL) and compound 10-3 (320 mg, 1.00 eq) were added to a 50 mL single-necked flask in sequence. The system was stirred at 80 °C for 12 h. The reaction solution was concentrated to dryness to give a crude product, and a saturated aqueous sodium carbonate solution (10 mL) was added to the crude product. The mixture was extracted with dichloromethane (20 mL × 3), and the organic phases were combined, dried, and concentrated to dryness to give another crude product. The crude product was purified once by preparative thin-layer chromatography and twice by preparative chromatography to give compound 10 (white solid, 10.0 mg, 45.37% purity). MS m/z (ESI): 486[M+H]⁺. ¹H NMR (400 MHz CDCl₃), δ = 6.33 (s, 1H), 5.23 - 5.03 (m, 1H), 3.68 (s, 3H), 3.24 - 3.00 (m, 1H), 2.50 - 2.39 (m, 2H), 2.35 - 2.23 (m, 3H), 2.14 - 2.01 (m, 2H), 1.93 - 1.75 (m, 8H), 1.58 (br d, J = 6.3 Hz, 1H), 1.52 - 1.42 (m, 1H), 1.26 (s, 3H), 1.13 - 1.00 (m, 2H), 0.93 - 0.78 (m, 7H).

### Example 8

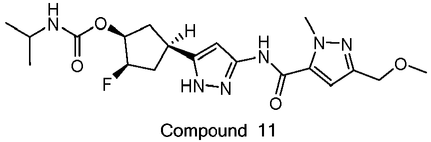

### Synthetic route:

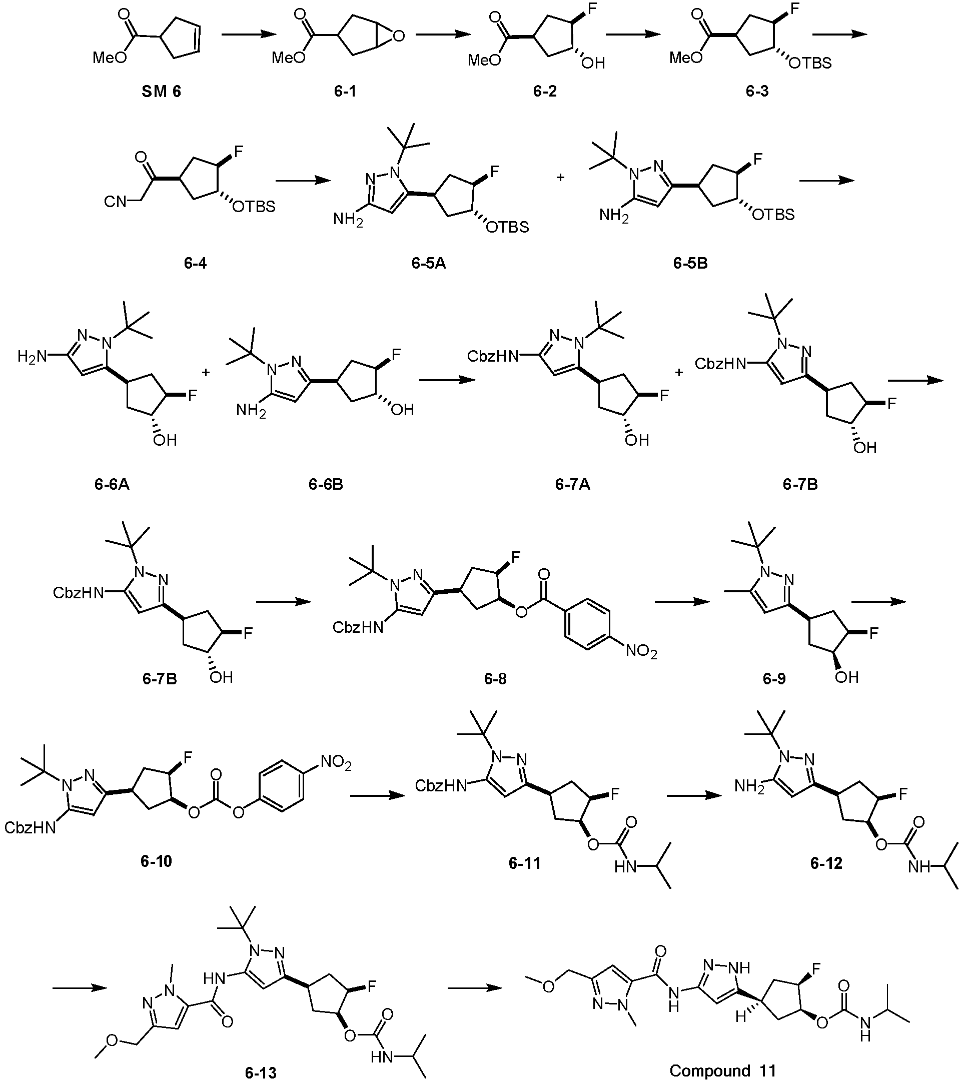

### Step 1: synthesis of 6-1

At 0 °C, m-CPBA (80.4 g, 396 mmol, 85.0% purity, 2.50 eq) was added to a mixture of SM 6 (20.0 g, 158 mmol, 1.00 eq) in DCM (400 mL). The mixture was stirred at 20 °C for 12 h. After TLC (petroleum ether: ethyl acetate = 3:1, Rf = 0.30, 0.17) indicated the complete depletion of the reactants, the reaction mixture was cooled, quenched with NaHCO₃ (500 mL), and was adjusted to pH 7-8. The separated organic layer was washed with Na₂S₂O₃ (200 mL × 2) and brine (300 mL × 2), dried over anhydrous Na₂SO₄, and concentrated at reduced pressure. The residue was purified by silica gel chromatography (column height: 250 mm, diameter: 100 mm, 100-200 mesh silica gel, petroleum ether/ethyl acetate = 100/1, 0/1) to give 6-1 (yellow oil, 15.0 g, 104 mmol, 66.2% yield, 99.5% purity).

### Step 2: synthesis of compound 6-2

At 20 °C, triethylamine trihydrofluoride (25.5 g, 158 mmol, 25.8 mL, 1.50 eq) was added to 6-1 (15.0 g, 105 mmol, 1.00 eq) in a N₂ atmosphere. The mixture was stirred at 120 °C for 12 h. After TLC (petroleum ether:ethyl acetate = 2:1, Rf = 0.30) indicated the complete depletion of the reactants, the mixture was cooled to 5 °C, quenched by adding saturated NaHCO₃ (500 mL), stirred for 1 h, and diluted with DCM (200 mL). The layers were separated, and the aqueous layer was extracted with additional DCM (200 mL × 4). The combined organic extracts were washed with brine, dried over anhydrous Na₂SO₄, filtered, and evaporated. The residue was purified by silica gel chromatography (column height: 250 mm, diameter: 100 mm, 100-200 mesh silica gel, petroleum ether/ethyl acetate = 100/1, 0/1) to give compound 6-2 (colorless oil, 13.0 g, 79.8 mmol, 75.6% yield, 99.6% purity). ¹H NMR: (400MHz, CDCl₃) δ = 4.97 - 4.75 (m, 1H), 4.45 - 4.32 (m, 1H), 3.81 - 3.64 (m, 3H), 3.17 - 3.04 (m, 1H), 2.54 - 2.35 (m, 1H), 2.33 - 2.10 (m, 2H), 1.98 (ddd, J = 2.4, 8.4, 14.0 Hz, 1H), 1.90 (br s, 1H); ¹⁹F NMR: (376MHz, CDCl₃) δ= -177.97 (s, 1F).

### Step 3: synthesis of compound 6-3

TBSCl (24.1 g, 160 mmol, 19.6 mL, 2.00 eq) and imidazole (21.8 g, 320 mmol, 4.00 eq) were added to a mixture of 6-2 (13.0 g, 80.1 mmol, 1.00 eq) in DMF (50.0 mL) at 20 °C in a N₂ atmosphere. The mixture was stirred at 30 °C for 12 h. After TLC (petroleum ether:ethyl acetate = 10:1, Rf = 0.49) indicated the complete depletion of the reactants, the reaction mixture was quenched by adding water (100 mL) at 10 °C and then extracted with ethyl acetate (50.0 mL × 3). The combined organic layers were washed with brine (50.0 mL × 2), dried over anhydrous Na₂SO₄, filtered, and concentrated at reduced pressure to give a residue. The residue was purified by silica gel chromatography (column height: 250 mm, diameter: 100 mm, 100-200 mesh silica gel, petroleum ether/ethyl acetate = 100/1, 0/1) to give compound 6-3 (colorless oil, 13.0 g, 45.7 mmol, 57.0% yield, 97.3% purity). ¹H NMR: (400MHz,CDCl₃) δ 4.89 - 4.62 (m, 1H), 4.35 - 4.22 (m, 1H), 3.77 - 3.65 (m, 3H), 3.17 - 3.01 (m, 1H), 2.52 - 2.28 (m, 1H), 2.21 - 2.06 (m, 2H), 1.92 (br dd, J = 8.1, 13.4 Hz, 1H), 0.87 (s, 9H), 0.10 - 0.05 (m, 6H); ¹⁹F NMR: (377MHz, CDCl₃) δ= -175.84 (s, 1F).

### Step 4: synthesis of compound 6-4

At 5 °C, t-BuOK (4.26 g, 37.9 mmol, 1.00 eq), MeCN (1.56 g, 37.9 mmol, 2.00 mL, 1.00 eq), and IPA (456 mg, 7.60 mmol, 581 µL, 0.20 eq) were added to a mixture of 6-3 (10.5 g, 37.9 mmol, 1.00 eq) in THF (100 mL). The mixture was stirred at 20 °C for 1 h. t-BuOK (4.26 g, 37.9 mmol, 1.00 eq) and MeCN (1.56 g, 37.9 mmol, 2.00 mL, 1.00 eq) were added to the mixture at 5 °C. The mixture was stirred at 20 °C for 2 h. After TLC (petroleum ether:ethyl acetate = 5:1, Rf = 0.30) indicated the complete depletion of the reactants, the reaction mixture was quenched by adding aqueous NH₄Cl (100 mL) and then extracted with DCM (50.0 mL × 3). The combined organic layers were washed with brine (50.0 mL × 2), dried over anhydrous Na₂SO₄, filtered, and concentrated at reduced pressure to give a residue. The residue was purified by silica gel chromatography (column height: 250 mm, diameter: 100 mm, 100-200 mesh silica gel, petroleum ether/ethyl acetate = 100/1, 0/1) to give 6-4 (yellow oil, 7.50 g, 26.2 mmol, 69.1% yield).

### Step 5: synthesis of compound 6-5

TEA (3.19 g, 31.5 mmol, 4.39 mL, 1.20 eq) and tert-butylhydrazine hydrochloride (3.93 g, 31.5 mmol, 1.20 eq) were added to a mixture of compound 6-4 (7.50 g, 26.2 mmol, 1.00 eq) in ethanol (100 mL) at 20 °C. The mixture was stirred at 70 °C for 12 h. After HPLC indicated the complete depletion of the reactants, the reaction mixture was filtered, and the filtrate was concentrated. The residue was purified by silica gel chromatography (column height: 250 mm, diameter: 100 mm, 100-200 mesh silica gel, petroleum ether/ethyl acetate = 100/1, 0/1) to give compound 6-5A and compound 6-5B (6.50 g, 18.2 mmol, 69.5% yield) as yellow oils. MS m/z (ESI): 356[M+H]⁺.

### Step 6: synthesis of compound 6-6

TBAF (1.00 M, 25.3 mL, 1.50 eq) was added to a mixture of compound 6-5A and compound 6-5B (6.00 g, 16.8 mmol, 1.00 eq) in THF (50.0 mL) at 20 °C. The mixture was stirred at 20 °C for 12 h. After TLC (petroleum ether:ethyl acetate = 1:1, Rf = 0.30, 0.11) indicated the complete depletion of the reactants, the mixture was poured into aqueous NH₄Cl (50.0 mL) and stirred for 20 min. The aqueous phase was extracted with ethyl acetate (30.0 mL × 3), and the combined organic phases were washed with brine (20 mL × 1), dried over anhydrous Na₂SO₄, and filtered and concentrated in vacuum. The residue was purified by silica gel chromatography (column height: 250 mm, diameter: 100 mm, 100-200 mesh silica gel, petroleum ether/ethyl acetate = 100/1, 0/1) to give compound 6-6A and compound 6-6B (4.07 g, crude) as yellow oils. MS m/z (ESI): 242[M+H]⁺.

### Step 7: synthesis of compound 6-7

CbzCl (8.63 g, 50.6 mmol, 7.19 mL, 3.00 eq) and NaHCO₃ (5.67 g, 67.4 mmol, 2.62 mL, 4.00 eq) were added to a mixture of compound 6-6A and compound 6-6B (4.07 g, 16.8 mmol, 1.00 eq) in MECN (20.0 mL). The mixture was stirred at 80 °C for 12 h. After TLC (petroleum ether:ethyl acetate = 2:1, Rf = 0.30, 0.46) indicated that the remaining amounts of compound 6-6A and compound 6-6B were both 20%, the reaction mixture was filtered, and the filtrate was concentrated. The residue was subjected to preparative high-performance liquid chromatography (column: Agela DuraShell C18 250 × 70 mm × 10 µm; mobile phase: [water (NH₄HCO₃)-ACN]; B%: 35% to -55%, 20 min) to give compound 6-7A (yellow solid, 1.00 g, crude) and compound 6-7B (yellow solid, 1.20 g, crude). MS m/z (ESI): 376[M+H]⁺.

### Step 8: synthesis of compound 6-8

At 20 °C, PNBA (1.07 g, 6.39 mmol, 2.00 eq) and PPh3 (1.68 g, 6.39 mmol, 2.00 eq) were added to a solution of compound 6-7B (1.20 g, 3.20 mmol, 1.00 eq) in THF (20.0 mL). At 5 °C, DEAD (1.11 g, 6.39 mmol, 1.16 mL, 2.00 eq) was added dropwise. The suspension was degassed in vacuum and purged several times with nitrogen. The mixture was stirred at 60 °C for 12 h in a nitrogen atmosphere. After TLC (petroleum ether:ethyl acetate = 3:1, Rf = 0.41) indicated the complete depletion of the reactants, the reaction mixture was concentrated at reduced pressure. The residue was purified by silica gel chromatography (column height: 250 mm, diameter: 100 mm, 100-200 mesh silica gel, petroleum ether/ethyl acetate = 100/1, 0/1) to give compound 6-8 (yellow oil, 1.90 g, 2.17 mmol, 68.0% yield, 60.7% purity). MS m/z (ESI): 525[M+H]⁺.

### Step 9: synthesis of compound 6-9

At 20 °C, NaHCO₃ (608 mg, 7.24 mmol, 281 µL, 2.00 eq) was added to a mixture of compound 6-8 (1.90 g, 3.62 mmol, 1.00 eq) in MeOH (20.0 mL). The mixture was stirred at 20 °C for 4 h. After TLC (petroleum ether:ethyl acetate = 1:1, Rf = 0.20) indicated the complete depletion of the reactants, the reaction mixture was filtered, and the filtrate was concentrated. The residue was purified by silica gel chromatography (column height: 250 mm, diameter: 100 mm, 100-200 mesh silica gel, petroleum ether/ethyl acetate = 100/1, 0/1) to give compound 6-9 (off-white solid, 600 mg, 1.60 mmol, 44.1% yield, purity n/a). MS m/z (ESI): 376[M+H]⁺.

### Step 10: synthesis of compound 6-10

At 20 °C, pyridine (379 mg, 4.79 mmol, 386 µL, 3.00 eq) and (4-nitrobenzene)carbonyl chloride (644 mg, 3.20 mmol, 2.00 eq) were added to a mixture of compound 6-9 (600 mg, 1.60 mmol, 1.00 eq) in DCM (2.00 mL). The mixture was stirred at 20 °C for 12 h. After TLC (petroleum ether:ethyl acetate = 3:1, Rf = 0.59) indicated the complete depletion of the reactants, the reaction mixture was concentrated at reduced pressure at 40 °C. The residue was purified by silica gel chromatography (column height: 250 mm, diameter: 100 mm, 100-200 mesh silica gel, petroleum ether/ethyl acetate = 100/1, 0/1) to give compound 6-10 (yellow solid, 800 mg, crude). MS m/z (ESI): 541[M+H]⁺.

### Step 11: synthesis of compound 6-11

DIEA (765 mg, 5.92 mmol, 1.03 mL, 4.00 eq) was added to a mixture of compound 6-10 (800 mg, 1.48 mmol, 1.00 eq) and propan-2-amine (104 mg, 1.78 mmol, 152 µL, 1.20 eq) in THF (10.0 mL). The mixture was stirred at 20 °C for 4 h. After thin-layer chromatography (petroleum ether:ethyl acetate = 1:1, Rf = 0.20) indicated the complete depletion of the reactants, the reaction mixture was concentrated at reduced pressure. The residue was purified by silica gel chromatography (column height: 250 mm, diameter: 100 mm, 100-200 mesh silica gel, petroleum ether/ethyl acetate = 100/1, 0/1) to give compound 6-11 (yellow oil, 600 mg, crude). MS m/z (ESI): 461[M+H]⁺.

### Step 12: synthesis of compound 6-12

In a N₂ atmosphere, Pd/C (300 mg, 651 µmol, 20.0% purity, 0.50 eq) was added to a solution of compound 6-11 (600 mg, 1.30 mmol, 1.00 eq) in ethyl acetate (20 mL) and tetrahydrofuran (10 mL). The suspension was degassed in vacuum and purged several times with H₂. The mixture was stirred at 20 °C for 12 h in a H₂ atmosphere (20 psi). After LCMS indicated the complete depletion of the reactants, the reaction mixture was filtered, and the filtrate was concentrated. The crude product was subjected to high-performance liquid chromatography (column: Waters Xbridge 150 × 25 mm × 5 µm; mobile phase: [water (NH₄HCO₃)-ACN]; B%: 25% to -55%, 8 min) to give compound 6-12 (brown solid, 200 mg, 549 µmol, 42.1% yield, 89.6% purity). MS m/z (ESI): 327[M+H]⁺.

### Step 13: synthesis of compound 6-13

At 20 °C, TCFH (85.9 mg, 306 µmol, 2.00 eq) and NMI (37.7 mg, 459 µmol, 36.6 µL, 3.00 eq) were added to a mixture of 3-(methoxymethyl)-1-methyl-1H-pyrazole-5-carboxylic acid (52.1 mg, 306 µmol, 2.00 eq) in MeCN (2.00 mL). The mixture was stirred at 20 °C for 30 min. Compound 6-12 (50.0 mg, 153 µmol, 1.00 eq) was added to the mixture at 20 °C. The mixture was stirred at 60 °C for 12 h. When LC-MS indicated about 14.20% of residual compound 6-12 and about 15.4% of the target compound 6-13, the reaction mixture was quenched by adding water (20.0 mL) at 10 °C, diluted with DCM (5 mL), and extracted with DCM (10 mL × 3). The combined organic layers were washed with brine (20 mL × 1), dried over Na₂SO₄, filtered, and concentrated at reduced pressure to give a residue. The residue was purified by thin-layer chromatography (petroleum ether:ethyl acetate = 0:1, Rf = 0.63) to give compound 6-13 (brown oil, 73.0 mg), which was used in the next step. MS m/z (ESI): 479[M+H]⁺.

### Step 14: synthesis of compound 11

At 20 °C, HCOOH (2.00 mL) was added to compound 6-13 (73.0 mg, 152 µmol, 1.00 eq). The mixture was stirred at 100 °C for 2 h. After LC-MS (ET57793-76-P1A, RT = 1.298) indicated the complete depletion of the reactants, the reaction mixture was concentrated at reduced pressure to remove HCOOH (2 mL). The residue was subjected to HPLC (column: Phenomenex C18 75 × 30 mm × 3 µm; mobile phase: [water (NH₄HCO₃)-ACN]; B%: 10% to -40%, 8 min) to give compound 11 (white solid, 23.0 mg, 54.3 µmol, 35.6% yield, 99.8% purity). MS m/z (ESI): 423.2[M+H]⁺; ¹H NMR (400 MHz DMSO-d6), δ= 12.28 (br s, 1H), 10.73 (br s, 1H), 7.25 (br d, J = 7.5 Hz, 1H), 7.11 (br s, 1H), 6.43 (br s, 1H), 5.17 - 4.96 (m, 1H), 4.94 - 4.78 (m, 1H), 4.34 (s, 2H), 4.05 (s, 3H), 3.70 - 3.50 (m, 1H), 3.29 - 3.11 (m, 4H), 2.63 - 2.51 (m, 1H), 2.45 - 2.36 (m, 1H), 2.00 - 1.74 (m, 2H), 1.05 (d, J = 6.5 Hz, 6H); 19F NMR: (377MHz, DMSO-d6), δ=-188.59 (br s, 1F).

### Example 9

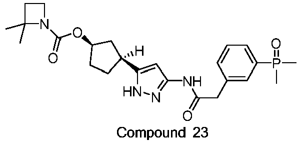

### Synthetic route:

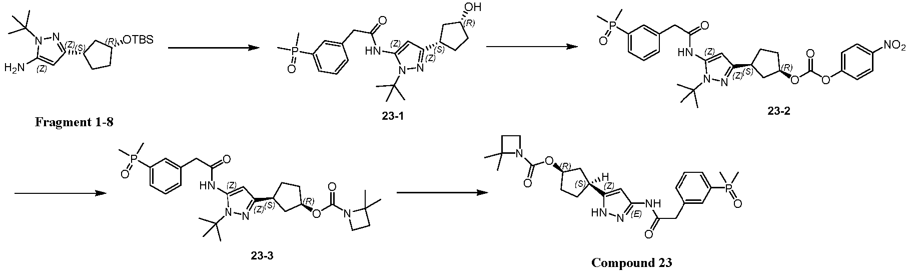

### Step 1: synthesis of compound 23-1

Compound 2-(3-(dimethylphosphoryl)phenyl)acetic acid (314 mg, 1.00 eq) and fragment 1-8 (500 mg, 1.00 eq) were dissolved in anhydrous N,N-dimethylformamide (8 mL), and the solution was added to a three-necked flask. Then, N-methylimidazole (364 mg, 354 µL, 3.00 eq) and N,N,N',N'-tetramethylchloroformamidinium hexafluorophosphate (498 mg, 1.78 mmol, 1.20 eq) were added in sequence. The reaction solution was heated to 100 °C and reacted for 6 h. The reaction system was then cooled to room temperature, and water (80 mL) was slowly added to the reaction solution at room temperature. The mixture was extracted with ethyl acetate (30 mL × 3). The organic phases were combined, washed with a saturated aqueous sodium chloride solution (30 mL), dried, and concentrated to dryness to give a crude residue. The crude product was separated and purified by preparative chromatography in an acidic condition to give compound 23-1 (184 mg, 32.9% yield, 93.8% purity, yellow oil). MS (ESI) m/z = 418.1 [M+H]⁺.

### Step 2: synthesis of compound 23-2

Compound 23-1(142 mg, 1.00 eq), p-nitrophenyl chloroformate (171 mg, 2.50 eq), 4-dimethylaminopyridine (4.16 mg, 0.10 eq), and pyridine (80.0 mg, 82.4 µL, 3.00 eq) were added to anhydrous tetrahydrofuran (2.5 mL) in sequence, and the reaction solution was heated to 75 °C and reacted for 22 h. The system was cooled to 30 °C, and the reaction solution was used directly in the next reaction. MS (ESI) m/z = 583.3 [M+H]⁺.

### Step 3: synthesis of compound 23-3

Compound 2,2-dimethylazetidine hydrochloride (82.6 mg, 2.00 eq) and N,N-diisopropylethylamine (131 mg, 177 µL, 3.00 eq) were dissolved in anhydrous tetrahydrofuran (3 mL), and the reaction solution was stirred at 25 °C for 15 min and then slowly added dropwise to the reaction solution from the previous step. The reaction solution was heated to 50 °C and reacted for 4 h, and then the system was cooled to room temperature. Water (5 mL) was slowly added to the reaction solution at room temperature, and the mixture was extracted with ethyl acetate (10 mL × 3). The organic phases were combined, dried, and concentrated to dryness to give a crude product. The crude product was separated and purified by preparative chromatography to give compound 23-3 (160 mg, 87.3% yield, 98.0% purity, pale yellow oil). MS (ESI) m/z = 529.5 [M+H]⁺.

### Step 4: synthesis of compound 23

Compound 23-3 (150 mg, 1.00 eq) was dissolved in trifluoroacetic acid (2 mL). The system was incubated for reaction at 70 °C for 12 h. The reaction solution was concentrated to dryness to give a crude product. The crude product was separated and purified by preparative chromatography to give compound 23 (white solid, 20.0 mg, 16.8% yield, 98.0% purity). MS (ESI) m/z = 473.3[M+H] ⁺. ¹H NMR (400 MHz, CDCl₃) δ 12.46 - 11.40 (m, 1H), 10.41 - 10.20 (m, 1H), 8.01 - 7.89 (m, 1H), 7.56 (br, 1H), 7.48 - 7.31 (m, 2H), 6.44 (m, 1H), 5.89 (s, 1H), 5.23 - 5.00 (m, 1H), 4. 80 - 4.66 (m, 1H), 3.66 (s, 2H), 3.38 - 3.08 (m, 3H), 2.50 - 2.28 (m, 1H), 2.17 (t, *J* = 6.7 Hz, 1H), 2.12 - 2.00 (m, 1H), 1.92 - 1.76 (m, 4H), 1.70 (br, 13.0 Hz, 6H), 1.67 - 1.61 (m, 2H), 1.21 (s, 4H).

### Example 10

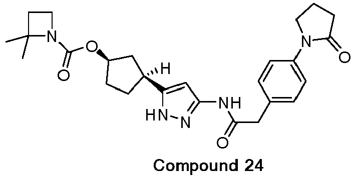

Referring to the synthesis methods of steps 1-4 in Example 9 (compound 23), compound 24 was synthesized. MS m/z (ESI): 480.1 [M+H]⁺. ¹H NMR (400 MHz, CDCl₃) δ 10.79 - 10.35 (m, 1H), 8.19 (br s, 1H), 7.49 (d, *J* = 8.5 Hz, 2H), 7.20 - 7.16 (m, 2H), 6.41 (br s, 1H), 5.07 - 4.99 (m, 1H), 3.74 (t, J = 7.0 Hz, 2H), 3.56 (s, 2H), 3.33 (t, *J =* 6.1 Hz, 2H), 3.11 (quin, J = 8.1 Hz, 1H), 2.53 (t, *J* = 8.1 Hz, 2H), 2.41 - 2.32 (m, 1H), 2.12 - 1.92 (m, 4H), 1.82 - 1.74 (m, 3H), 1.57 (t, *J =* 6.1 Hz, 2H), 1.25 (s, 6H).

### Example 11

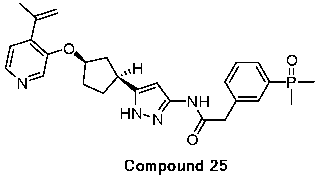

### Synthetic route:

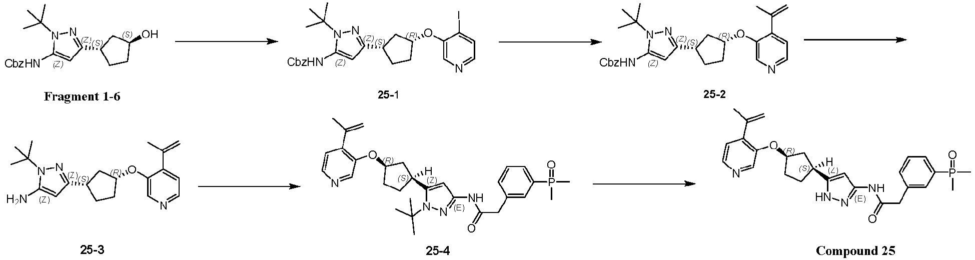

### Step 1: synthesis of compound 25-1

Tetrahydrofuran (52 mL), fragment 1-6 (5.20 g, 1.00 eq), 4-iodo-3-hydroxypyridine (3.53 g, 2.00 eq), and triphenylphosphine (5.71 g, 1.50 eq) were added into a three-necked flask in sequence, and the mixture was cooled to 0 °C. Then, diisopropyl azodicarboxylate (4.40 g, 1.50 eq) was added. The system was stirred at 20 °C for 12 h. Water (50 mL) was added to the reaction solution, and the mixture was extracted with ethyl acetate (50 mL × 2). The organic phases were combined, dried, and concentrated to dryness to give a crude product. The crude product was separated and purified by preparative chromatography to give compound 25-1 (off-white solid, 8.13 g, 93% purity). MS (ESI) m/z = 561 [M+H]⁺. ¹H NMR (400 MHz, CDCl₃) δ 8.08 (s, 1H), 7.85 (d, *J =* 5.0 Hz, 1H), 7.75 (d, *J =* 5.0 Hz, 1H), 7.37 (br s, 4H), 6.21 (br s, 1H), 5.20 (s, 2H), 4.98 (br d, *J =* 4.8 Hz, 1H), 3.23 - 3.13 (m, 1H), 2.69 - 2.59 (m, 1H), 2.18 - 1.98 (m, 6H), 1.58 (s, 9H).

### Step 2: synthesis of compound 25-2

Dimethoxyethane (24 mL), water (6 mL), compound 25-1 (3.00 g, 1.00 eq), isopropenylboronic acid pinacol ester (1.35 g, 2.00 eq), potassium carbonate (1.48 g, 2.00 eq), and tetrakis(triphenylphosphine)palladium(0) (309 mg, 0.05 eq) were added into a 250 mL three-necked flask in sequence, and the system was stirred at 100 °C for 12 h. Water (20 mL) was added to the reaction solution, and the mixture was extracted with ethyl acetate (50 mL × 2). The organic phases were combined, dried, and concentrated to dryness to give a crude product. The crude product was separated and purified by preparative chromatography to give compound 25-2 (yellow oil, 1.0 g, 99.2% purity). MS (ESI) m/z = 475.3 [M+H] ⁺.

### Step 3: synthesis of compound 25-3

Compound 25-2 (1.19 g, 2.50 mmol, 1.00 eq) was dissolved in acetonitrile (12.0 mL), and the reaction solution was transferred to an ice-water bath and cooled to 0 °C. Trimethylsilyl iodide (1.50 g, 7.51 mmol, 1.02 mL, 3.00 eq) was slowly added to the reaction solution. The ice-water bath was then removed, and the system was stirred for reaction at room temperature (20 °C) for 16 h. Water (40.0 mL) was added to the reaction solution, and the mixture was washed with petroleum ether (30.0 mL × 3). The aqueous phases were collected, combined, and extracted with ethyl acetate (30.0 mL). The organic phase was dried over anhydrous Na₂SO₄ and filtered, and the filtrate was concentrated in vacuum to give a crude product. The crude product was separated by preparative chromatography to give compound 25-3 (yellow oil, 850 mg, 20.6% yield, 67.3% purity). MS (ESI) m/z = 341.2 [M+H] +.

### Step 4: synthesis of compound 25-4

N,N-Dimethylformamide (3.00 mL), compound 25-3 (300 mg, 1.00 eq), N-methylimidazole (217 mg, 3.00 eq), and N,N,N,N-tetramethylchloroformamidinium hexafluorophosphate (370 mg, 1.50 eq) were added into a 10.00 mL reaction flask in sequence, and the system was incubated for reaction at 70 °C for 4 h in a nitrogen atmosphere. Water (20.0 mL) was added, and the mixture was extracted with ethyl acetate (20.0 mL × 2). The organic phase was washed with brine, dried, and concentrated to dryness to give a crude product. The crude product was separated and purified by preparative chromatography to give compound 25-4 (white solid, 25.0 mg, 5.20% yield, 98% purity). MS (ESI) m/z = 535.3 [M + H]⁺.

### Step 5: synthesis of compound 25

Compound 25-4 (20.0 mg, 1.00 eq) was dissolved in trifluoroacetic acid (1.00 mL). The system was reacted at 70 °C for 4 h. The reaction solution was concentrated to dryness to give a crude product. The crude product was separated and purified by preparative chromatography to give compound 25 (white solid, 6.00 mg, 32.2% yield, 96.0% purity). MS (ESI) m/z = 479.1 [M+H] ⁺. ¹H NMR (400 MHz, CDCl₃) δ 12.28 - 11.44 (m, 1H), 9.98 (br s, 1H), 8.19 (s, 1H), 8.15 (d, *J* = 4.8 Hz, 1H), 7.99 (br d, *J* = 12.1 Hz, 1H), 7.61 (br d, *J* = 7.0 Hz, 1H), 7.49 - 7.37 (m, 2H), 7.08 (d, *J* = 4.8 Hz, 1H), 6.45 (br s, 1H), 5.20 (br d, *J =* 12.4 Hz, 2H), 4.92 (br d, *J* = 3.1 Hz, 1H), 3.66 (s, 2H), 3.29 - 3.19 (m, 1H), 2.69 - 2.59 (m, 1H), 2.20 - 2.11 (m, 1H), 2.06 (s, 3H), 2.02 - 1.88 (m, 4H), 1.72 (d, *J* = 13.0 Hz, 6H).

### Example 12

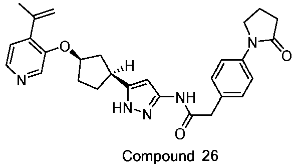

Referring to the synthesis methods of steps 1-5 in Example 11 (compound 25), compound 26 was synthesized. MS m/z (ESI)=486.2 [M+H]⁺. ¹H NMR (400 MHz, CDCl₃) δ 8.23 (s, 1H), 8.19 (d, *J* = 4.8 Hz, 1H), 8.02 (br s, 1H), 7.60 (br d, *J* = 8.1 Hz, 2H), 7.29 (br d, *J* = 8.3 Hz, 2H), 7.11 (d, *J* = 4.8 Hz, 1H), 6.45 (br s, 1H), 5.33 (s, 1H), 5.21 (s, 1H), 4.98 (br s, 1H), 3.85 (t, *J* = 7.0 Hz, 2H), 3.67 (s, 2H), 3.30 (quin, *J* = 7.8 Hz, 1H), 2.62 (t, *J* = 8.1 Hz, 2H), 2.53 (ddd, *J* = 5.9, 9.3, 14.6 Hz, 1H), 2.18 (quin, *J* = 7.5 Hz, 4H), 2.09 (s, 3H), 2.07 - 1.80 (m, 4H).

### Example 13

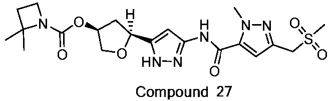

### Synthetic route:

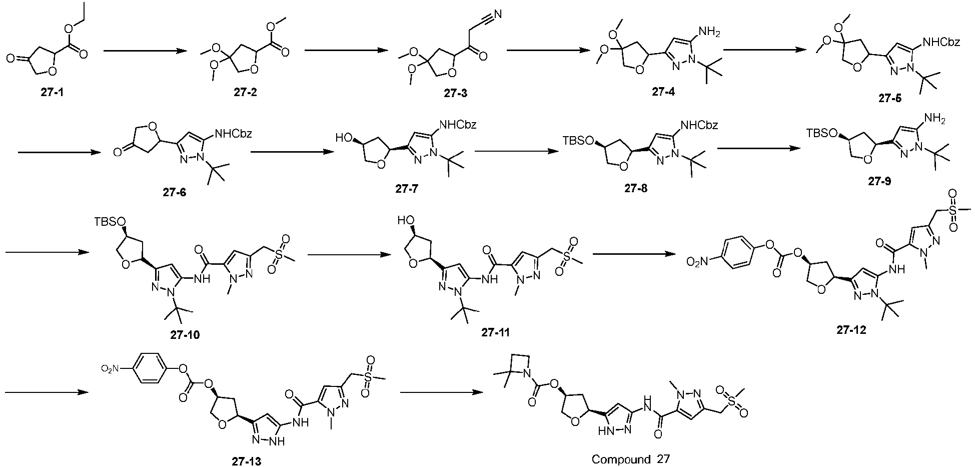

### Step 1: synthesis of compound 27-2

p-Toluenesulfonic acid (653.3 mg, 3.79 mmol) and trimethyl orthoformate (90.0 g, 848.55 mmol) were added to a solution of ethyl 4-oxotetrahydrofuran-2-carboxylate (20 g, 126.46 mmol) in methanol (200 mL) at 30 °C. The reaction solution was stirred at 30 °C for 12 h, quenched with a saturated aqueous NaHCO₃ solution (50 mL), concentrated at reduced pressure to remove the methanol, and then extracted with ethyl acetate (50 mL × 3). The organic phase was washed with brine, dried over anhydrous Na₂SO₄, and then concentrated at reduced pressure. The crude product was purified by separation with silica column chromatography (petroleum ether/ethyl acetate = 20/1 to 15/1) to give compound 27-2 (23 g, 120.93 mmol, 95.63% yield). ¹H NMR (CDCl₃, 400 MHz) δ 4.62 - 4.58 (m, 1H), 4.00 - 3.92 (m, 1H), 3.91 - 3.84 (m, 1H), 3.77 (s, 3H), 3.25 (s, 3H), 3.23 (s, 3H), 2.43 - 2.40 (m, 1H), 2.32 - 2.27 (m, 1H).

### Step 2: synthesis of compound 27-3

27-2 (19 g, 99.90 mmol) and acetonitrile (12.30 g, 299.70 mmol) were dissolved in tetrahydrofuran (100 mL), and then lithium bis(trimethylsilyl)amide (1 M, 199.80 mL) was slowly and dropwise added to the reaction solution at -78 °C. The mixture was stirred at -78 °C for 2 h, quenched with a saturated ammonium chloride solution (200 mL), and extracted with ethyl acetate (300 mL × 3). The organic phase was washed with brine (300 mL × 3), dried over anhydrous sodium sulfate, and concentrated at reduced pressure. The crude product was purified by separation with silica chromatography (petroleum ether/ethyl acetate = 6/1 to 2/1) to give compound 27-3 (20 g, 100.40 mmol, 83.02% yield). ¹H NMR (CDCl₃, 400 MHz) δ 4.51 (dd, *J₁* = 3.6 Hz, *J₂* = 9.6 Hz, 1H), 4.01 - 3.98 (m, 1H), 3.88 - 3.72 (m, 3H), 3.28 (s, 3H), 3.19 (s, 3H), 2.49 - 2.42 (m, 1H), 2.36 - 2.27 (m, 1H).

### Step 3: synthesis of compound 27-4

NaOH (3.86 g, 96.38 mmol) was added to a solution of tert-butylhydrazine hydrochloride (20 g, 96.38 mmol) in ethanol (150 mL), and after 1 h of stirring at 25 °C, compound 27-3 (16 g, 80.32 mmol) was added at 25 °C. The mixture was stirred at 50 °C for 5 h. The reaction solution was filtered, and the filtrate was concentrated at reduced pressure to give a crude product. The crude product was purified by separation with silica chromatography (petroleum ether/ethyl acetate = 10/1 to 4/1) to give compound 27-4 (23 g, 85.39 mmol, 85.05% yield). MS m/z (ESI) = 238.1 [M-OMe]⁺. ¹H NMR (CDCl₃, 400 MHz) δ 4.96 (dd, *J₁* = 6.8 Hz, *J₂* = 9.2 Hz, 1H), 3.98 (d, *J =* 9.2 Hz, 1H), 3.80 (d, *J =* 9.2 Hz, 1H), 3.67 - 3.37 (m, 2H), 3.28 (d, *J =* 2.0 Hz, 6H), 2.43 (dd, *J₁* = 6.8 Hz, *J₂* = 12.8 Hz, 1H), 2.25 (dd, *J₁* = 9.2 Hz, *J₂* = 12.8 Hz, 1H), 1.62 (s, 9H).

### Step 4: synthesis of compound 27-5

Compound 27-4 (23 g, 55.69 mmol, 1 eq) was dissolved in tetrahydrofuran (200 mL), and water (20 mL), sodium bicarbonate (14.04 g, 167.08 mmol), and benzyl chloroformate (14.25 g, 83.54 mmol, 1.5 eq) were added at 25 °C. The mixture was stirred at 25 °C for 12 h. The reaction solution was added to water (100 mL), and the mixture was extracted with ethyl acetate (100 mL × 3). The organic phase was washed with brine (100 mL × 3), dried over anhydrous sodium sulfate, and concentrated at reduced pressure to give a crude product. The crude product was purified by separation with silica chromatography (petroleum ether/ethyl acetate = 10/1 to 3/1) to give compound 27-5 (30 g, 74.35 mmol, 87.07% yield). MS m/z (ESI) = 404.3 [M+H]⁺. ¹H NMR (CDCl₃, 400 MHz) δ 7.39 ~ 7.32 (m, 5H), 6.35 ~ 6.27 (m, 1H), 6.27 (br s, 1H), 5.19 (s, 2H), 5.01 (dd, *J₁* = 6.4 Hz, *J₂* = 9.4 Hz, 1H), 3.97 (d, *J =* 9.2 Hz, 1H), 3.81 (d, *J =* 9.2 Hz, 1H), 3.27 (d, *J =* 2.0 Hz, 6H), 2.47 - 2.39 (m, 1H), 2.35 - 2.26 (m, 1H), 1.57 (s, 9H).

### Step 5: synthesis of compound 27-6

An aqueous hydrochloric acid solution (12 M, 20 mL) was added to a solution of compound 27-5 (30 g, 74.35 mmol) in tetrahydrofuran (150 mL), and the system was stirred at 25 °C for 1 h. The reaction solution was added to water (100 mL), and the mixture was extracted with ethyl acetate (100 mL × 3). The organic phase was washed with brine (100 mL × 3), dried over anhydrous Na₂SO₄, and then concentrated at reduced pressure to give compound 27-6 (26 g, 72.75 mmol, 97.84% yield). MS m/z (ESI) = 358.0 [M+H]⁺. ¹H NMR (CDCl₃, 400 MHz) δ 7.40 ~ 7.35 (m, 5H), 6.35 ~ 6.31 (m, 2H), 5.34 (t, *J* = 7.2 Hz, 1H), 5.21 (s, 2H), 4.09 (d, *J =* 8.8 Hz, 1H), 3.94 (d, *J =* 8.8 Hz, 1H), 2.97 - 2.86 (m, 1H), 2.83 - 2.72 (m, 1H), 1.59 (s, 9H).

### Step 6: synthesis of compound 27-7

Compound 27-6 (15 g, 41.97 mmol) was dissolved in an ethanol solution (150 mL), and NaBH₄ (7.94 g, 209.85 mmol) was slowly added at 0 °C. The mixture was stirred at 25 °C for 2 h. The reaction mixture was quenched with a saturated ammonium chloride solution (300 mL) and extracted with ethyl acetate (300 mL × 3). The organic phase was washed with brine (300 mL × 3) and dried over anhydrous Na₂SO₄, and then the filtrate was concentrated at reduced pressure to give a crude product. The crude product was purified by high-performance liquid chromatography (0.1% FA/MeCN) and then separated by SFC to give compound 27-7 (2.5 g, 6.89 mmol, 16.41% yield). MS m/z (ESI) = 360.2 [M+H]⁺. ¹H NMR (400 MHz, CDCl₃) δ 7.39 ~ 7.34 (m, 5H), 6.26 ~ 6.22 (m, 2H), 5.20 (s, 2H), 5.14 (dd, *J₁* = 6.4 Hz, *J₂* = 9.2 Hz, 1H), 4.63 ~ 4.61 (m, 1H), 4.12 (dd, *J₁* = 4.4 Hz, *J₂* = 9.6 Hz, 1H), 3.80 (d, *J =* 10.0 Hz, 1H), 2.38 - 2.30 (m, 1H), 2.29 - 2.19 (m, 1H), 1.59 (s, 9H).

### Step 7: synthesis of compound 27-8

Compound 27-7 (6 g, 16.69 mmol, 1 eq) and imidazole (5.68 g, 83.47 mmol, 5 eq) were dissolved in dichloromethane (50 mL), and tert-butyldimethylchlorosilane (5.03 g, 33.39 mmol, 2 eq) was added at 0 °C. The mixture was stirred at 25 °C for 2 h. The reaction solution was quenched with a saturated aqueous sodium bicarbonate solution (50 mL) and extracted with ethyl acetate (50 mL × 3). The organic phase was washed with brine (50 mL × 3) and dried over anhydrous Na₂SO₄, and then the filtrate was concentrated at reduced pressure to give a crude product. The crude product was purified by separation with silica chromatography (petroleum ether/ethyl acetate = 10/1 to 3/1) to give compound 27-8 (7 g, 14.78 mmol, 88.52% yield). MS m/z (ESI) = 474.3 [M+H]⁺. ¹H NMR (400 MHz, CDCl₃) δ 7.39 ~ 7.34 (m, 5H), 6.35 (br s, 1H), 6.23 ~ 6.19 (m, 1H), 5.20 (s, 2H), 4.85 (t, *J =* 7.6 Hz, 1H), 4.56 - 4.52 (m, 1H), 3.89 - 3.86 (m, 1H), 3.84 - 3.80 (m, 1H), 2.55 - 2.43 (m, 1H), 2.11 - 2.08 (m, 1H), 1.58 (s, 9H), 0.89 (s, 9H), 0.07 (s, 6H).

### Step 8: synthesis of compound 27-9

Palladium on carbon (1 g, 10% purity) was added to a solution of compound 27-8 (7 g, 14.78 mmol, 1 eq) in methanol (20 mL). The mixture was stirred at 25 °C for 12 h in a hydrogen atmosphere (20 psi). The reaction solution was filtered through celite, and the filtrate was concentrated at reduced pressure to give compound 27-9 (4.5 g, 89.6% yield). MS m/z (ESI) = 340.3 [M+H]⁺. ¹H NMR (CDCl₃, 400 MHz) δ 5.72 (s, 1H), 4.78 (t, *J =* 8.0 Hz, 1H), 4.55 - 4.50 (m, 1H), 3.86 - 3.79 (m, 2H), 3.69 - 3.17 (m, 2H), 2.52 - 2.45 (m, 1H), 2.06 - 2.00 (m, 1H), 1.62 (s, 9H), 0.90 (s, 9H), 0.08 (s, 6H).

### Step 9: synthesis of compound 27-10

4-Dimethylaminopyridine (323.8 mg, 2.65 mmol, 1.5 eq), 1-ethyl-(3-dimethylaminopropyl)carbodiimide hydrochloride (338.7 mg, 1.77 mmol, 1 eq), and compound 27-9 (0.6 g, 1.77 mmol, 1 eq) were added to a solution of fragment 4-3 (385.6 mg, 1.77 mmol, 1 eq) in dichloromethane (6 mL). The mixture was stirred at 25 °C for 12 h. The mixture was added to water (10 mL) and extracted with ethyl acetate (10 mL × 3). The organic phase was washed with brine (10 mL × 3), dried over anhydrous Na₂SO₄, and then concentrated at reduced pressure to give a crude product. The crude product was purified by separation with silica chromatography (petroleum ether/ethyl acetate = 5/1 to 1/1) to give compound 27-10 (0.6 g, 1.11 mmol, 62.91% yield). MS m/z (ESI) = 540.3 [M+H]⁺. ¹H NMR (400 MHz, CDCl₃) δ 7.63 (br s, 1H), 6.82 (br s, 1H), 6.44 (s, 1H), 4.89 (t, *J =* 7.6 Hz, 1H), 4.57 - 4.54 (m, 1H), 4.32 (s, 2H), 4.20 (s, 3H), 3.89 - 3.86 (m, 1H), 3.82 - 3.78 (m, 1H), 2.92 (s, 3H), 2.57 - 2.49 (m, 1H), 2.15 - 2.11 (m, 1H), 1.64 (s, 9H), 0.89 (s, 9H), 0.08 (s, 6H).

### Step 10: synthesis of compound 27-11

Tetrabutylammonium fluoride (1 M, 2.22 mL, 2 eq) was added to a solution of compound 27-10 (0.6 g, 1.11 mmol, 1 eq) in tetrahydrofuran (10 mL). The system was stirred at 25 °C for 1 h. The mixture was added to water (10 mL) and extracted with ethyl acetate (10 mL × 3). The organic phase was washed with brine (10 mL × 3), dried over anhydrous Na₂SO₄, and then concentrated at reduced pressure to give compound 27-11 (0.4 g, 940.07 µmol, 84.57% yield). MS m/z (ESI) = 426.2 [M+H]⁺. ¹H NMR (400 MHz, CDCl₃) δ 8.00 (br s, 1H), 6.90 (br s, 1H), 6.31 (s, 1H), 5.11 (d, *J =* 8.4 Hz, 1H), 4.50 ~ 4.47 (m, 1H), 4.31 (s, 2H), 4.20 (s, 3H), 3.99 (s, 2H), 2.92 (s, 3H), 2.52 - 2.40 (m, 1H), 2.37 - 2.28 (m, 1H), 1.62 (s, 9H).

### Step 11: synthesis of compound 27-12

At 0 °C, sodium carbonate (498.2 mg, 4.70 mmol, 5 eq) and 4-nitrophenyl chloroformate (568.5 mg, 2.82 mmol, 3 eq) were added to a solution of compound 27-11 (0.4 g, 940.07 µmol, 1 eq) in dichloromethane (5 mL). The mixture was stirred at 25 °C for 12 h. The reaction solution was filtered, and the filtrate was concentrated at reduced pressure to give a crude product. The crude product was purified by separation with silica chromatography (petroleum ether/ethyl acetate = 10/1 to 3/1) to give compound 27-12 (0.35 g, 592.61 µmol, 63.04% yield). MS m/z (ESI) = 591.3[M+H]⁺. ¹H NMR (400 MHz, CDCl₃) δ 8.28 (dd, *J₁* = 2.0 Hz, *J₂ =* 7.2 Hz, 2H), 7.52 (br s, 1H), 7.40 (dd, *J₁* = 2.0 Hz, *J₂* = 7.2 Hz, 2H), 6.83 (br s, 1H), 6.50 (s, 1H), 5.43 - 5.38 (m, 1H), 4.97 (t, *J =* 7.6 Hz, 1H), 4.33 (s, 2H), 4.26 (d, *J =* 11.2 Hz, 1H), 4.21 (s, 3H), 3.98 (dd, *J₁* = 4.8 Hz, *J₂* = 11.2 Hz, 1H), 2.92 (s, 3H), 2.82 - 2.75 (m, 1H), 2.52 - 2.43 (m, 1H), 1.66 (s, 9H).

### Step 12: synthesis of compound 27-13

Compound 27-12 (0.35 g, 592.61 µmol, 1 eq) was dissolved in formic acid (4 mL), and the mixture was stirred at 75 °C for 1 h. The reaction solution was concentrated at reduced pressure to give compound 27-13 (0.3 g, 98% yield). MS m/z (ESI) = 535.2[M+H]⁺.

### Step 13: synthesis of compound 27

At 25 °C, N,N-diisopropylethylamine (217.6 mg, 1.68 mmol, 3 eq) and compound 27-13 (0.3 g, 561.27 µmol, 1 eq) were added to a solution of 2,2-dimethylazetidine hydrochloride (68.3 mg, 561.27 µmol, 1 eq, HCl) in tetrahydrofuran (5 mL). The system was stirred at 25 °C for 1 h. The mixture was added to water (10 mL) and extracted with ethyl acetate (10 mL × 3). The organic phase was washed with brine (10 mL × 3), dried over anhydrous Na₂SO₄, and concentrated at reduced pressure. The crude product was purified by separation with silica chromatography (dichloromethane/methanol = 15/1 to 10/1) to give compound 27 (0.1 g, 208.10 µmol, 51% yield). MS m/z (ESI) = 481.2 [M+H]⁺. ¹H NMR (, 400 MHz, DMSO-*d₆*) δ 12.50 (br s, 1H), 10.93 (br s, 1H), 7.22 (s, 1H), 6.58 (s, 1H), 5.21 - 5.12 (m, 1H), 4.99 ~ 4.95 (m, 1H), 4.47 (s, 2H), 4.08 (s, 3H), 3.91 - 3.81 (m, 2H), 3.75 - 3.62 (m, 2H), 3.00 (s, 3H), 2.69 - 2.55 (m, 1H), 2.10 - 2.05 (m, 1H), 1.95 - 1.84 (m, 2H), 1.38 (d, *J =* 4.8 Hz, 3H), 1.26 (s, 3H).

Referring to the synthesis methods of steps 1-13 in Example 13 (compound 27), compounds listed in the following table were synthesized:

| Compound No. | Structure | MS m/z | Nuclear magnetic resonance |
|---|---|---|---|
| Compou nd 28 | 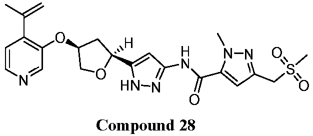 | 467.1 [M+H]⁺ | ¹H NMR (400 MHz, CDCl₃) δ 11.34 (br s, 1H), 10.75 - 9.69 (m, 1H), 7.06 (s, 1H), 6.76 (s, 1H), 5.16 (br s, 1H), 4.73 (br s, 1H), 4.34 - 4.25 (m, 2H), 4.22 (s, 3H), 3.86 - 3.72 (m, 1H), 3.25 (q, *J =* 8.2 Hz, 1H), 3.13 (q, *J =* 7.4 Hz, 2H), 2.53 - 2.42 (m, 1H), 2.15 - 2.04 (m, 1H), 1.89 (br s, 2H), 1.87 - 1.76 (m, 2H), 1.46 (t, *J* = 7.4 Hz, 3H), 1.17 - 1.12 (m, 6H). |
| Compou nd 30 | 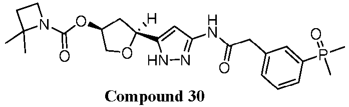 | 475.2 [M+H]⁺ | ¹H NMR (400 MHz, CDCl₃) δ 8.51 - 8.42 (m, 1H), 7.85 - 7.82 (m, 1H), 7.60 - 7.27 (m, 3H), 6.61 - 6.54 (m, 1H), 5.35 - 5.29 (m, 1H), 5.11 - 5.08 (m, 1H), 4.10 - 3.96 (m, 2H), 3.78 - 3.74 (m, 3H), 3.69 - 3.66 (m, 1H), 2.70 - 2.58 (m, 1H), 2.25 - 2.19 (m, 1H), 1.97 - 1.93 (m, 2H), 1.78 - 1.74 (m, 6H), 1.44 (s, 3H), 1.22 (d, *J* = 4.8 Hz, 3H). |
| Compound 31 | 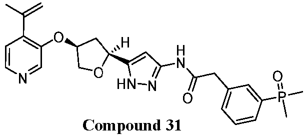 | 481.2 [M+H]⁺ | ¹H NMR (400 MHz, CDCl₃) δ 8.24 - 8.16 (m, 3H), 7.78 (d, *J =* 12.0 Hz, 1H), 7.63 - 7.48 (m, 3H), 7.11 (d, *J* = 4.0 Hz, 1H), 6.57 - 6.56 (m, 1H), 5.28 (s, 1H), 5.17 - 5.14 (m, 3H), 4.28 (d, *J =* 12.0 Hz, 1H), 4.03 (dd, *J₁* = 4.0 Hz, *J₂* =10.4 Hz, 1H), 3.75 (s, 2H), 2.81 - 2.73 (m, 1H), 2.34 (dd, *J₁* = 4.4 Hz, *J₂* =13.2 Hz, 1H), 2.03 (s, 3H), 1.77 (s, 3H), 1.74 (s, 3H). |

### Example 14

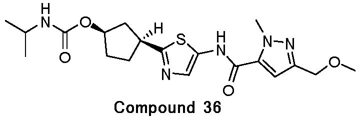

### Synthetic route:

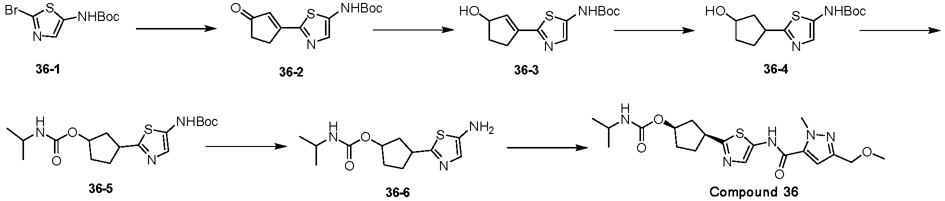

### Step 1: synthesis of compound 36-2

At 25 °C, 3-(4,4,5,5-tetramethyl-1,3,2-dioxaborolan-2-yl)cyclopent-2-en-1-one (4.84 g, 23.28 mmol) and potassium carbonate (7.43 g, 53.73 mmol) were added to a mixed solution of compound 36-1 (5 g, 17.91 mmol) in dioxane (90 mL) and water (9 mL), and the mixture was degassed and purged thrice with nitrogen. [1,1'-Bis(diphenylphosphino)ferrocene]palladium(II) dichloride (1.31 g, 1.79 mmol) was added to the reaction mixture, and after 3 purges with nitrogen, the reaction mixture was stirred at 90 °C for 12 h in a nitrogen atmosphere. The reaction mixture was poured into water (100 mL) and then extracted with ethyl acetate (50 mL × 4), and the combined organic phases were washed with saturated brine (50 mL × 3), dried over anhydrous sodium sulfate, and filtered and concentrated at reduced pressure to give a crude product. The crude product was purified by column chromatography (SiO₂, petroleum ether/ethyl acetate = 3/1 to 0/1) to give compound 36-2 (brown solid, 5 g, 83.64% yield). ¹H NMR (400 MHz, CDCl₃) δ 7.54 - 7.51 (m, 1H), 6.56 - 6.54 (m, 1H), 3.17 (s, 2H), 2.61 - 2.56 (m, 2H), 1.55 (s, 9H).

### Step 2: synthesis of compound 36-3

At 0 °C, sodium borohydride (860 mg, 22.73 mmol) was added to a solution of compound 36-2 (5 g, 17.84 mmol, 1 eq) in methanol (150 mL). The reaction mixture was stirred at 0 °C for 0.5 h. The reaction mixture was poured into water (200 mL) to quench the reaction and then extracted with ethyl acetate (200 mL × 4), and the combined organic layers were washed with saturated brine (100 mL × 3), dried over anhydrous sodium sulfate, and filtered and concentrated at reduced pressure to give a residue. The residue was purified by column chromatography (SiO₂, petroleum ether/ethyl acetate = 4/1 to 0/1) to give compound 36-3 (yellow solid, 3.5 g, 59% yield). ¹H NMR (400 MHz, CDCl₃) δ 7.32 (s, 1H), 7.17 - 7.04 (m, 1H), 6.34 (q, *J =* 2.0 Hz, 1H), 5.07 - 4.99 (m, 1H), 3.07 - 2.93 (m, 1H), 2.81 - 2.69 (m, 1H), 2.53 - 2.41 (m, 1H), 1.93 - 1.83 (m, 1H), 1.53 (s, 9H).

### Step 3: synthesis of compound 36-4

In an argon atmosphere, wet palladium on carbon (1.50 g, 1.41 mmol, 10% purity) was added to a solution of compound 36-3 (3.5 g, 12.40 mmol) in methanol (30 mL). The reaction solution was degassed and purged thrice with argon and hydrogen. The reaction mixture was stirred at 25 °C for 5 h in a hydrogen atmosphere (50 Psi). The reaction solution was filtered through celite and concentrated at reduced pressure. The obtained crude product was purified by column chromatography (silica gel column, petroleum ether/ethyl acetate = 5/1 to 0/1) to give compound 36-4 (pale yellow solid, 3.33 g, 90.69% yield). ¹H NMR (400 MHz, CDCl₃) δ 7.20 - 7.17 (m, 1H), 4.54 - 4.38 (m, 1H), 3.76 - 3.63 (m, 1H), 3.57 - 3.44 (m, 1H), 2.40 - 2.08 (m, 3H), 2.03 - 1.81 (m, 3H), 1.52 (s, 9H).

### Step 4: synthesis of compound 36-5

At 18 °C, cuprous chloride (1.16 g, 11.71 mmol, 280.02 µL) and 2-isocyanatopropane (1.10 g, 12.88 mmol, 1.26 mL) were added to a solution of compound 36-4 (3.33 g, 11.71 mmol) in N,N-dimethylformamide (100 mL). The reaction mixture was stirred at 25 °C for 5 h. The reaction mixture was poured into water (200 mL) for dilution and then extracted with ethyl acetate (100 mL × 4), and the combined organic layers were washed with saturated brine (50 mL × 3), dried over anhydrous sodium sulfate, and filtered and concentrated at reduced pressure. The crude product was purified by column chromatography (silica gel column, petroleum ether/ethyl acetate = 5/1 to 0/1) to give compound 36-5 (dark brown oil, 4.25 g, 9.66 mmol, 82.51% yield). ¹H NMR (400 MHz, CDCl₃) δ 8.06 - 7.99 (m, 2H), 7.16 - 7.06 (m, 1H), 5.29 - 5.13 (m, 1H), 4.63 - 4.39 (m, 1H), 4.12 - 3.94 (m, 1H), 3.50 - 3.31 (m, 1H), 2.96 (s, 6H), 2.91 - 2.87 (m, 6H), 2.68 - 2.49 (m, 1H), 2.24 - 2.15 (m, 1H), 2.11 - 1.88 (m, 4H), 1.51 (s, 9H), 1.15 (d, *J* = 6.4 Hz, 6H).

### Step 5: synthesis of compound 36-6

At 18 °C, hexafluoroisopropanol (955.08 mg, 5.68 mmol, 160 mL) was added to compound 36-5 (2.1 g, 5.68 mmol). The system was stirred at 60 °C for 60 h. The reaction mixture was filtered and concentrated at reduced pressure to give a residue, and the solid residue was diluted with water (100 mL) and then extracted with ethyl acetate (50 mL × 3). The combined organic layers were washed with saturated brine (50 mL × 1), dried over anhydrous sodium sulfate, and filtered and concentrated at reduced pressure. The crude product was purified by column chromatography (silica gel column, petroleum ether/ethyl acetate = 2/1 to 0/1) to give compound 36-6 (yellow liquid, 4.8 g, 77.2% yield). ¹H NMR (400 MHz, CDCl₃) δ 6.91 (s, 1H), 5.27 - 5.13 (m, 1H), 4.55 - 4.40 (m, 1H), 3.91 - 3.70 (m, 2H), 3.59 - 3.47 (m, 1H), 3.39 - 3.29 (m, 1H), 2.63 - 2.52 (m, 1H), 2.29 - 2.13 (m, 2H), 1.98 - 1.90 (m, *J* = 5.1 Hz, 3H), 1.16 (dd, *J* = 4.4, 6.4 Hz, 6H).

### Step 6: synthesis of compound 36

At 20 °C, 4-dimethylaminopyridine (605.48 mg, 4.96 mmol) and 1-ethyl-(3-dimethylaminopropyl)carbodiimide hydrochloride (950.09 mg, 4.96 mmol) were added to a solution of 3-(methoxymethyl)-1-methyl-1H-pyrazole-5-carboxylic acid (562.24 mg, 3.30 mmol) in dichloromethane (35 mL). The reaction solution was then cooled to 0 °C and stirred for 0.25 h. Then, compound 36-6 (890 mg, 3.30 mmol) was added at 0 °C, and the mixture was then stirred at 25 °C for 5 h. The reaction mixture was poured into water (100 mL) to quench the reaction and then extracted with ethyl acetate (100 mL × 6), and the combined organic layers were washed with saturated brine (50 mL × 6), dried over anhydrous sodium sulfate, and filtered and concentrated at reduced pressure to give a residue. The crude product was separated by SFC to give compound 36 (pale yellow solid, 550 mg, 39.10% yield). MS m/z (ESI) = 422.1 [M+H]⁺. ¹H NMR (400 MHz, CDCl₃) δ 8.33 - 8.28 (m, 1H), 7.48 - 7.44 (m, 1H), 6.72 (s, 1H), 5.32 - 5.24 (m, 1H), 4.51 - 4.43 (m, 3H), 4.23 - 4.20 (m, 3H), 3.88 - 3.75 (m, 1H), 3.69 - 3.57 (m, 1H), 3.43 (s, 3H), 2.37 - 2.10 (m, 4H), 1.98 - 1.80 (m, 2H), 1.17 (d, *J =* 6.8 Hz, 6H).

### Example 15

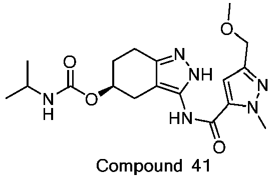

### Synthetic route:

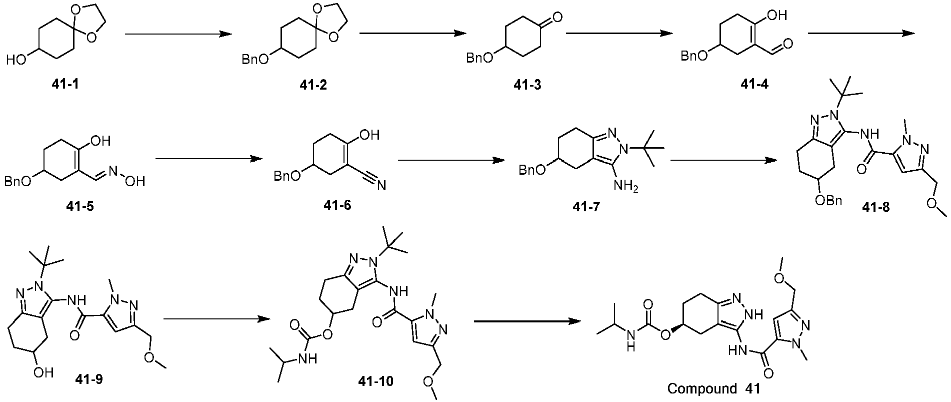

### Step 1: synthesis of compound 41-2

Sodium hydrogen (21.24 g, 530.99 mmol, 60% purity) was added to a solution of compound 41-1 (40 g, 252.85 mmol) in tetrahydrofuran (200 mL) at 0 °C, and after the reaction solution was stirred at 0 °C for 1.5 h, benzyl bromide (51.90 g, 303.42 mmol, 36.04 mL) was slowly and dropwise added. After the addition, the mixture was warmed to 25 °C and stirred for 12 h. After the reaction was completed, the system was quenched with a saturated aqueous NH₄Cl solution (300 mL), diluted with water (100 mL), and then extracted with ethyl acetate (500 mL × 3). The organic phase was washed with saturated brine (500 mL), dried over anhydrous Na₂SO₄, filtered, and then concentrated at reduced pressure. The crude product was purified by separation with silica column chromatography (ethyl acetate: petroleum ether = 0-20%) to give compound 41-2 (55 g, 221.49 mmol, 93% yield). ¹H NMR (400 MHz, DMSO-*d₆*) δ 7.41 - 7.20 (m, 5H), 4.47 (s, 2H), 3.88 - 3.80 (m, 4H), 3.54 - 3.42 (m, 1H), 1.83 - 1.73 (m, 2H), 1.73 - 1.59 (m, 4H), 1.52 - 1.42 (m, 2H).

### Step 2: synthesis of compound 41-3

p-Toluenesulfonic acid monohydrate (114.90 g, 604.07 mmol) was added to a solution of compound 41-2 (30 g, 120.81 mmol) in methanol (300 mL) and water (30 mL) at 20 °C, and the reaction solution was stirred at 0 °C for 12 h. After the reaction was completed, the residue obtained after concentration at reduced pressure was slowly poured into water (500 mL), and the mixture was extracted with ethyl acetate (500 mL × 3). The organic phase was washed with brine (500 mL × 3), dried over anhydrous sodium sulfate, filtered, and concentrated at reduced pressure. The crude product was purified by separation with silica chromatography (ethyl acetate: petroleum ether = 0-30%) to give compound 41-3 (23 g, 112.60 mmol, 93.2% yield). ¹H NMR (400 MHz, CDCl₃) δ 7.41 - 7.32 (m, 4H), 7.31 - 7.25 (m, 1H), 4.56 (s, 2H), 3.83 - 3.76 (m, 1H), 2.47 - 2.35 (m, 2H), 2.28 - 2.16 (m, 2H), 2.00 - 1.95 (m, 4H).

### Step 3: synthesis of compound 41-4

At 0 °C, sodium methoxide (60.83 g, 337.80 mmol, 30% purity) was added to a solution of compound 41-3 (23 g, 112.60 mmol) in toluene (200 mL). After the reaction solution was stirred at 0 °C for 10 min, ethyl formate (50.5 g, 675.60 mmol) was slowly added dropwise. After the addition, the reaction system was stirred at 20 °C for 12 h. After the reaction was completed, the system was quenched with water (500 mL) and then extracted with ethyl acetate (500 mL × 3). The organic phase was washed with saturated brine (500 mL × 2), dried over anhydrous Na₂SO₄, filtered, and then concentrated at reduced pressure. The crude product was purified by separation with silica column chromatography (ethyl acetate:petroleum ether = 0-30%) to give compound 41-4 (23.7 g, 90.6% yield). ¹H NMR (400 MHz, CDCl₃) δ 14.44 (br. s, 1H), 8.55 (s, 1H), 7.38 - 7.33 (m, 4H), 7.32 - 7.28 (m, 1H), 4.65 - 4.53 (m, 2H), 3.85 - 3.76 (m, 1H), 2.69 - 2.55 (m, 2H), 2.53 - 2.46 (m, 1H), 2.45 - 2.33 (m, 1H), 1.96 - 1.88 (m, 2H).

### Step 4: synthesis of compound 41-5

Compound 41-4 (23.7 g, 102.03 mmol) and hydroxylamine hydrochloride (7.80 g, 112.24 mmol) were added to acetic acid (150 mL). The reaction solution was stirred at 100 °C for 3 h and added to water (200 mL). The mixture was adjusted to pH 7 with a saturated NaHCO₃ solution and then extracted with ethyl acetate (500 mL × 3). The organic phase was washed with brine (500 mL × 3), dried over anhydrous sodium sulfate, filtered, and concentrated at reduced pressure to give a crude product. The crude product was purified by separation with silica chromatography (ethyl acetate:petroleum ether = 10%-30%) to give compound 41-5 (6.7 g, 26.6% yield). ¹HNMR (400 MHz, CDCl₃) δ 8.15 - 8.01 (m, 1H), 7.39 - 7.25 (m, 5H), 4.68 - 4.52 (m, 2H), 3.99 - 3.82 (m, 1H), 3.08 - 2.54 (m, 4H), 2.19 - 1.94 (m, 2H).

### Step 5: synthesis of compound 41-6

At 0 °C, sodium methoxide (6.34 g, 35.22 mmol, 30% purity) was added to a solution of compound 41-5 (6.7 g, 27.09 mmol) in toluene (70 mL). The reaction solution was stirred at 25 °C for 12 h. After the reaction was completed, the system was quenched with water (300 mL) and then extracted with ethyl acetate (150 mL × 3). The organic phase was washed with saturated brine (1000 mL × 2), dried over anhydrous Na₂SO₄, filtered, and then concentrated at reduced pressure. The crude product was purified by separation with silica column chromatography (ethyl acetate:petroleum ether = 10-30%) to give compound 41-6 (4.8 g, 77.3% yield). ¹H NMR (400 MHz, DMSO-*d₆*) δ 10.37 (br. s, 1H), 7.37 - 7.25 (m, 5H), 4.51 (s, 2H), 3.75 - 3.61 (m, 1H), 2.47 - 2.37 (m, 1H), 2.30 - 2.13 (m, 3H), 1.88 - 1.70 (m, 2H).

### Step 6: synthesis of compound 41-7

After N,N-diisopropylethylamine (4.17 g, 32.28 mmol) was added dropwise to a solution of tert-butylhydrazine hydrochloride in ethanol (40 mL) and the mixture was stirred for 1 h at 25 °C, compound 41-6 (4.8 g, 20.94 mmol) was added to the reaction solution, and the system was stirred at 90 °C for 3 h. After the reaction was completed, the system was quenched by adding water (200 mL), and the mixture was extracted with ethyl acetate (200 mL × 3). The organic phase was washed with brine (200 mL × 2) and dried over anhydrous Na₂SO₄, and then the filtrate was concentrated at reduced pressure to give a crude product. The crude product was purified by separation with silica column chromatography (ethyl acetate: petroleum ether = 10%-100%) to give compound 41-7 (4 g, 13.36 mmol, 82.8% yield). ¹H NMR (400 MHz, DMSO-*d₆*) δ 7.40 - 7.31 (m, 4H), 7.31 - 7.23 (m, 1H), 4.55 (s, 2H), 4.46 (s, 2H), 3.76 - 3.64 (m, 1H), 2.73 - 2.62 (m, 1H), 2.49 - 2.46 (m, 1H), 2.42 - 2.32 (m, 1H), 2.27 - 2.18 (m, 1H), 1.98 - 1.89 (m, 1H), 1.80 - 1.67 (m, 1H), 1.48 (s, 9H).

### Step 7: synthesis of compound 41-8

Compound 41-7 (2 g, 6.68 mmol) and 5-(methoxymethyl)-2-methylpyrazole-3-carboxylic acid (1.48 g, 8.68 mmol) were dissolved in dichloromethane (40 mL), and N,N-diisopropylethylamine (3.45 g, 26.72 mmol) and propylphosphonic anhydride (50% solution in ethyl acetate, 12.65 g, 19.88 mmol) were slowly added at 0 °C. The mixture was stirred at 25 °C for 36 h. The reaction solution was quenched with water (100 mL), and the mixture was extracted with ethyl acetate (100 mL × 3). The organic phase was washed with brine (100 mL × 2), dried over anhydrous Na₂SO₄, and filtered, and the filtrate was concentrated at reduced pressure to give a crude product. The crude product was purified by separation with silica chromatography (ethyl acetate:petroleum ether = 10%-100%) to give compound 41-8 (1.6 g, 53.0% yield). ¹H NMR (400 MHz, DMSO-*d₆*) δ 9.84 (br. s, 1H), 7.38 - 7.29 (m, 4H), 7.29 - 7.23 (m, 1H), 7.03 (s, 1H), 4.58 - 4.49 (m, 2H), 4.37 (s, 2H), 4.05 - 4.02 (m, 3H), 3.84 - 3.73 (m, 1H), 3.29 (s, 3H), 2.72 - 2.53 (m, 3H), 2.35 - 2.26 (m, 1H), 1.98 - 1.82 (m, 2H), 1.50 (s, 9H).

### Step 7: synthesis of compound 41-9

Palladium on carbon (0.16 g, 10% purity) was added to a solution of compound 41-8 (1.6 g, 3.54 mmol) in methanol (20 mL). The reaction solution was stirred for 1 h in a hydrogen atmosphere (30 psi). After the reaction was completed, the reaction solution was filtered through celite, and the filtrate was concentrated at reduced pressure to give compound 41-9 (1.2 g, 93.7% yield). ¹H NMR (400 MHz, DMSO-*d₆*) δ 9.82 (br. s, 1H), 7.02 (s, 1H), 4.37 (s, 2H), 4.03 (s, 3H), 3.88 - 3.79 (m, 2H), 3.29 (s, 3H), 2.68 - 2.54 (m, 2H), 2.48 - 2.42 (m, 1H), 2.15 - 2.03 (m, 1H), 1.94 - 1.81 (m, 1H), 1.71 - 1.59 (m, 1H), 1.49 (s, 9H).

### Step 8: synthesis of compound 41-10

Cuprous chloride (273.90 mg, 2.77 mmol) and isopropyl isocyanate (323.8 mg, 2.65 mmol) were added to a solution of compound 41-9 (1.2 g, 3.32 mmol) in N,N-dimethylformamide (5 mL). The mixture was stirred at 25 °C for 3 h. The mixture was added to water (100 mL) and extracted with ethyl acetate (100 mL × 3). The organic phase was washed with brine (100 mL × 3) and dried over anhydrous Na₂SO₄, filtered, and concentrated at reduced pressure to give a crude product. The crude product was purified by separation with silica chromatography (ethyl acetate: petroleum ether = 10%-100%) to give compound 41-10 (1.1 g, 89.0% yield). ¹H NMR (400 MHz, DMSO-*d₆*) δ 9.85 (br. s, 1H), 7.10 - 6.92 (m, 2H), 4.98 - 4.83 (m, 1H), 4.37 (s, 2H), 4.03 (s, 3H), 3.66 - 3.50 (m, 1H), 3.29 (s, 3H), 2.69 - 2.55 (m, 3H), 2.35 - 2.24 (m, 1H), 1.98 - 1.82 (m, 2H), 1.50 (s, 9H), 1.08 - 0.97 (m, 6H).

### Step 9: synthesis of compound 41

A solution of compound 41-10 (1.1 g, 2.46 mmol) in formic acid (10 mL) was heated to 90 °C and stirred for 24 h. After the reaction was completed, the reaction solution was concentrated at reduced pressure to give a crude product. The crude product was purified by separation with silica chromatography (ethyl acetate:petroleum ether = 0-50%) to give another crude product (0.9 g, 85.9% yield), which was then separated and purified by SFC to give compound 41 (394 mg, 1.01 mmol, 43.78% yield, 100% purity). MS m/z (ESI) = 391.2 [M+H]⁺. ¹H NMR (400 MHz, DMSO-*d₆*) δ 12.19 (br. s, 1H), 10.23 (br. s, 1H), 7.04 (s, 1H), 7.01 - 6.92 (m, 1H), 5.04 - 4.84 (m, 1H), 4.34 (s, 2H), 4.03 (s, 3H), 3.66 - 3.48 (m, 1H), 3.27 (s, 3H), 2.76 - 2.60 (m, 3H), 2.46 - 2.31 (m, 1H), 2.02 - 1.79 (m, 2H), 1.13 - 0.89 (m, 6H).

### Example 16

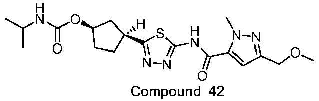

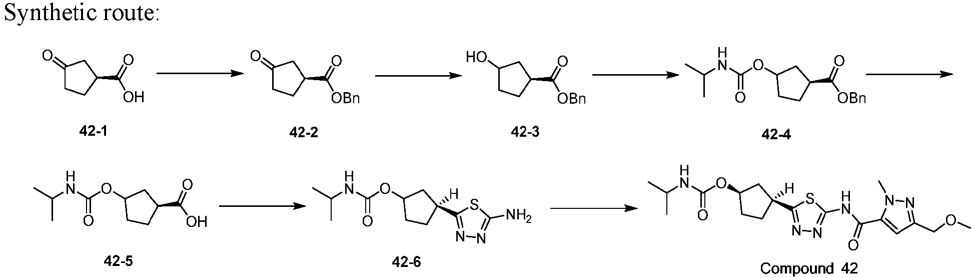

### Step 1: synthesis of compound 42-2

A solution of compound 42-1 (9 g, 70.24 mmol), benzyl bromide (13.22 g, 77.27 mmol), potassium carbonate (29.12 g, 210.73 mmol), and potassium iodide (233.21 mg, 1.40 mmol) in N,N-dimethylformamide (20 mL) was stirred at 25 °C for 3 h. After the reaction was completed, the system was quenched with water (200 mL) and extracted with ethyl acetate (200 mL × 3). The organic phase was washed with brine (200 mL × 2), dried over anhydrous Na₂SO₄, filtered, and then concentrated at reduced pressure. The crude product was purified by separation with silica column chromatography (ethyl acetate: petroleum ether = 5%-30%) to give compound 42-2 (15 g, 97.8% yield). ¹H NMR (400 MHz, DMSO-*d₆*) δ 7.47 - 7.24 (m, 5H), 5.13 (s, 2H), 3.28 - 3.18 (m, 1H), 2.44 - 2.29 (m, 2H), 2.29 - 2.11 (m, 3H), 2.05 - 1.98 (m, 1H).

### Step 2: synthesis of compound 42-3

Sodium borohydride (4.14 g, 109.44 mmol) was slowly added in portions to a solution of compound 42-2 (15 g, 68.73 mmol) in methanol (150 mL) at 0 °C, followed by stirring at 0 °C for 0.3 h. The reaction solution was quenched with a saturated ammonium chloride solution (200 mL). The mixture was adjusted to pH 7 with a hydrochloric acid solution (1 M) and then extracted with ethyl acetate (500 mL × 3). The organic phase was washed with brine (500 mL × 3), dried over anhydrous sodium sulfate, filtered, and concentrated at reduced pressure. The crude product was purified by separation with silica chromatography (ethyl acetate:petroleum ether = 10%-50%) to give compound 42-3 (12.3 g, 81.3% yield). ¹H NMR (400 MHz, DMSO-*d₆*) δ 7.52 - 7.27 (m, 5H), 5.19 - 5.04 (m, 2H), 4.63 - 4.51 (m, 1H), 4.25 - 4.04 (m, 1H), 3.05 - 2.73 (m, 1H), 2.12 - 2.02 (m, 1H), 1.96 - 1.85 (m, 1H), 1.83 - 1.59 (m, 3H), 1.57 - 1.46 (m, 1H).

### Step 3: synthesis of compound 42-4

Cuprous chloride (1.80 g, 18.16 mmol) and isopropyl isocyanate (1.85 g, 21.79 mmol) were added to a solution of compound 42-3 (4 g, 18.16 mmol) in N,N-dimethylformamide (40 mL). The reaction system was stirred at 25 °C for 3 h. After the reaction was completed, the reaction solution was filtered, diluted with water (100 mL), and extracted with ethyl acetate (100 mL × 3). The organic phase was washed with brine (100 mL × 3), dried over anhydrous sodium sulfate, filtered, and then concentrated at reduced pressure. The crude product was purified by separation with silica chromatography (ethyl acetate: petroleum ether = 5%-30%) to give compound 42-4 (5 g, 90.2% yield). ¹H NMR (400 MHz, DMSO-*d₆*) δ 7.52 - 7.22 (m, 5H), 7.00 - 6.76 (m, 1H), 5.10 (s, 2H), 5.04 - 4.83 (m, 1H), 3.66 - 3.46 (m, 1H), 3.09 - 2.78 (m, 1H), 2.35 - 2.16 (m, 1H), 1.97 - 1.73 (m, 4H), 1.71 - 1.54 (m, 1H), 1.09 - 0.89 (m, 6H).

### Step 4: synthesis of compound 42-5

Palladium on carbon (500 mg, 10% purity) was added to a solution of compound 42-4 (5 g, 16.37 mmol) in methanol (20 mL). The mixture was stirred at 25 °C for 3 h in a hydrogen atmosphere (30 psi). The reaction solution was filtered through celite, and the filtrate was concentrated at reduced pressure to give compound 42-5 (3.4 g, 96.5% yield). ¹H NMR (400 MHz, DMSO-*d₆*) δ 11.96 (br. s, 1H), 6.90 (br. s, 1H), 5.09 - 4.80 (m, 1H), 3.70 - 3.47 (m, 1H), 2.87 - 2.63 (m, 1H), 2.34 - 2.11 (m, 1H), 2.00 - 1.70 (m, 4H), 1.69 - 1.52 (m, 1H), 1.09 - 0.96 (m, 6H).

### Step 5: synthesis of compound 42-6

Phosphorus oxychloride (10 mL) was added to a mixture of compound 42-5 (30 g, 74.35 mmol) and thiosemicarbazide (1.44 g, 15.80 mmol), and the mixture was purged thrice with nitrogen. The reaction system was stirred at 40 °C for 3 h. After the reaction was completed, the reaction solution was added to water (200 mL) to quench the reaction, and the mixture was adjusted to pH 9 with a saturated sodium bicarbonate solution. The aqueous phase was extracted with ethyl acetate (200 mL × 3). The organic phase was washed with brine (100 mL × 3), dried over anhydrous sodium sulfate, filtered, and concentrated at reduced pressure. The crude product was purified by separation with silica chromatography (methanol:dichloromethane = 10%-20%) to give compound 42-6 (3.7 g, 13.69 mmol, 86.6% yield). ¹H NMR (400 MHz, DMSO-*d₆*) δ 7.06 - 7.00 (m, 2H), 6.98 - 6.85 (m, 1H), 5.12 - 4.89 (m, 1H), 3.64 - 3.50 (m, 1H), 2.39 - 2.24 (m, 1H), 2.16 - 1.99 (m, 2H), 1.92 - 1.59 (m, 4H), 1.07 - 0.96 (m, 6H).

### Step 6: synthesis of compound 42

Compound 42-6 (1 g, 3.70 mmol) and 5-(methoxymethyl)-2-methylpyrazole-3-carboxylic acid (629.43 mg, 3.70 mmol) were dissolved in dichloromethane (20 mL), and N,N-diisopropylethylamine (1.43 g, 11.10 mmol) and butylphosphonic anhydride (50% solution in ethyl acetate, 5.33 g, 7.40 mmol) were slowly added at 0 °C. The mixture was stirred at 25 °C for 12 h. The reaction solution was quenched with water (100 mL), and the mixture was extracted with ethyl acetate (100 mL × 3). The organic phase was washed with brine (100 mL × 2), dried over anhydrous Na₂SO₄, and filtered, and the filtrate was concentrated at reduced pressure to give a crude product (1 g, 61.8% yield). The crude product was then separated and purified by SFC to give compound 42 (540 mg, 1.25 mmol, 52.9% yield, 98.1% purity). MS m/z (ESI) = 423.2 [M+H]⁺. ¹H NMR (400 MHz, DMSO-*d₆*) δ 12.99 (br. s, 1H), 7.21 - 7.04 (m, 1H), 7.02 - 6.90 (m, 1H), 5.21 - 5.01 (m, 1H), 4.35 (s, 2H), 4.10 (s, 3H), 3.72 - 3.49 (m, 2H), 3.27 (s, 3H), 2.29 - 2.19 (m, 1H), 2.17 - 2.06 (m, 3H), 1.88 - 1.77 (m, 1H), 1.75 - 1.64 (m, 1H), 1.05 (d, *J =* 6.4 Hz, 6H).

### Example 17

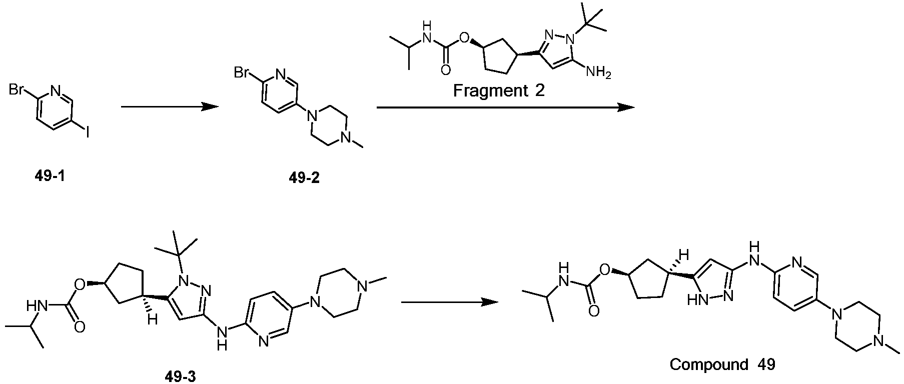

### Step 1: synthesis of compound 49-2

Toluene (20 mL), compound 2-bromo-5-iodopyridine (1.00 g, 1.00 *eq),* N-methylpiperazine (352 mg, 1.00 *eq),* 4,5-bis(diphenylphosphino)-9,9-dimethylxanthene (122 mg, 0.06 *eq),* sodium tert-butoxide (507 mg, 1.50 eq), and tris(dibenzylideneacetone)dipalladium (64.5 mg, 0.02 *eq)* were added into a 100 mL three-necked flask in sequence, and the system was incubated for reaction at 100 °C for 12 h in a nitrogen atmosphere. The reaction solution was filtered, and water (200 mL) was added. The mixture was extracted with ethyl acetate (200 mL × 2). The organic phase was washed with brine, dried, and concentrated to dryness to give a crude product. The crude product was purified by column chromatography to give compound **49-2** (red oil, 880 mg, 93.63% yield, 96% purity). MS (ESI) m/z: [M + H] ⁺ = 256. ¹H NMR (400 MHz, CHLORO FORM-d) δ = 8.01 (d, *J* = 3.1 Hz, 1H), 7.29 (d, *J=* 8.8 Hz, 1H), 7.07 (dd, *J =* 3.3, 8.8 Hz, 1H), 3.24 - 3.19 (m, 4H), 2.60 - 2.55 (m, 4H), 2.36 (s, 3H).

### Step 2: synthesis of compound 49-3

Dioxane (25 mL), compound 49-2 (415 mg, 1.00 *eq),* compound fragment 2 (500 mg, 1.00 *eq*), 4,5-bis(diphenylphosphino)-9,9-dimethylxanthene (46.9 mg, 0.05 *eq),* cesium carbonate (1.06 g, 2.00 *eq),* and tris(dibenzylideneacetone)dipalladium (74.2 mg, 0.05 *eq)* were added into a 100 mL three-necked flask in sequence, and the system was incubated for reaction at 100 °C for 12 h in a nitrogen atmosphere. The reaction solution was filtered, and water (200 mL) was added. The mixture was extracted with ethyl acetate (200 mL × 2). The organic phase was washed with brine, dried, and concentrated to dryness to give a crude product. The crude product was separated and purified by preparative chromatography to give compound **49-3** (pink oil, 26.0 mg, 3.625% yield, 98% purity). MS (ESI) m/z:[M + H] ⁺ = 484.

### Step 3: synthesis of compound 49

Compound 49-3 (26 mg, 1.00 *eq)* was dissolved in formic acid (1 mL). The system was reacted at 80 °C for 12 h. The reaction solution was concentrated to dryness to give a crude product. The crude product was separated and purified by preparative chromatography to give compound **49** (white solid, 7 mg, 30.30% yield, 98.5% purity). MS (ESI) m/z = 428 [M+H] ⁺. ¹H NMR (400 MHz, CHLOROFORM-d) δ = 7.88 (d, *J =* 2.6 Hz, 1H), 7.25 (dd, *J =* 2.9, 9.1 Hz, 1H), 7.02 (br d, *J =* 8.4 Hz, 2H), 5.86 (br s, 1H), 5.19 (br s, 1H), 4.66 (br s, 1H), 3.80 (br s, 1H), 3.22 - 3.06 (m, 5H), 2.70 - 2.59 (m, 4H), 2.55 - 2.44 (m, 1H), 2.40 (s, 3H), 2.15 - 2.05 (m, 1H), 1.99 - 1.80 (m, 4H), 1.19 - 1.07 (m, 6H).

### Example 18

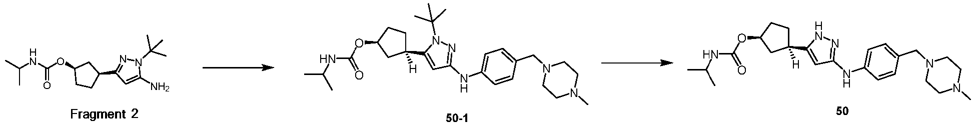

### Step 1: synthesis of compound 50-1

Compound fragment 2 (1.8 g, 5.84 mmol), 1-[(4-bromophenyl)methyl]-4-methylpiperazine (2.04 g, 7.59 mmol), and cesium carbonate (3.8 g, 11.67 mmol) were added to tert-amyl alcohol (50 mL), followed by the addition of (SP-4-1)-[1,3-bis[2,6-bis(1-ethylpropyl)phenyl]-4,5-dichloro-1,3-dihydro-2H-imidazol-2-ylidene]dichloro(2-methylpyridine)palladium (490.33 mg, 0.58 mmol). The reaction solution was purged with nitrogen and stirred at 100 °C for 5 h in a nitrogen atmosphere. The reaction mixture was filtered, and the filtrate was concentrated. The crude product was purified by separation with reversed-phase chromatography (0.1% formic acid, 0-45% acetonitrile) to give compound 50-1 (2.2 g, brown solid, 75.89% yield). MS (ESI) m/z = 497.4 [M+H]⁺.

### Step 2: synthesis of compound 50

Compound 50-1 (2.2 g, 4.43 mmol) was added to methanesulfonic acid (20 mL). The reaction solution was stirred at 30 °C for 3 h. After the reaction was completed, the reaction solution was cooled in an ice bath and slowly added dropwise to ice water to quench the reaction. The crude product was purified by separation with reversed-phase chromatography (0.1% formic acid system and 0-40% acetonitrile) and further purified (0.1% ammonium hydroxide solution system and 15-45% acetonitrile) to give compound 50 (1.2 g, white solid, 61.49% yield). MS (ESI) m/z = 441.3 [M+H] ⁺. ¹H NMR (400 MHz, DMSO-*d₆*) δ11.65 (s, 1H), 8.19 (s, 1H), 7.28 - 7.17 (m, 2H), 7.05 (d, *J =* 8.4 Hz, 2H), 6.97 (d, *J =* 7.2 Hz, 1H), 5.60 (s, 1H), 4.99 (m, 1H), 3.65 - 3.50 (m, 1H), 3.29 (s, 2H), 3.10 - 2.96 (m, 1H), 2.46 - 2.19 (m, 8H), 2.12 (s, 3H), 2.02 - 1.52 (m, 6H), 1.03 (d, *J =* 6.4 Hz, 6H).

### Example 19

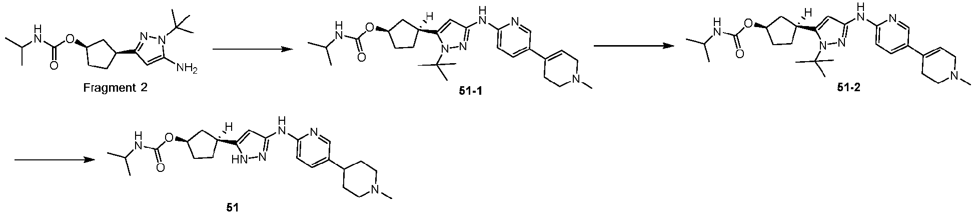

### Step 1: synthesis of compound 51-1

Compound fragment 2 (101 mg, 1.00 *eq),* 6-bromo-1'-methyl-1',2',3',6'-tetrahydro-3,4'-bipyridine (100 mg, 1.20 *eq),* cesium carbonate (161 mg, 1.50 *eq),* and Pd G4 (30.3 mg, 0.10 eq) were added to a mixed solvent of tert-butanol (1 mL) and dioxane (1 mL), and the system was reacted in microwaves at 100 °C for 2 h. After the reaction was completed, water was added, and the mixture was extracted with ethyl acetate, dried, and concentrated to dryness by rotary evaporation to give a crude product. The crude product was separated and purified by preparative chromatography and preparative thin-layer chromatography to give compound 51-1 (white solid, 245 mg). MS (ESI) m/z = 481.3 [M+H]⁺. ¹H NMR (400 MHz, CHLOROFORM-d) δ 8.09 (d, *J* = 2.8 Hz, 1H), 7.25 (d, *J =* 8.6 Hz, 1H), 6.98 - 6.94 (m, 1H), 6.45 (t, *J =* 3.4 Hz, 1H), 5.93 (s, 1H), 5.14 (br s, 1H), 5.05 (s, 1H), 4.52 - 4.35 (m, 1H), 3.86 - 3.68 (m, 1H), 3.18 (br s, 2H), 3.13 - 3.01 (m, 1H), 2.69 (br s, 3H), 2.56 - 2.46 (m, 1H), 2.43 (s, 3H), 2.07 - 1.99 (m, 1H), 1.93 - 1.74 (m, 5H), 1.61 (s, 9H), 1.11 (br d, *J =* 6.5 Hz, 6H).

### Step 2: synthesis of compound 51-2

Compound 51-1 (245 mg, 1.00 *eq)* was dissolved in methanol (2.5 mL), and in an argon atmosphere, wet Pd/C (49.0 mg, 10.0% purity, 0.09 *eq)* was added. The system was stirred at 30 °C for 12 h in a H₂ atmosphere (30 Psi). After the reaction was completed, the reaction solution was filtered and concentrated to dryness by rotary evaporation to give crude compound 51-2 (yellow gelatinous substance, 188 mg, 76.4% yield). MS (ESI) m/z = 483.3 [M+H]⁺.

### Step 3: synthesis of compound 51

Compound 51-2 (173 mg, 1.00 eq) was dissolved in formic acid (7 mL), and the system was stirred at 80 °C for 7 h and then stirred at 85 °C for 14 h. After the reaction was completed, the reaction solution was concentrated to dryness by rotary evaporation, adjusted to pH 7-8 with a saturated aqueous sodium carbonate solution, and extracted with dichloromethane. The organic phase was dried and concentrated to dryness by rotary evaporation to give a crude product. The crude product was separated and purified by preparative chromatography and purified by chiral resolution to give compound 51 (white solid, 36.0 mg, 23.1% yield, 98.0% purity). ¹H NMR (400 MHz, CHLOROFORM-d) δ 10.47 - 9.29 (m, 1H), 8.35 (d, *J =* 2.8 Hz, 1H), 7.61 - 7.53 (m, 1H), 7.07 (d, *J* = 8.5 Hz, 1H), 6.19 (br s, 1H), 5.79 (s, 1H), 5.19 (br s, 1H), 4.77 - 4.62 (m, 1H), 3.90 - 3.70 (m, 1H), 3.21 - 3.11 (m, 1H), 3.00 (br d, *J =* 11.5 Hz, 2H), 2.69 - 2.59 (m, 1H), 2.55 - 2.44 (m, 1H), 2.34 (s, 3H), 2.10 (br t, *J =* 10.9 Hz, 3H), 1.99 - 1.90 (m, 4H), 1.88 - 1.78 (m, 4H), 1.18 - 1.07 (m, 6H).

### Example 20

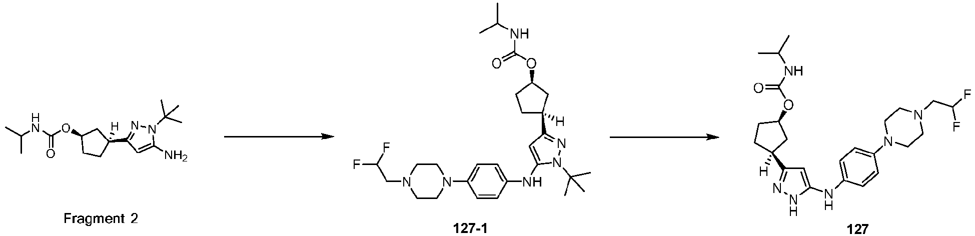

### Step 1: synthesis of compound 127-1

Compound fragment 2 (405 mg, 1.00 *eq),* 1-(2,2-difluoroethyl)-4-(4-iodophenyl)piperazine (555 mg, *1.20 eq),* Cs₂CO₃ (642 mg, 1.50 *eq*)*,* and BrettPhos PdG₃ (119 mg, 0.10 *eq*) were added to a solution of tert-butanol (4 mL)/dioxane (4 mL) in sequence in a nitrogen atmosphere. The reaction solution was placed in an oil bath at 100 °C and stirred for 5 h. The solvent was removed by concentration, and water (10 mL) was added. The mixture was adjusted to pH 5 with HCl (1 M) and extracted with dichloromethane (10 mL × 2). The organic phases were combined and concentrated to give a crude product. The crude product was separated by preparative chromatography to give compound 127-1 (yellow solid, 293 mg, 40.8% yield, 97.2% purity). MS (ESI) *m*/*z* = 533.3[M+H]⁺. ¹HNMR(400 MHz, CHLOROFORM-d) δ 6.94 - 6.78 (m, 2H), 6.70 (br d, *J =* 8.6 Hz, 2H), 6.13 - 6.03 (m, 1H), 5.98 - 5.77 (m, 1H), 5.14 (br s, 1H), 4.93 - 4.77 (m, 1H), 4.50 - 4.34 (m, 1H), 3.89 - 3.72 (m, 1H), 3.25 - 3.01 (m, 5H), 2.95 - 2.69 (m, 6H), 2.51 (td, *J=* 7.4, 14.3 Hz, 1H), 2.10 - 1.98 (m, 1H), 1.96 - 1.82 (m, 2H), 1.82 - 1.72 (m, 2H), 1.62 (s, 9H), 1.12 (br d, *J =* 6.5 Hz, 6H).

### Step 2: synthesis of compound 127

Compound 127-1 (233 mg, 1.00 *eq)* and TFA (18.4 g, 12 mL, 369 *eq)* were added into a 25 mL reaction flask, and the system was placed in an oil bath at 100 °C and stirred for 24 h. The reaction solution was concentrated in vacuum to give a crude product. The crude product was separated by preparative chromatography to give compound 127 (gray solid, 42.9 mg, 19.9% yield, 96.6% purity). MS (ESI)m/z = 477 [M+H]⁺. ¹HNMR (400 MHz, CHLOROFORM-d) δ7.09 (br d, *J* = 8.5 Hz, 2H), 6.89 (br d, *J =* 8.6 Hz, 2H), 6.09 - 5.78 (m, 2H), 5.75 (s, 1H), 5.20 (br s, 1H), 4.59 (br d, *J =* 2.6 Hz, 1H), 3.86 - 3.76 (m, 1H), 3.20 - 3.10 (m, 5H), 2.86 - 2.80 (m, 2H), 2.78 - 2.75 (m, 4H), 2.54 - 2.43 (m, 1H), 2.16 - 1.79 (m, 6H), 1.15 (br d, *J =* 6.3 Hz, 6H).

### Example 21

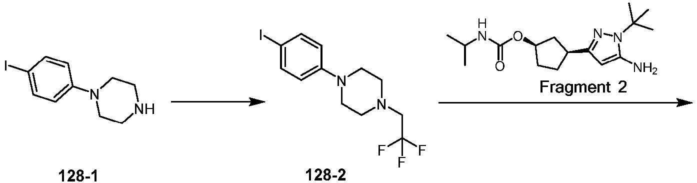

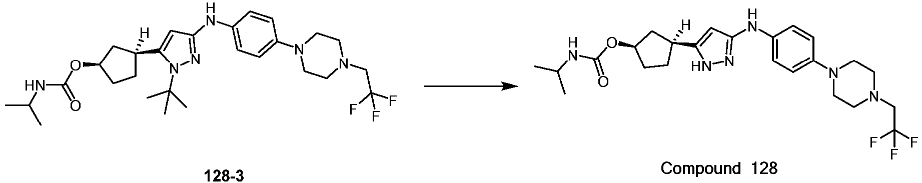

### Step 1: synthesis of compound 128-2

N,N-Dimethylformamide (10 mL), 2,2-difluoroethyl p-toluenesulfonate (3.22 g, 2.00 *eq),* compound 128-1 (2.00 g, 1.00 *eq),* and triethylamine (1.93 mL, 2.00 *eq)* were added to a 100 mL single-necked flask, and the system was reacted at 100 °C for 12 h. Water (100 mL) was added to the reaction solution, and the mixture was extracted with ethyl acetate (50 mL × 3), dried, and concentrated to dryness by rotary evaporation to give a crude product. The crude product was purified by column chromatography to give compound **128-2** (white solid, 1.10 g, 41.1% yield, 96% purity). MS (ESI) m/z: [M+H] ⁺ = 371. ¹H NMR (400 MHz, CHLOROFORM-d) δ = 7.55 - 7.50 (m, 2H), 6.69 (d, *J* = 8.9 Hz, 2H), 3.21 - 3.17 (m, 4H), 3.04 (q, *J =* 9.5 Hz, 2H), 2.86 - 2.81 (m, 4H).

### Step 2: synthesis of compound 128-3

tert-Butanol (3 mL), dioxane (3 mL), compound fragment 2 (300 mg, 972.71 µmol, 100 *eq),* compound 128-2 (432.06 mg, 1.17 mmol, 1.20 *eq),* Brettphos Pd G3 (88.18 mg, 97.27 µmol, 0.1 *eq),* and cesium carbonate (475.39 mg, 1.46 mmol, 1.50 *eq)* were added to a sealed tube in sequence, and the system was purged with nitrogen, warmed to an external temperature of 100 °C, and stirred for 3 h. The reaction solution was concentrated to dryness, and water (10 mL) was added. The mixture was adjusted to pH 5 with hydrochloric acid (1 N) and then extracted with dichloromethane (10 mL × 2). The organic phase was concentrated to dryness to give a crude product, and the crude product was separated and purified by preparative chromatography to give compound **128-3** (white solid, 250 mg, 45% yield, 96% purity). MS (ESI) m/z: [M+H]⁺ = 551.4.

### Step 3: synthesis of compound 128

Compound 128-3 (250 mg, 1.00 eq) was dissolved in formic acid (15 mL). The system was incubated for reaction at 80 °C for 12 h. The reaction solution was concentrated to dryness to give a crude product. The crude product was separated and purified by preparative chromatography to give compound 128 (white solid, 42 mg, 17% yield, 98.63% purity). MS m/z (ESI): 495.3[M+H]⁺. ¹HNMR (400 MHz, CHLOROFORM-d) δ 7.08 (br d, *J* = 8.4 Hz, 2H), 6.88 (br d, *J* = 8.6 Hz, 2H), 5.96 - 5.82 (m, 1H), 5.75 (s, 1H), 5.24 - 5.16 (m, 1H), 4.64 (br s, 1H), 3.80 (br d, *J =* 5.6 Hz, 1H), 3.18 - 3.09 (m, 5H), 2.87 - 2.82 (m, 4H), 2.54 - 2.42 (m, 1H), 2.16 - 2.06 (m, 1H), 1.97 - 1.79 (m, 5H), 1.16 - 1.12 (m, 6H).

### Example 22

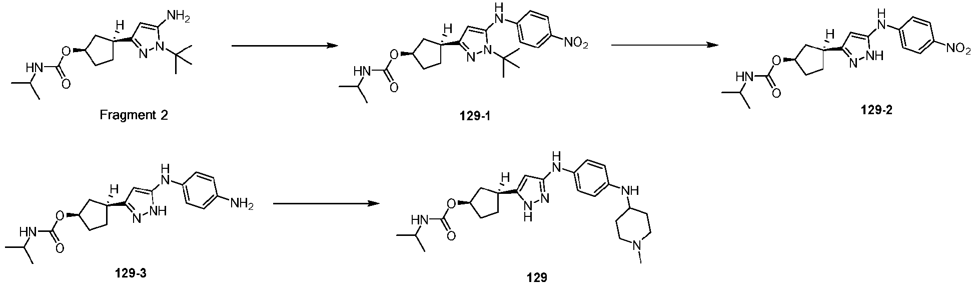

### Step 1: synthesis of compound 129-1

Compound fragment 2 (550 mg, 1.78 mmol, 1 eq) and p-bromonitrobenzene (648.42 mg, 3.21 mmol, 1.8 eq) were dissolved in anhydrous 1,4-dioxane (8 mL). Cesium carbonate (1.16 g, 3.57 mmol, 2 eq) was added to the reaction solution, and the mixture was purged thrice with nitrogen. Then, BrettPhos (Pd, G4) (164.16 mg, 178.33 µmol, 0.1 eq) was added, and the system was purged thrice with nitrogen again and reacted at 100 °C for 5 h. When LCMS indicated the completion of the reaction, the reaction mixture was poured into water (40 mL) and then extracted with ethyl acetate (20 mL × 4), and the combined organic phases were washed with saturated brine (30 mL × 3), dried over anhydrous sodium sulfate, and filtered and concentrated at reduced pressure to give a crude product. The crude product was purified on a normal-phase chromatography column (SiO₂ column, petroleum ether:ethyl acetate = 1/0 to 1/10) and concentrated to give compound 129-1 (720 mg, yellow solid, 94% yield). MS (ESI) *m*/*z* =430.3 [M+H]⁺. ¹H NMR (400 MHz, CDCl₃) δ 8.12 (d, *J* = 8.4 Hz, 2H), 6.69 (d, *J* = 8.8 Hz, 2H), 6.00 (s, 1H), 5.66 (s, 1H), 5.15 (br s, 1H), 4.41 (s, 1H), 3.80 (s, 1H), 3.16 - 3.01 (m, 1H), 2.59 - 2.47 (m, 1H), 2.13 - 1.92 (m, 2H), 1.90 - 1.74 (m, 3H), 1.59 (s, 9H), 1.18 - 1.08 (m, 6H).

### Step 2: synthesis of compound 129-2

Compound 129-1 (700 mg, 1.63 mmol, 1 eq) was added to formic acid (10 mL). The system was stirred at 80 °C for 24 h. When LCMS indicated the completion of the reaction, the reaction solution was concentrated to give compound 129-2 (800 mg, yellow oil, 98.59% yield). MS (ESI) *m*/*z* =374.2 [M+H]⁺.

### Step 3: synthesis of compound 129-3

Compound 129-2 (800 mg, 1.54 mmol, 1 eq, 76% purity) was dissolved in anhydrous tetrahydrofuran (4 mL), and Pd/C (80 mg, 10% purity) was added to the reaction solution in a nitrogen atmosphere. Then, the system was purged thrice with hydrogen and stirred at 20 °C for 3 h. When LCMS indicated the completion of the reaction, the reaction solution was filtered, and the filtrate was concentrated to give a crude product. The crude product was purified by reversed-phase column chromatography (0.1% formic acid, acetonitrile:water = 0-60%) and lyophilized to give compound 129-3 (223 mg, gray solid, 40.41% yield). MS (ESI) *m*/*z* =344.3 [M+H]⁺. ¹H NMR (400 MHz, CDCl₃) δ 8.19 (s, 1H), 6.97 (d, *J =* 8.4 Hz, 2H), 6.67 (d, *J =* 8.8 Hz, 2H), 5.69 (s, 1H), 5.62 - 5.44 (m, 1H), 5.25 (br s, 1H), 3.88 - 3.78 (m, 3H), 3.31 - 3.18 (m, 1H), 2.49 - 2.28 (m, 1H), 2.21 - 2.07 (m, 1H), 2.05 - 1.95 (m, 1H), 1.94 - 1.81 (m, 3H), 1.16 (d, *J =* 6.4 Hz, 6H).

### Step 4: synthesis of compound 129

Compound 129-3 (0.1 g, 262.07 µmol, 1 eq) and N-methylpiperidone (59.31 mg, 524.14 µmol, 60.96 µL, 2 eq) were dissolved in absolute ethanol (1 mL), followed by the addition of sodium cyanoborohydride (82.34 mg, 1.31 mmol, 5 eq) and acetic acid (62.95 mg, 1.05 mmol, 60.01 µL, 4 eq). The system was stirred at 80 °C for 4 h. When LCMS indicated the completion of the reaction, the reaction mixture was filtered and concentrated at reduced pressure to give a crude product. The crude product was purified by reversed-phase column chromatography (0.1% ammonium hydroxide solution, acetonitrile:water = 0-75%), lyophilized, and purified by reversed-phase column chromatography (0.1% formic acid, acetonitrile:water = 0-55%), and lyophilized again to give 129 (37.51 mg, yellow gelatinous substance, 29.41% yield). MS (ESI) *m*/*z* = 441.3 [M+H]⁺. ¹H NMR (400 MHz, METHANOL-d₄) δ 8.48 (s, 1H), 7.00 (s, 2H), 6.79 - 6.49 (m, 2H), 5.64 (br s, 1H), 5.07 (br s, 1H), 4.83 - 4.67 (m, 1H), 3.80 - 3.59 (m, 1H), 3.56 - 3.36 (m, 2H), 3.17 - 2.92 (m, 3H), 2.80 (s, 3H), 2.58 - 2.43 (m, 1H), 2.32 - 2.01 (m, 3H), 1.99 - 1.57 (m, 6H), 1.10 (d, *J =* 6.4 Hz, 6H).

### Example 23

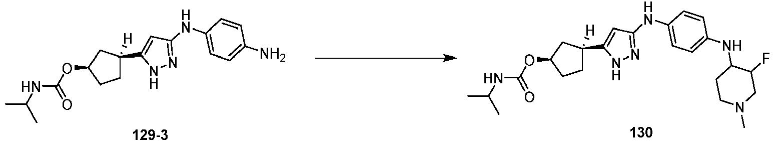

### Step 1: synthesis of compound 130

Compound 3-fluoro-1-methyl-4-piperidone (69 mg, 526.12 µmol, 3.01 eq) and compound 129-3 (60 mg, 174.71 µmol, 1 eq) were added to absolute methanol (2 mL), followed by the addition of acetic acid (11 mg, 183.17 µmol, 1.05 eq) and sodium cyanoborohydride (44 mg, 700.17 µmol, 4.0 eq) in sequence. The reaction solution was stirred at 30 °C for 48 h. The reaction solution was concentrated to give a concentrated crude product, and the crude product was purified by prep-HPLC (column: C18 150 × 30 mm; mobile phase: [water (FA)-ACN]; gradient: 8%-38% B over 7 min) and lyophilized to give compound 130 (23.79 mg, yellow solid, 26.98% yield). MS (ESI) m/z = 459.3 [M+H]⁺. ¹H NMR (400 MHz, DMSO-d₆) δ 7.01 (d, *J* = 8.8 Hz, 2 H ), 6.62 (d, *J* = 8.4 Hz, 2 H ), 5.69 (s, 1 H), 5.43 - 5.10 (m, 2 H), 4.82 (d, *J* = 49.2 Hz, 1 H ), 4.60 - 4.25 (m, 2 H), 4.21 - 3.95 (m, 2 H), 3.82 - 3.77 (m, 1 H), 3.40 - 3.20 (m, 2 H), 3.00 (d, *J =* 11.6 Hz, 1 H ), 2.47 - 2.34 (m, 4 H), 2.33 - 2.05 (m, 3 H), 1.97 (s, 2 H), 1.96 - 1.94 (m, 1 H), 1.90 - 1.80 (m, 3 H), 1.17 (s, 3 H), 1.15 (s, 3 H).

### Example 24

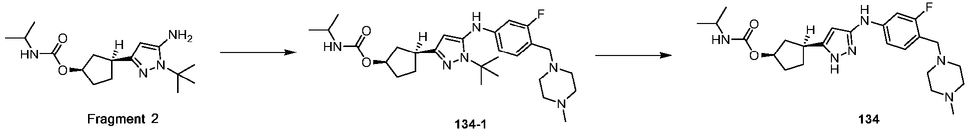

### Step 1: synthesis of compound 134-1

Compound 1-(4-bromo-2-fluorobenzyl)-4-methylpiperazine (120 mg, 389.08 µmol) was dissolved in dioxane (3 mL), and compound fragment 2 (138.53 mg, 466.90 µmol), cesium carbonate (380.31 mg, 1.17 mmol), and BrettPhos (Pd, G4) (35.82 mg, 38.91 µmol) were added in sequence. The reaction solution was purged thrice with nitrogen, stirred at 100 °C for 3 h, and filtered, and the filtrate was concentrated to dryness by rotary evaporation to give a crude product. The crude product was purified by reversed-phase column chromatography (0.1% trifluoroacetic acid in water, 0-75% acetonitrile) and lyophilized to give 134-1 (150 mg, white solid, 74.90% yield). MS m/z (ESI): 515.3[M+H]⁺.

### Step 2: synthesis of compound 134

Compound 134-1 (120 mg, 194.30 µmol) was dissolved in methanesulfonic acid (2 mL), and the system was stirred at 30 °C for 12 h. The reaction solution was slowly and dropwise added to ice water (3 mL) at 0 °C, purified by reversed-phase column chromatography (C18 column, 0.1% formic acid solution), and lyophilized to give a crude product. The crude product was purified again by reversed-phase column chromatography (0.1% ammonium hydroxide solution, 0-75% acetonitrile) and lyophilized to give compound 134 (31.44 mg, white solid, 29.41% yield). MS m/z (ESI): 459.3[M+H]⁺. ¹H NMR (DMSO-*d₆*, 400MHz) δ 11.75 (s, 1H), 8.52 (s, 1H), 7.27 (d, *J =* 12.4 Hz, 1H), 7.09 (t, *J =* 12.4 Hz, 1H), 7.00-6.85 (m, 2H), 5.61 (s, 1H), 5.03-4.93 (m, 1H), 3.65-3.49 (m, 1H), 3.35 (s, 2H), 3.09-2.98 (m, 1.1H), 2.38-2.24(m, 8H), 2.13 (s, 3H), 2.05-1.83 (m, 3H), 1.76-1.66 (m, 2H), 1.64.1.53 (m, 1H), 1.03 (d, *J =* 6.4 Hz, 6H).

Referring to the synthesis methods of steps 1-2 in Example 24 (compound 134), compounds listed in the following table were synthesized:

| Compound No. | Structure | MS m/z | Nuclear magnetic resonance |
|---|---|---|---|
| Compound 136 | 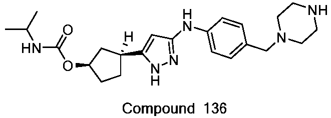 | 449.2 [M+Na]⁺ | ¹H NMR (400 MHz, DMSO-*d₆*) δ 12.63 - 10.66 (m, 1H), 9.98 - 9.27 (m, 2H), 9.25 - 8.10 (m, 1H), 7.42 - 7.36 (m, 2H), 7.35 - 7.30 (m, 2H), 7.04 - 6.90 (m, 1H), 5.75 - 5.65 (m, 1H), 5.06 - 4.91 (m, 1H), 4.31 - 4.17 (m, 2H), 3.24 (br s, 8H), 3.14 - 2.95 (m, 2H), 2.49 - 2.40 (m, 1H), 2.08 - 1.97 (m, 1H), 1.95 - 1.82 (m, 1H), 1.79 - 1.65 (m, 2H), 1.63 - 1.52 (m, 1H), 1.06 - 0.99 (m, 6H). |
| Compound 137 | 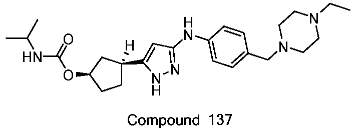 | 455.2 [M+H]⁺ | ¹H NMR (400 MHz, DMSO-*d₆*) δ 8.21 (s, 1H), 8.20 (s, 1H), 7.23 (d, *J* = 8.4 Hz, 2H), 7.06 (d, *J =* 8.4 Hz, 2H), 6.96 (br d, *J =* 7.4 Hz, 1H), 5.61 (s, 1H), 5.00 (br s, 1H), 3.68 - 3.51 (m, 1H), 3.34 (s, 2H), 3.13 - 2.93 (m, 1H), 2.50 - 2.23 (m, 11H), 2.20 - 1.35 (m, 6H), 1.12 - 0.90 (m, 9H). |

### Test Example 1. CDK2 Kinase Antagonistic Activity Assay

### 1.1 Materials

| **Material** | **Manufacturer** | **Cat. No.** |
|---|---|---|
| ATP | Thermo Fisher | 18330019 |
| CDK2/Cyclin E1 protein | Invitrogen | PV6296 |
| CDK1/Cyclin B protein | Invitrogen | PR4768C |
| MLight-MBP peptide | PerkinElmer | TRF0109-M |
| Eu-anti-P-MBP antibody | PerkinElmer | TRF0201-M |
| Lance Detection Buffer | PerkinElmer | CR97-100 |

### 2.2 Instruments

| **Instrument** | **Manufacturer** | **Model** |
|---|---|---|
| Microplate vibrating screen | VWR | 12620-928 |
| 384-well plate | Greiner | 781201 |
| Perkin Elmer Envision | BioTek Instruments, Inc. (USA) | Synergy H1 |

### 2.3. Procedures

Compounds were dissolved in DMSO and serially diluted 3-fold to generate 11 concentrations. 100 nL of each compound was added to 384-well plates, followed by the addition of 5 µL of 2× CDK2/Cyclin E1 or CDK1/Cyclin B enzyme mixed solution. 5 µL of buffer (50 mM Hepes (pH 7.5), 10 mM MgCl₂, 1 mM EDTA, 0.01% Brij-35, 2 mM DTT (prepared freshly)) was added to the complete inhibition control wells. The plates were centrifuged at 1000 rpm for 30 s and incubated at 23 °C for 15 min. Then, 5 µL of 2× MLight-MBP peptide (containing 10 µM ATP) was added to each well of each assay plate. The plates were centrifuged again at 1000 rpm for 30 s and incubated at 23 °C for 90 min.

After the incubation was completed, the reaction was stopped with a detection buffer containing 15 mM EDTA and 2 nM Eu-anti-P-MBP antibody. After centrifugation at 1000 rpm for about 1 min, the plates were incubated at 23 °C for 60 min and read on Perkin Elmer Envision. The TR-FRET ratio (value at 665 nm/value at 615 nm) was automatically calculated by Envision. The TR-FRET ratio was normalized, and the inhibition rate (%) was calculated: inhibition rate = (100% inhibition control sample - sample data)/(100% inhibition control sample - 0% inhibition control sample) × 100, where the 0% inhibition control sample was a mixture of enzyme, peptide, and ATP (containing no compound); the 100% inhibition control sample was a mixture of assay buffer and peptide containing ATP (containing no enzyme solution).

The IC₅₀ values of the compounds were calculated using GraphPad Prism nonlinear fitting equation. The results are shown in Table 1.

**Table 1. Kinase activity assay**

| Compound No. | CDK2/Cyclin E1 IC₅₀ (nM) | CDK1/Cyclin B1 IC₅₀ (nM) |
|---|---|---|
| Compound 1 | 2.24 | 78.2 |
| Compound 2 | 5.15 | 42.48 |
| Compound 3 | 1.22 | 78.2 |
| Compound 4 | 2.58 | 459.94 |
| Compound 5 | / | 845.16 |
| Compound 6 | 0.95 | 78.4 |
| Compound 7 | 0.64 | 90.04 |
| Compound 8 | / | 3110.26 |
| Compound 9 | 4.38 | 137.58 |
| Compound 10 | 8.71 | / |
| Compound 11 | 1.07 | / |
| Compound 12 | 0.61 | 28.49 |
| Compound 13 | 3.37 | 48.25 |
| Compound 16 | 0.16 | 3.07 |
| Compound 23 | 5.01 | 1846.59 |
| Compound 24 | 2.58 | 589.08 |
| Compound 25 | 3.09 | 11.18 |
| Compound 26 | 0.92 | 0.27 |
| Compound 27 | 9.32 | 54.18 |
| Compound 28 | 18.03 | 124.75 |
| Compound 30 | 8.22 | / |
| Compound 31 | 23.62 | 495.62 |
| Compound 33 | 0.80 | 171.56 |
| Compound 36 | >1000 | / |
| Compound 41 | >1000 | / |
| Compound 42 | >1000 | / |
| Compound 49 | 10.74 | 723.71 |
| Compound 50 | 2.35 | 1274.27 |
| Compound 51 | 6.90 | 153.65 |
| Compound 127 | 12.47 | / |
| Compound 128 | 12.83 | / |
| Compound 134 | 2.14 | 663.68 |
| Compound 136 | 5.53 | 1023.17 |
| Compound 137 | 3.74 | 975.68 |

| | | |
|---|---|---|
| Conclusion: Most compounds of the present disclosure exhibited good CDK2 kinase inhibitory activity but weak CDK1 kinase inhibitory activity. | | |

### Test Example 2. Antagonistic Effect Assay Against HCT116 Cell Proliferation

### 2.1 Materials

| **Material** | **Manufacturer** | **Cat. No.** |
|---|---|---|
| CellTitle-Glo detection kit | Promega | G7570 |
| HCT116 cells | Wuhan Procell Life Science & Technology Co., Ltd. | CL-0096 |
| RPMI-1640 medium | Gibco | 11875093 |
| Fetal bovine serum | Gibco | 10100147 |
| Dimethyl sulfoxide (DMSO) | Gibco | 20688 |

### 2.2 Instruments

| **Instrument** | **Manufacturer** | **Model** |
|---|---|---|
| Electric thermostatic incubator | Shanghai Yiheng | DHP-9032 |
| Microplate vibrating screen | VWR | 12620-928 |
| BIOTEK multimode microplate reader | BioTek Instruments, Inc. (USA) | Synergy H1 |

### 2.3. Procedures

Frozen HCT116 cells were rapidly transferred from liquid nitrogen to a 37 °C water bath and rapidly shaken for thawing. The cell suspension was transferred to a 15 mL centrifuge tube and centrifuged at 1500 rpm for 5 min, and the supernatant was discarded. The cell pellet was resuspended in RPMI-1640 complete medium (containing 10% fetal bovine serum and 1% penicillin/streptomycin) and transferred to a cell culture dish containing 10 mL of medium. The cells were cultured in an incubator at 37 °C/5% CO₂. The cells were passaged after adhesion to the wall.

HCT116 cells in the logarithmic growth phase and in good condition were taken, digested with a trypsin solution, resuspended in a certain amount of RPMI-1640 complete medium to form a single-cell suspension, counted on a cell counting plate, and seeded into a 96-well white plate at a density of 800 cells/well. The plate was left to stand for 5 min and then incubated in an incubator at 37 °C/5% CO₂ overnight. The next day, the cells were treated with the compound at different concentrations, and the control group was treated with DMSO at the corresponding concentration. The compound, dissolved in DMSO, was diluted in RPMI-1640 complete medium at a ratio of 1:1000 to obtain final concentrations of 10.00 µM, 3.33 µM, 1.11 µM, 0.37 µM, 0.12 µM, 0.04 µM, and 0.01 µM. Each group was set up with 3 replicate wells, and 100 µL of the treatment solution was added to each well. The plate was incubated in an incubator at 37 °C/5% CO₂ for 72 h.

After 72 h of treatment, the CellTitle-Glo assay was performed. 100 µL of CellTitle-Glo solution was added to each well of the control and treatment groups. The plate was mixed on an oscillator for 2 min to induce cell lysis. The 96-well plate was incubated at room temperature for 10 min to stabilize the luminescence signal value. The luminescence signal was measured using a microplate reader. The DMSO-treated cell group was used as the control group, and 50% inhibitory concentration (IC₅₀) was calculated according to the median effect equation as follows: Inhibition rate (%) = (L value of the control group - L value of the drug-treated group)/A value of the control group × 100%

The IC₅₀ values of the compounds were calculated using GraphPad Prism nonlinear fitting equation. The results are shown in Table 2.

**Table 2. Results for the inhibitory effect of the compounds of the present disclosure on HCT116 cell proliferation**

| Compound No. | Antiproliferative IC₅₀ (nM) |
|---|---|
| Compound 1 | 3655.95 |
| Compound 2 | 2471.72 |
| Compound 3 | 2089.30 |
| Compound 4 | 2338.84 |
| Compound 5 | >10000 |
| Compound 6 | 2642.41 |
| Compound 7 | 2254.24 |
| Compound 8 | >10000 |
| Compound 9 | 2624.22 |
| Compound 10 | 7112.13 |
| Compound 11 | 1606.94 |
| Compound 12 | 1862.34 |
| Compound 13 | 2153.78 |
| Compound 16 | 847.32 |
| Compound 26 | 387.94 |
| Compound 27 | 3006.54 |
| Compound 33 | 4325.74 |
| Compound 49 | 1297.18 |
| Compound 50 | 1274.32 |
| Compound 51 | 3532.19 |
| Compound 127 | 9795.78 |
| Compound 128 | 9954.21 |
| Compound 129 | 10209.39 |
| Compound 130 | 18113.14 |
| Compound 134 | 1127.19 |
| Compound 136 | 8433.34 |
| Compound 137 | 1506.60 |

Conclusion: Most compounds of the present disclosure exhibited a good inhibitory effect on HCT116 cell proliferation.

### Test Example 3. Evaluation of In-Vitro Liver Microsomal Stability

Instrument: water purifier, Millipore; electronic balance, Mettler Toledo; high-speed benchtop centrifuge, Thermo; water bath thermostatic oscillator, Shanghai Yiheng Technology Co., Ltd.; vortex oscillator, Thermo; LC-MS/MS, AB SCIEX.

Method: The metabolic stability of test substances was evaluated by using human, dog, and SD rat liver microsomes. The test substances were incubated with liver microsomes from different species and NADPH in a water bath at 37 °C. In the incubation system, the final concentration of the test or control sample was 1 µM, the final concentration of liver microsomes was 0.5 mg/mL, the final concentration of NADPH was 1 mM, and the final concentration of MgCl₂ was 3 mM. At designated time points (5 min, 10 min, 20 min, 30 min, and 60 min), the reaction was stopped by adding a cold acetonitrile solution containing tolbutamide (at a concentration of 200 ng/mL) as an internal standard. For the 0-min sample, the stop solution was added before the addition of the NADPH working solution. Testosterone and dextromethorphan were used as positive control samples in the same conditions to assess the stability and reliability of the system. After sample pretreatment, semiquantitative analysis was performed using the LC-MS/MS method. The retention times of the analytes and internal standard, the acquisition of chromatograms and the integration of chromatograms were processed using software Analyst (AB Sciex, Framingham, Massachusetts, USA). The remaining percentage of the test samples after 60 min of incubation was calculated, and the test results are shown in Table 3.

**Table 3. Test data for the compounds of the present disclosure in human, dog, and rat liver microsomes**

| Compound No. | Remaining amount% at 60 min | | |
|---|---|---|---|
| | Human | Dog | Rat |
| Compound 1 | 80.3 | 92.0 | 85.0 |
| Compound 2 | 5.38 | 12.7 | 3.87 |
| Compound 3 | 90.9 | 86.7 | 74.6 |
| Compound 4 | 93.5 | 77.9 | 77.9 |
| Compound 6 | 11.0 | 30.3 | 1.80 |
| Compound 7 | 93.2 | 101 | 101 |
| Compound 9 | 93.2 | 7.34 | 27.7 |
| Compound 10 | 103 | 95 | 92 |
| Compound 11 | 105 | 99 | 104 |
| Compound 13 | 80.0 | / | 71.2 |
| Compound 24 | 15.4 | / | 22.8 |
| Compound 27 | 91.3 | 96.7 | 81.2 |
| Compound 33 | 73.5 | / | 39.7 |
| Compound 50 | 86.0 | / | 72.1 |

| | | | |
|---|---|---|---|
| Conclusion: compounds 1, 3, 4, 7, 10, 11, and 27 all exhibited low clearance in rat, dog, and human liver microsomes, and compounds 9, 13, 27, 33, and 50 exhibited low clearance in human liver microsomes, all demonstrating good stability and druggability. | | | |

### Test Example 4. Evaluation of Permeability and Transporter Substrate

Cell: Caco-2 cells
Instrument: water purification equipment, ELGA LabWate; biosafety cabinet, Nuaire; thermostatic CO₂ incubator, Thermo; microplate reader, PerkinElmer; LC-MS/MS, AB SCIEX.
Method: The purpose of this study was to determine the two-way permeability of the compounds and evaluate whether they are effluxed and transported by P-glycoprotein (P-gp) using a Caco-2 monolayer cell model. In the experiment, Caco-2 cells were seeded into a 96-well cell plate and cultured for 24 consecutive days for the transport experiment. The compounds were administered bidirectionally at a concentration of 2.00 µM, with or without verapamil. After 120 min of incubation, the samples at the apical end A and the basal end B were collected. The content of the test samples in each sample was measured using the liquid chromatography-tandem mass spectrometry (LC-MS/MS) method. The apparent permeability coefficient and the efflux rate were calculated. Two experimental systems were used successively in this part, with the reference compound (PF-07104091) included. The data are summarized in Tables 4 and 5.

**Table 4. Permeability test results**

| Compound No. | Mean Pₐₚₚ (10⁻⁶ cm/s) | | Efflux Ratio |
|---|---|---|---|
| | A to B | B to A | |
| Compound 1 | <0.480 | 19.3 | >40.2 |
| Compound 2 | 0.218 | 43.4 | 199 |
| Compound 3 | 0.218 | 32.8 | 150 |
| Compound 4 | 0.0791 | 30.8 | 390 |
| Compound 6 | 0.540 | 26.3 | 48.7 |
| Compound 7 | <0.260 | 14.4 | >55.4 |
| Compound 9 | 0.520 | 43.8 | 84.2 |
| PF-07104091 | 0.106 | 25.5 | 241 |

| | | | |
|---|---|---|---|
| Conclusion: Compounds 6, 9, and 24 exhibited higher permeability than the reference compound PF-07104091, showing moderate permeability, while the other compounds exhibited low permeability. | | | |

**Table 5. Permeability test results**

| Compound No. | Mean Pₐₚₚ(10⁻⁶ cm/s) | | Efflux Ratio |
|---|---|---|---|
| | A to B | B to A | |
| Compound 49 | 2.40 | 12.2 | 5.09 |
| Compound 50 | 14.4 | 12.4 | 1.09 |
| Compound 134 | 7.08 | 23.4 | 3.30 |
| PF-07104091 | 4.30 | 37.0 | 8.57 |

| | | | |
|---|---|---|---|
| Conclusion: Compounds 50 and 134 exhibited higher permeability than the reference compound PF-07104091. | | | |

## Claims

1. A compound of formula (IIA) or a pharmaceutically acceptable salt, a stereoisomer, or a deuteride thereof: 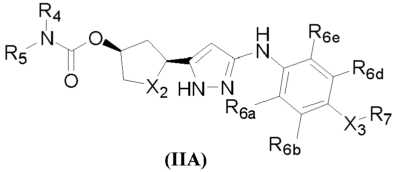 wherein,
X₂ is selected from the group consisting of CH₂ or O;
X₃ is selected from the group consisting of CH₂, NH, O, or a bond;
R₄ and R₅ are independently selected from the group consisting of H, C₁₋₆ alkyl, cyano-substituted C₁₋₆ alkyl, C₂₋₆ alkenyl, C₂₋₆ fluoroalkenyl, or C₂₋₆ alkynyl;
or R₄ and R₅, together with the N atom to which they are linked, form 4- to 6-membered heterocyclyl or 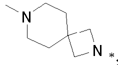 , wherein the 4- to 6-membered heterocyclyl is optionally substituted with 1-2 methyl; R₆ₐ, R_{6b}, R_{6d}, and R₆ₑ are each independently selected from the group consisting of H, halogen, C₁₋₆ alkyl, (CH₃)₂-P(O)-, or CH₃-S(O)₂-, wherein the C₁₋₆ alkyl is optionally substituted with C₁₋₆ alkoxy; R₇ is independently selected from the group consisting of 6- to 10-membered heterocyclyl, wherein the 6- to 10-membered heterocyclyl is optionally substituted with 1, 2, 3, 4, or 5 halogens, C₁₋₆ alkyl, or C₁₋₆ haloalkyl.

2. The compound or the pharmaceutically acceptable salt, the stereoisomer, or the deuteride thereof according to claim 1,
wherein,
R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl;
or R₄ and R₅, together with the N atom to which they are linked, form azetidinyl, 2,2-dimethylazetidinyl, or 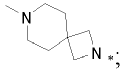
R₆ₐ, R_{6b}, R_{6d}, and R₆ₑ are each independently selected from the group consisting of H, F, Cl, Br, I, methyl, ethyl, n-propyl, isopropyl, n-butyl, isobutyl, tert-butyl, (CH₃)₂-P(O)-, or CH₃-S(O)₂-, wherein the methyl, ethyl, n-propyl, isopropyl, n-butyl, isobutyl, or tert-butyl is optionally substituted with methoxy, ethoxy, n-propoxy, or isopropoxy;
R₇ is independently selected from the group consisting of piperidinyl or piperazinyl, wherein the piperidinyl or piperazinyl is optionally substituted with 1, 2, 3, 4, or 5 F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

3. The compound or the pharmaceutically acceptable salt, the stereoisomer, or the deuteride thereof according to claim 1 or 2,
wherein,
R₇ is independently selected from the group consisting of 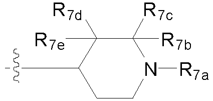 or 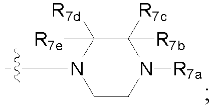 R₇ₐ, R_{7b}, R_{7c}, R_{7d}, and R₇ₑ are independently selected from the group consisting of H, F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

4. The compound or the pharmaceutically acceptable salt, the stereoisomer, or the deuteride thereof according to claim 3,
wherein,
X₂ is selected from the group consisting of CH₂; X₃ is selected from the group consisting of CH₂; R₄ is selected from the group consisting of H; R₅ is selected from the group consisting of C₁₋₆ alkyl, preferably isopropyl;
R₆ₐ, R_{6b}, R_{6d}, and R₆ₑ are each independently selected from the group consisting of H or halogen.

5. A compound of formula (II) or a pharmaceutically acceptable salt, a stereoisomer, or a deuteride thereof, wherein, 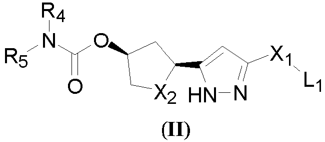
X₁ is selected from the group consisting of NH or O;
X₂ is selected from the group consisting of CH₂ or O;
L₁ is selected from the group consisting of 6- to 10-membered aryl, 6- to 10-membered heteroaryl, or 6- to 10-membered heterocyclyl, wherein the 6- to 10-membered aryl, 6- to 10-membered heteroaryl, or 6- to 10-membered heterocyclyl is optionally substituted with 1, 2, or 3 R₆;
R₄ and R₅ are independently selected from the group consisting of H, C₁₋₆ alkyl, cyano-substituted C₁₋₆ alkyl, C₂₋₆ alkenyl, C₂₋₆ fluoroalkenyl, or C₂₋₆ alkynyl;
or R₄ and R₅, together with the N atom to which they are linked, form 4- to 6-membered heterocyclyl or 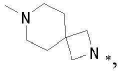 wherein the 4- to 6-membered heterocyclyl is optionally substituted with 1-2 methyl;
each R₆ is independently selected from the group consisting of H, halogen, C₁₋₆ alkyl, (CH₃)₂-P(O)-, CH₃-S(O)₂-, -CH₂R₇, -NHR₇, or 6- to 10-membered heterocyclyl, wherein the C₁₋₆ alkyl is optionally substituted with C₁₋₆ alkoxy, and the 6- to 10-membered heterocyclyl is optionally substituted with 1, 2, 3, 4, or 5 halogens, C₁₋₆ alkyl, or C₁₋₆ haloalkyl;
R₇ is independently selected from the group consisting of 6- to 10-membered heterocyclyl, wherein the 6- to 10-membered heterocyclyl is optionally substituted with 1, 2, 3, 4, or 5 halogens, C₁₋₆ alkyl, or C₁₋₆ haloalkyl.

6. A compound of formula (I) or a pharmaceutically acceptable salt, a stereoisomer, or a deuteride thereof, 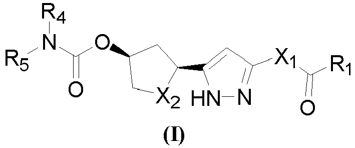 wherein,
X₁ is selected from the group consisting of NH or O;
X₂ is selected from the group consisting of CH₂ or O;
R₁ is selected from the group consisting of 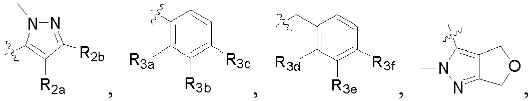 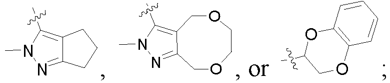
R₂ₐ and R_{2b} are each independently selected from the group consisting of H or C₁₋₆ alkyl, wherein the C₁₋₆ alkyl is optionally substituted with C₁₋₆ alkoxy, R_{2c}-S(O)₂-, or R_{2c}-S(O)(NH)-, and R_{2c} is selected from the group consisting of H, C₁₋₆ alkyl, C₃₋₆ cycloalkyl, or amino;
R₃ₐ, R_{3b}, R_{3c}, R_{3d}, R₃ₑ, and R_{3f} are each independently selected from the group consisting of H, CH₃-S(O)₂-CH₂-, (CH₃)₂-P(O)-, or 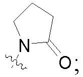
R₄ and R₅ are independently selected from the group consisting of H, C₁₋₆ alkyl, C₂₋₆ alkenyl, C₂₋₆ alkynyl, C₁₋₆ fluoroalkyl, C₂₋₆ fluoroalkenyl, C₂₋₆ fluoroalkynyl, or C₃₋₁₀ cycloalkyl, wherein the terminal C atom of each C₁₋₆ alkyl is optionally substituted with cyano, and each C₃₋₁₀ cycloalkyl is optionally substituted with difluoromethylene;
or R₄ and R₅, together with the N atom to which they are linked, form 4- to 6-membered heterocyclyl or 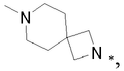 wherein the 4- to 6-membered heterocyclyl is optionally substituted with 1-2 methyl.

7. A compound as shown below or a pharmaceutically acceptable salt, a stereoisomer, or a deuteride thereof, wherein the compound is selected from the group consisting of:

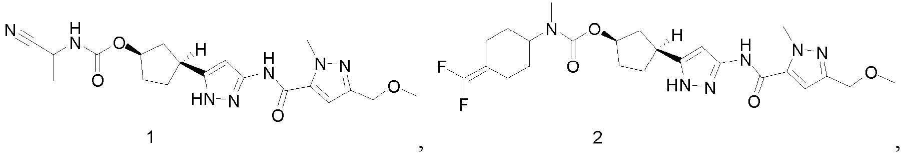

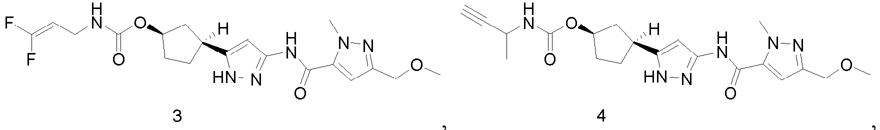

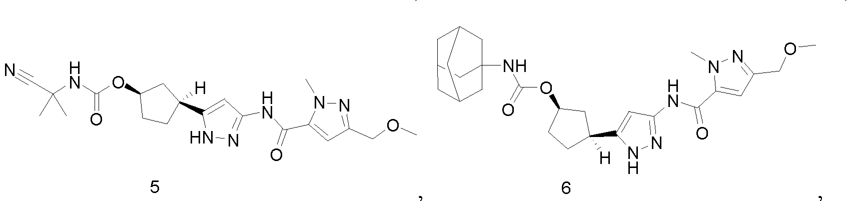

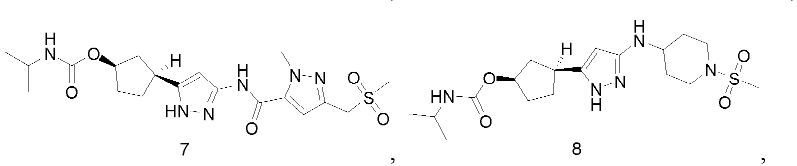

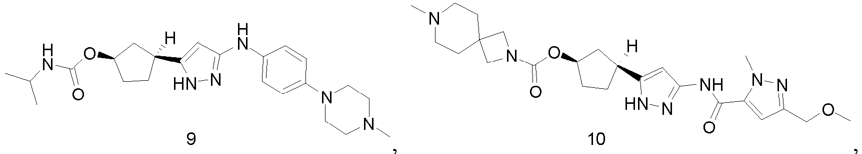

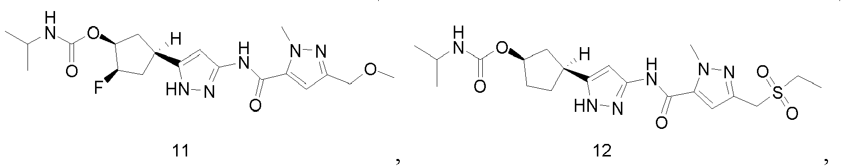

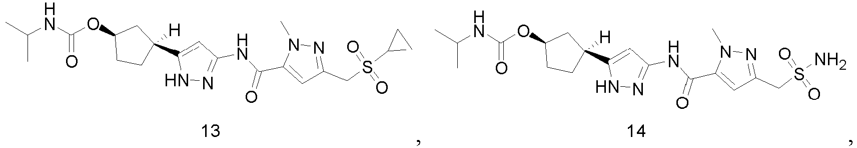

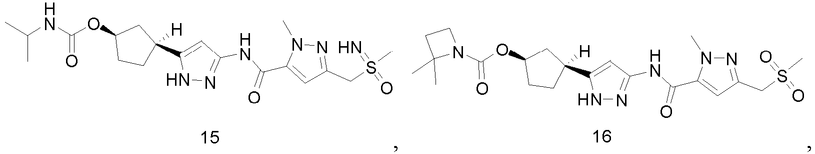

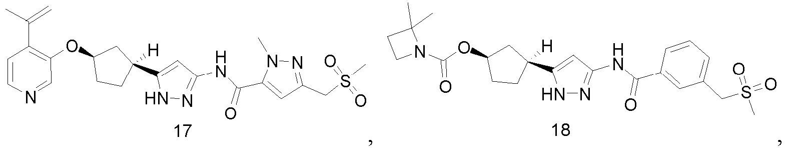

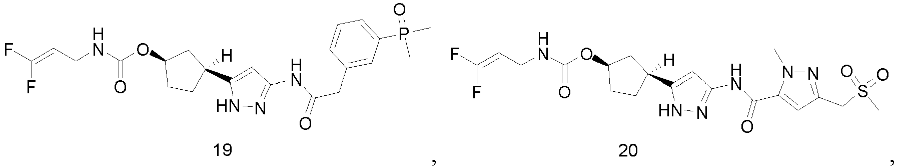

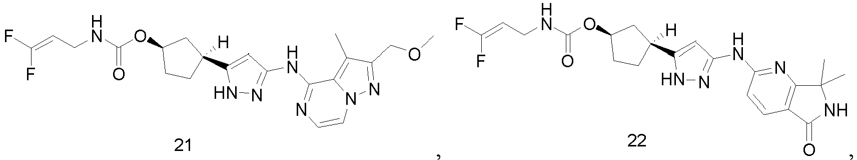

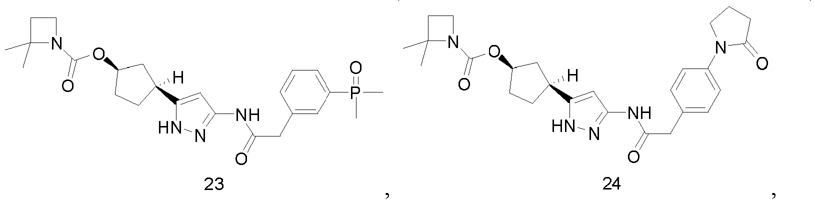

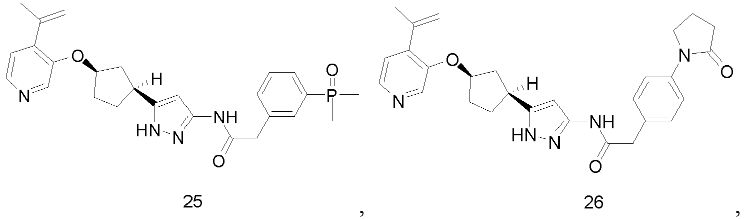

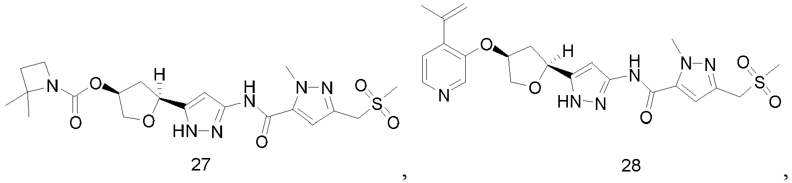

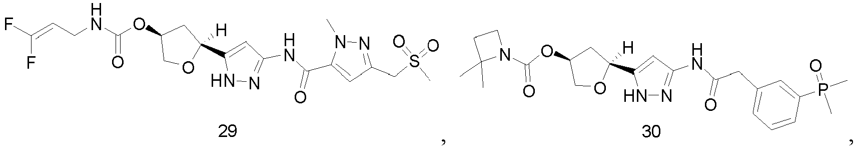

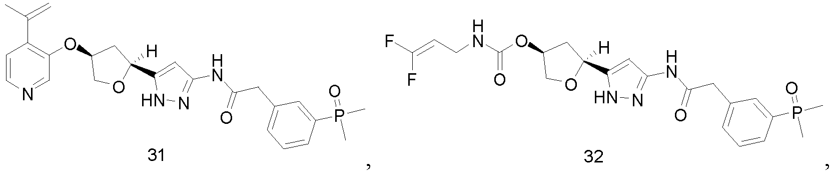

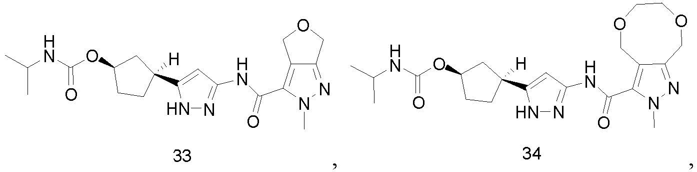

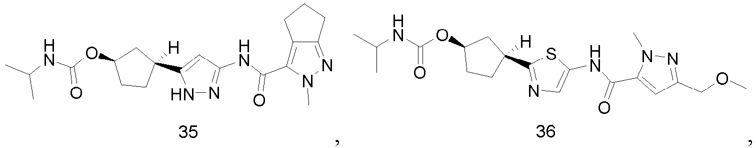

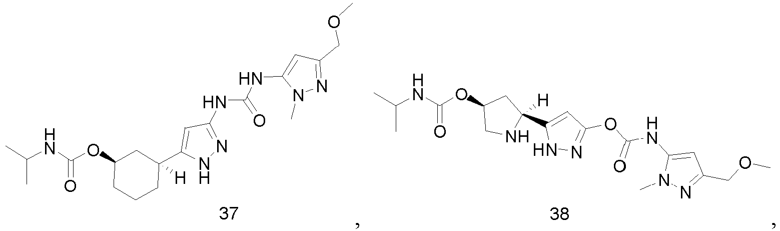

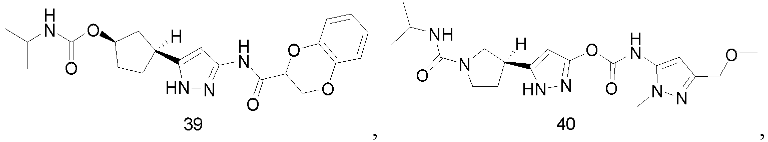

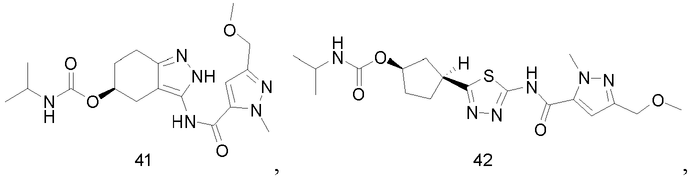

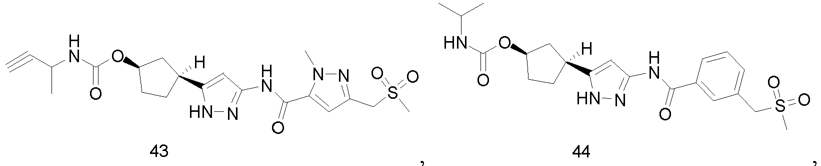

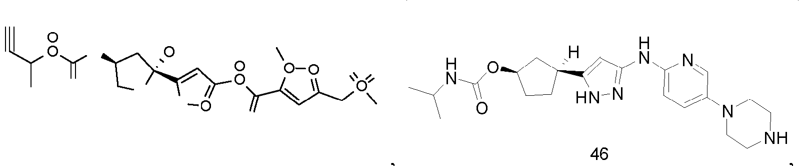

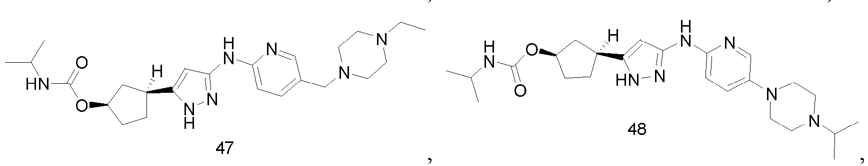

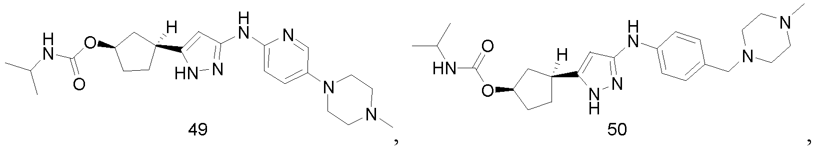

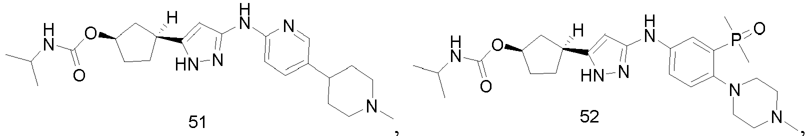

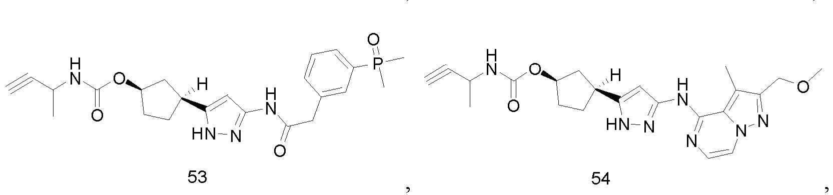

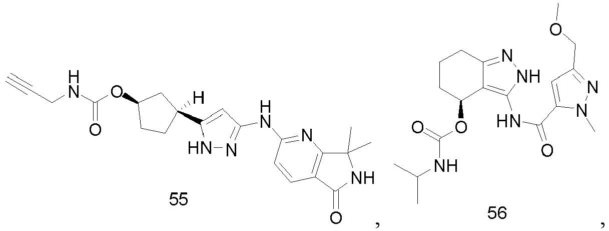

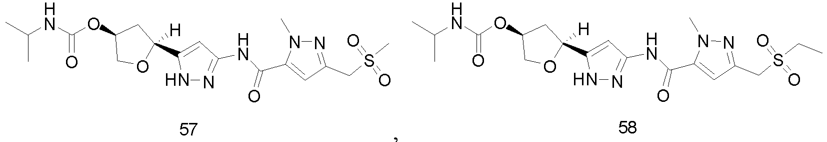

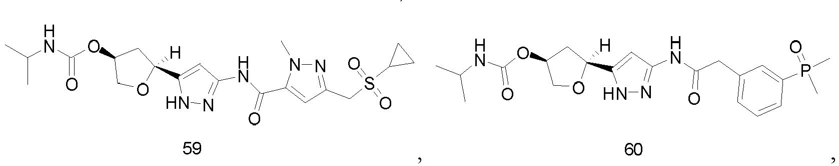

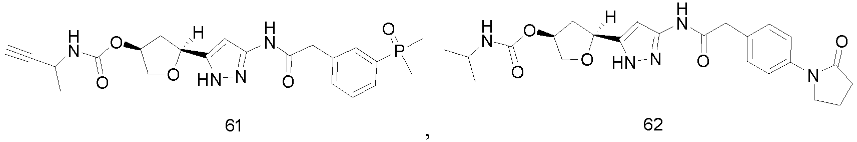

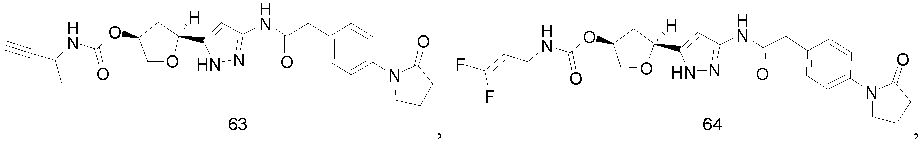

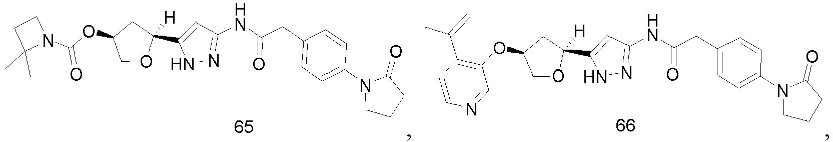

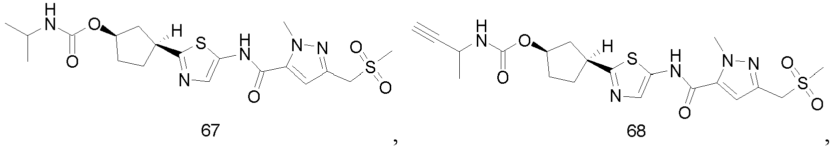

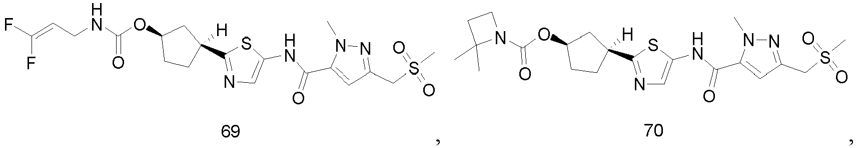

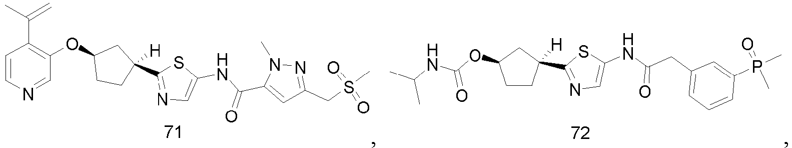

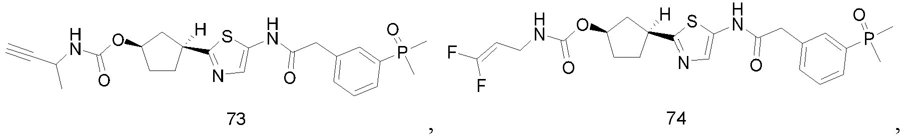

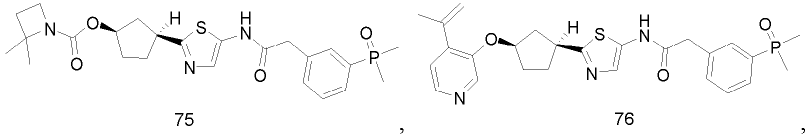

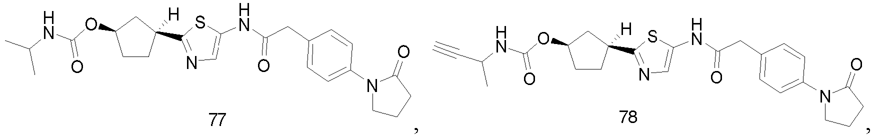

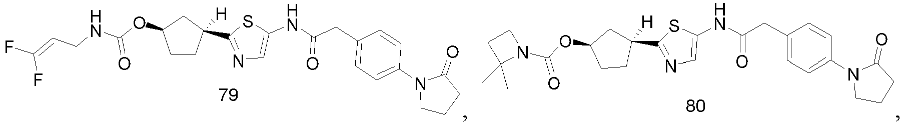

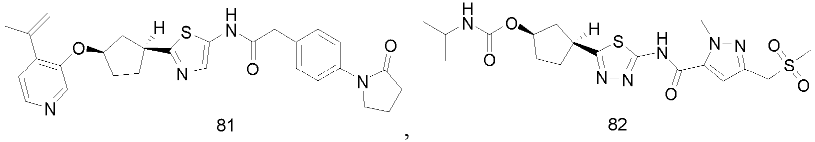

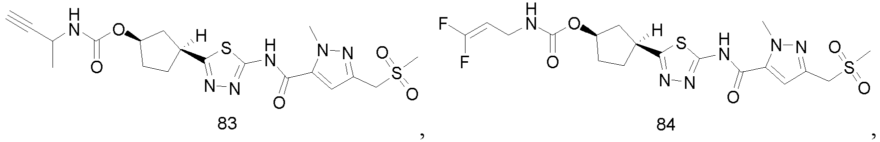

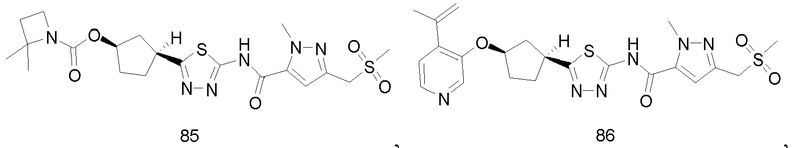

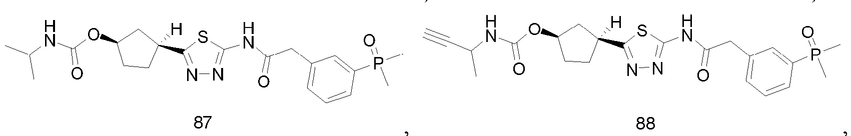

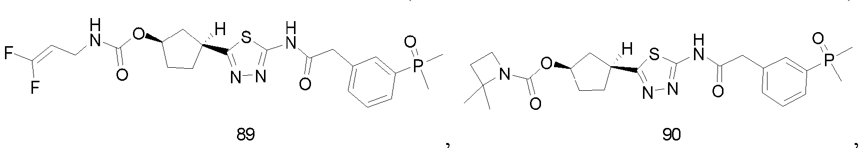

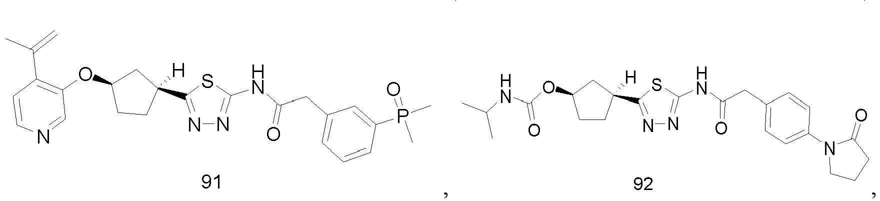

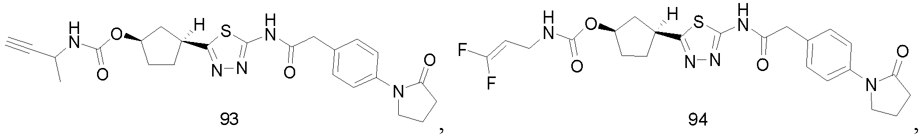

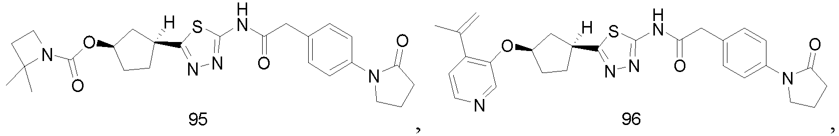

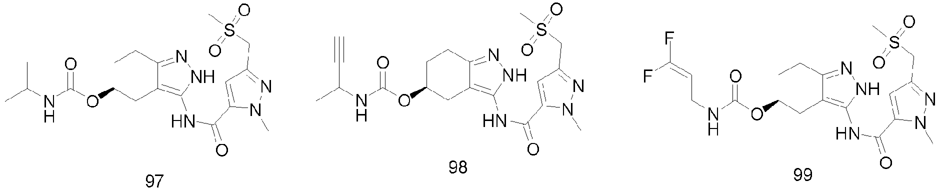

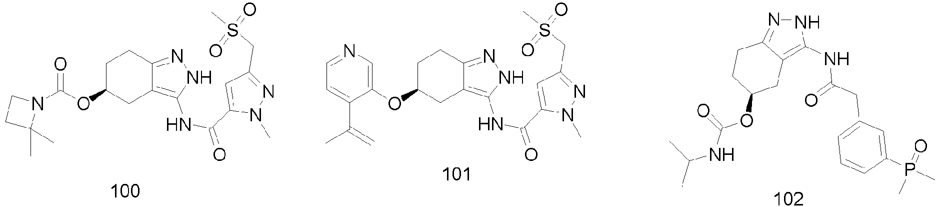

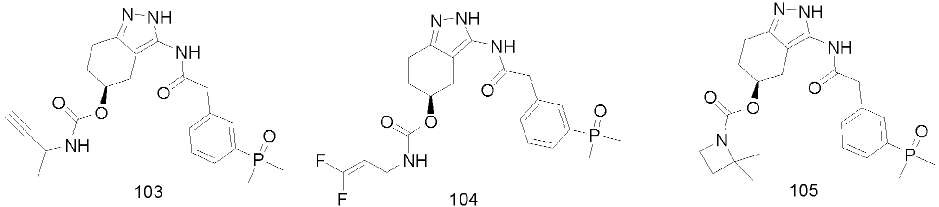

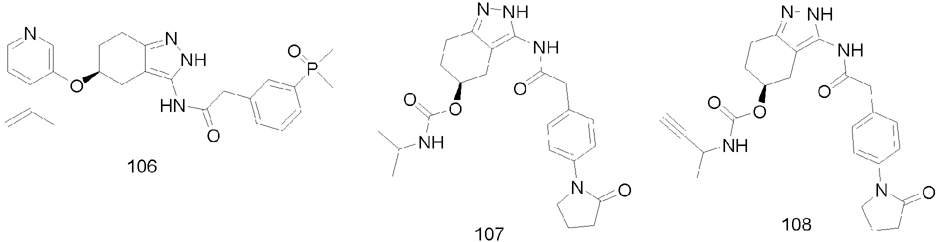

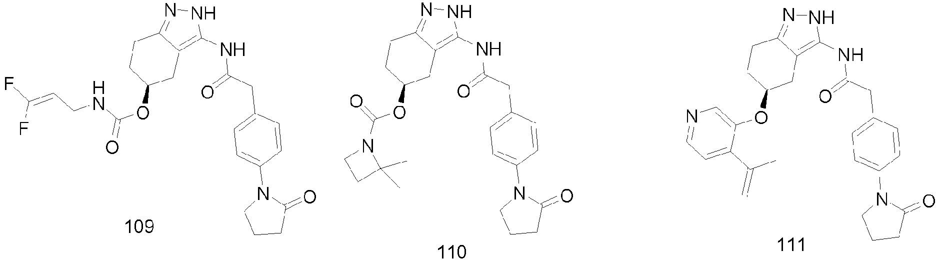

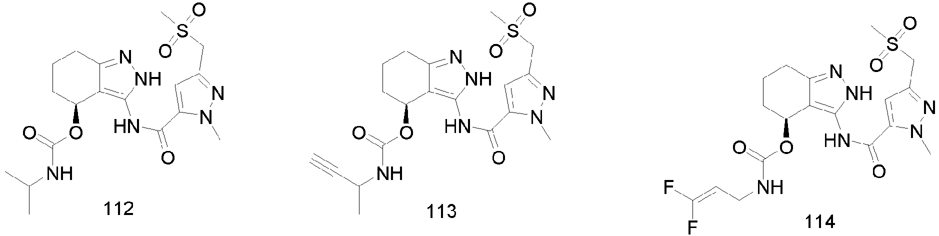

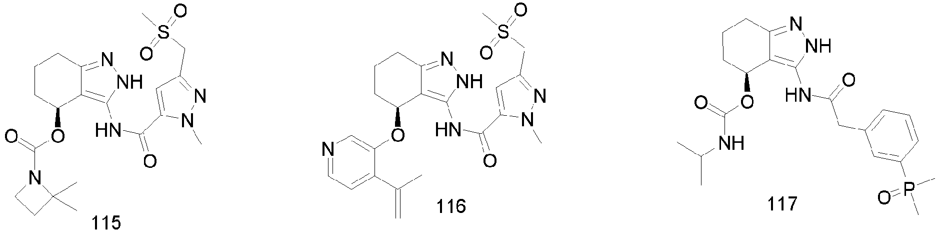

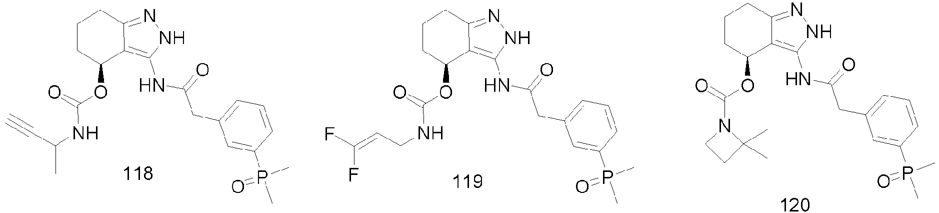

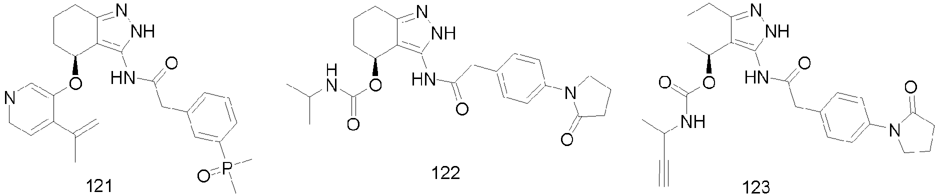

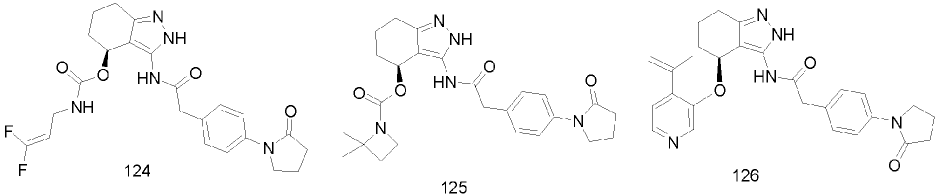

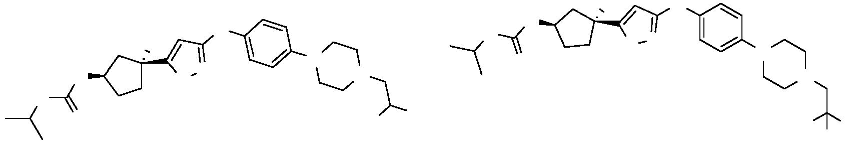

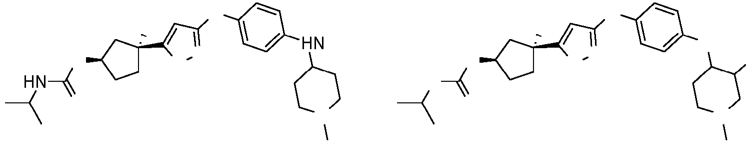

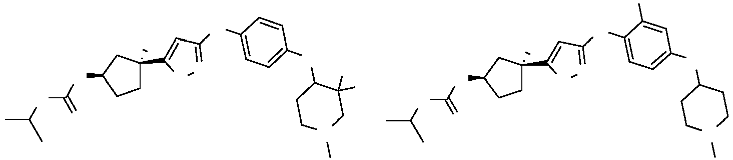

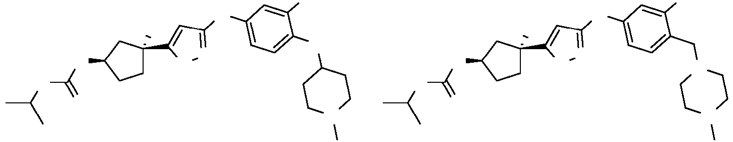

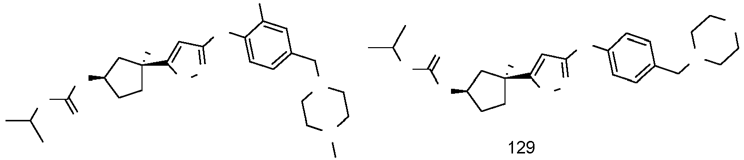

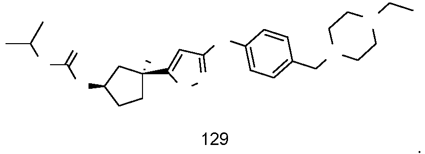

8. A pharmaceutical composition, comprising the compound or the pharmaceutically acceptable salt, the stereoisomer, or the deuteride thereof according to any one of claims 1-7 and a pharmaceutically acceptable carrier.

9. Use of the compound or the pharmaceutically acceptable salt, the stereoisomer, or the deuteride thereof according to any one of claims 1-7 or the pharmaceutical composition according to claim 7 in preparing a medicament.

10. The use according to claim 8, wherein the medicament is used for preventing or treating a related disease mediated by CDK2.

11. The use according to claim 8, wherein the medicament is used for preventing or treating abnormal cell growth in a subject.

12. The use according to claim 11, wherein the abnormal cell growth is a cancer selected from the group consisting of breast cancer, ovarian cancer, bladder cancer, uterine cancer, cervical cancer, prostate cancer, lung cancer, esophageal cancer, head and neck cancer, colorectal cancer, renal cancer, liver cancer, pancreatic cancer, gastric cancer, thyroid cancer, skin cancer, esophageal cancer, lymphoma, sarcoma, multiple myeloma, or a solid tumor.

## Amended claims

### Amended claims under Art. 19.1 PCT

1. A compound of formula (IIA) or a pharmaceutically acceptable salt, a stereoisomer, or a deuteride thereof: 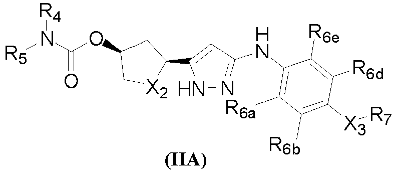 wherein,
X₂ is selected from the group consisting of CH₂ or O;
X₃ is selected from the group consisting of CH₂, NH, O, or a bond;
R₄ and R₅ are independently selected from the group consisting of H, C₁₋₆ alkyl, cyano-substituted C₁₋₆ alkyl, C₂₋₆ alkenyl, C₂₋₆ fluoroalkenyl, or C₂₋₆ alkynyl;
or R₄ and R₅, together with the N atom to which they are linked, form 4- to 6-membered heterocyclyl or 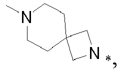 wherein the 4- to 6-membered heterocyclyl is optionally substituted with 1-2 methyl;
R₆ₐ, R_{6b}, R_{6d}, and R₆ₑ are each independently selected from the group consisting of H, halogen, C₁₋₆ alkyl, (CH₃)₂-P(O)-, or CH₃-S(O)₂-, wherein the C₁₋₆ alkyl is optionally substituted with C₁₋₆ alkoxy; R₇ is independently selected from the group consisting of 6- to 10-membered heterocyclyl, wherein the 6- to 10-membered heterocyclyl is optionally substituted with 1, 2, 3, 4, or 5 halogens, C₁₋₆ alkyl, or C₁₋₆ haloalkyl.

2. The compound or the pharmaceutically acceptable salt, the stereoisomer, or the deuteride thereof according to claim 1,
wherein,
R₄ and R₅ are independently selected from the group consisting of H, methyl, ethyl, n-propyl, isopropyl, n-butyl, isobutyl, tert-butyl, propionitril-2-yl, 2-methylpropionitril-2-yl, ethenyl, 1-propenyl, 2-propenyl, 3,3-difluoro-2-propenyl, ethynyl, 1-propynyl, 2-propynyl, n-but-1-yn-3-yl, or n-but-1-yn-4-yl;
or R₄ and R₅, together with the N atom to which they are linked, form azetidinyl, 2,2-dimethylazetidinyl, or 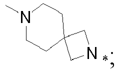
R₆ₐ, R_{6b}, R_{6d}, and R₆ₑ are each independently selected from the group consisting of H, F, Cl, Br, I, methyl, ethyl, n-propyl, isopropyl, n-butyl, isobutyl, tert-butyl, (CH₃)₂-P(O)-, or CH₃-S(O)₂-, wherein the methyl, ethyl, n-propyl, isopropyl, n-butyl, isobutyl, or tert-butyl is optionally substituted with methoxy, ethoxy, n-propoxy, or isopropoxy;
R₇ is independently selected from the group consisting of piperidinyl or piperazinyl, wherein the piperidinyl or piperazinyl is optionally substituted with 1, 2, 3, 4, or 5 F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

3. The compound or the pharmaceutically acceptable salt, the stereoisomer, or the deuteride thereof according to claim 1 or 2,
wherein,
R₇ is independently selected from the group consisting of 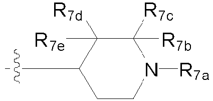 or 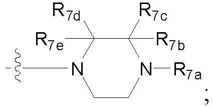 R₇ₐ, R_{7b}, R_{7c}, R_{7d}, and R₇ₑ are independently selected from the group consisting of H, F, Cl, Br, I, methyl, ethyl, n-propyl, isopropyl, fluoromethyl, difluoromethyl, trifluoromethyl, 2-fluoroethyl, 2,2-difluoroethyl, or 2,2,2-trifluoroethyl.

4. The compound or the pharmaceutically acceptable salt, the stereoisomer, or the deuteride thereof according to claim 3,
wherein,
X₂ is selected from the group consisting of CH₂; X₃ is selected from the group consisting of CH₂; R₄ is selected from the group consisting of H; R₅ is selected from the group consisting of C₁₋₆ alkyl, preferably isopropyl;
R₆ₐ, R_{6b}, R_{6d}, and R₆ₑ are each independently selected from the group consisting of H or halogen.

5. A compound of formula (II) or a pharmaceutically acceptable salt, a stereoisomer, or a deuteride thereof, wherein, 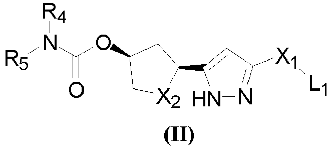
X₁ is selected from the group consisting of NH or O;
X₂ is selected from the group consisting of CH₂ or O;
L₁ is selected from the group consisting of 6- to 10-membered aryl, 6- to 10-membered heteroaryl, or 6- to 10-membered heterocyclyl, wherein the 6- to 10-membered aryl, 6- to 10-membered heteroaryl, or 6- to 10-membered heterocyclyl is optionally substituted with 1, 2, or 3 R₆;
R₄ and R₅ are independently selected from the group consisting of H, C₁₋₆ alkyl, cyano-substituted
C₁₋₆ alkyl, C₂₋₆ alkenyl, C₂₋₆ fluoroalkenyl, or C₂₋₆ alkynyl;
or R₄ and R₅, together with the N atom to which they are linked, form 4- to 6-membered heterocyclyl or 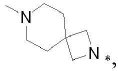 wherein the 4- to 6-membered heterocyclyl is optionally substituted with 1-2 methyl;
each R₆ is independently selected from the group consisting of H, halogen, C₁₋₆ alkyl, (CH₃)₂-P(O)-, CH₃-S(O)₂-, -CH₂R₇, -NHR₇, or 6- to 10-membered heterocyclyl, wherein the C₁₋₆ alkyl is optionally substituted with C₁₋₆ alkoxy, and the 6- to 10-membered heterocyclyl is optionally substituted with 1, 2, 3, 4, or 5 halogens, C₁₋₆ alkyl, or C₁₋₆ haloalkyl;
R₇ is independently selected from the group consisting of 6- to 10-membered heterocyclyl, wherein the 6- to 10-membered heterocyclyl is optionally substituted with 1, 2, 3, 4, or 5 halogens, C₁₋₆ alkyl, or C₁₋₆ haloalkyl.

6. A compound of formula (I) or a pharmaceutically acceptable salt, a stereoisomer, or a deuteride thereof, 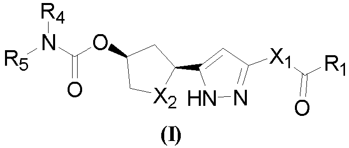 wherein,
X₁ is selected from the group consisting of NH or O;
X₂ is selected from the group consisting of CH₂ or O;
R₁ is selected from the group consisting of 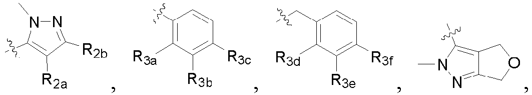 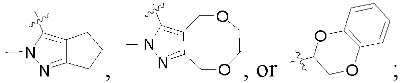
R₂ₐ is H;
R_{2b} is C₁₋₆ alkyl, wherein the C₁₋₆ alkyl is substituted with R_{2c}-S(O)₂- or R_{2c}-S(O)(NH)-, and R_{2c} is selected from the group consisting of H, C₁₋₆ alkyl, C₃₋₆ cycloalkyl, or amino;
R₃ₐ, R_{3b}, and R_{3c} are each independently selected from the group consisting of H, CH₃-S(O)₂-CH₂-, (CH₃)₂-P(O)-, or 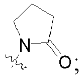
when R_{3d} and R_{3f} are each independently selected from the group consisting of H, R₃ₑ is independently selected from the group consisting of CH₃-S(O)₂-CH₂- or (CH₃)₂-P(O)-; or when R_{3d} and R₃ₑ are each independently selected from the group consisting of H, R_{3f} is independently selected from the group consisting of 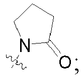
R₄ and R₅ are independently selected from the group consisting of H, C₁₋₆ alkyl, C₂₋₆ alkenyl, C₂₋₆ alkynyl, C₁₋₆ fluoroalkyl, C₂₋₆ fluoroalkenyl, C₂₋₆ fluoroalkynyl, or C₃₋₁₀ cycloalkyl, wherein the terminal C atom of each C₁₋₆ alkyl is optionally substituted with cyano, and each C₃₋₁₀ cycloalkyl is optionally substituted with difluoromethylene;
or R₄ and R₅, together with the N atom to which they are linked, form 4- to 6-membered heterocyclyl or 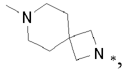 wherein the 4- to 6-membered heterocyclyl is optionally substituted with 1-2 methyl.

7. A compound of formula (I) or a pharmaceutically acceptable salt, a stereoisomer, or a deuteride thereof, 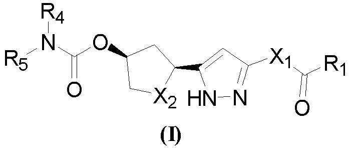 wherein,
X₁ is NH;
X₂ is selected from the group consisting of CH₂ or O;
R₁ is 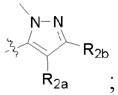
R₂ₐ and R_{2b} are each independently selected from the group consisting of H or methyl, wherein the methyl is optionally substituted with methoxy, ethoxy, n-propoxy, isopropoxy, CH₃-S(O)₂-, CH₃CH₂-S(O)₂-, cyclopropyl-S(O)₂-, NH₂-S(O)₂-, or CH₃-S(O)(NH)-;
R₄ is selected from the group consisting of H or methyl;
R₅ is selected from the group consisting of propionitril-2-yl, 2-methylpropionitril-2-yl, 3,3-difluoro-2-propenyl, n-but-1-yn-3-yl, difluoromethylenecyclohexyl, or adamantyl;
or R₄ and R₅, together with the N atom to which they are linked, form azetidinyl, 2,2-dimethylazetidinyl, or

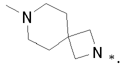

8. A compound as shown below or a pharmaceutically acceptable salt, a stereoisomer, or a deuteride thereof, wherein the compound is selected from the group consisting of:

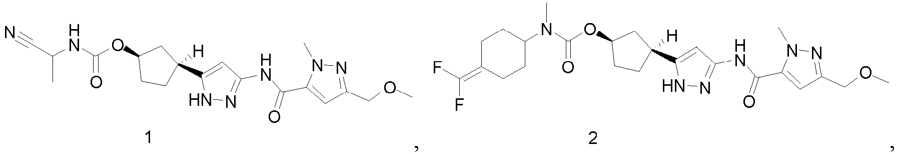

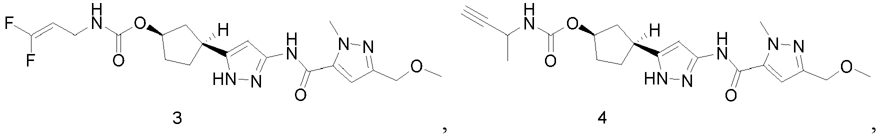

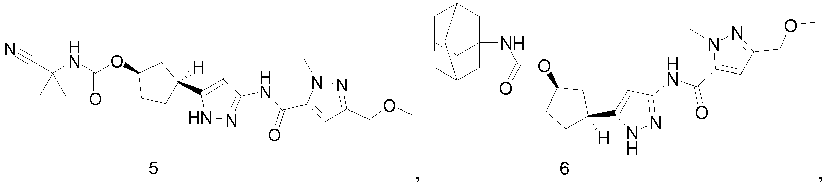

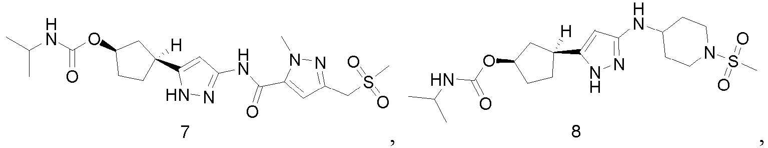

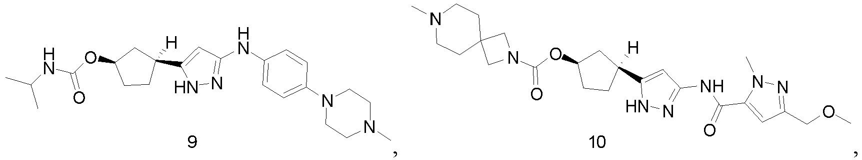

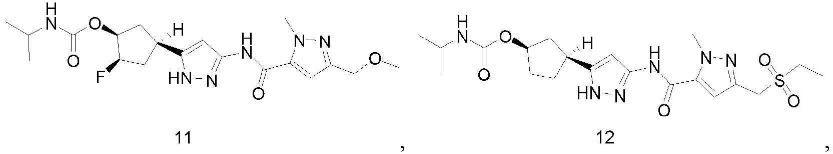

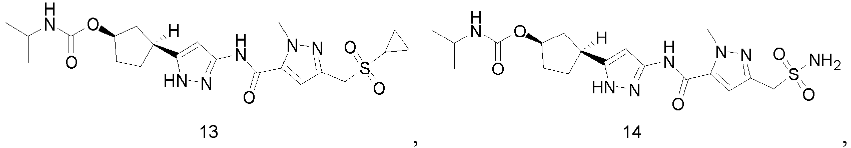

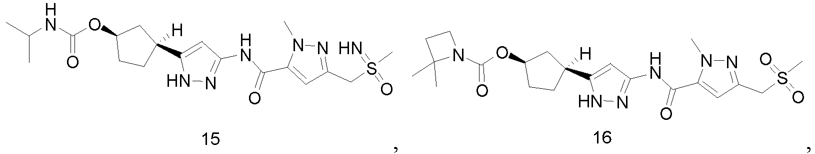

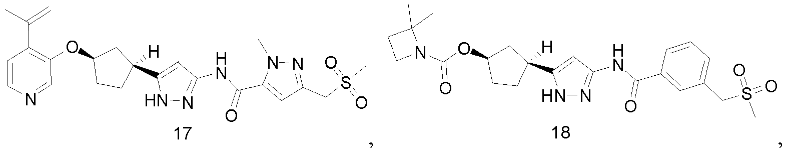

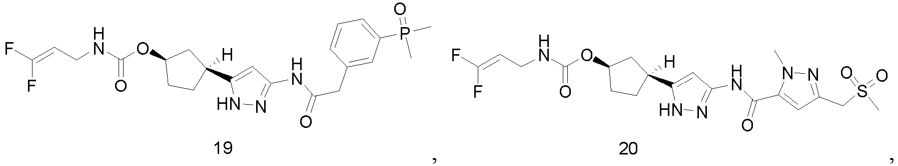

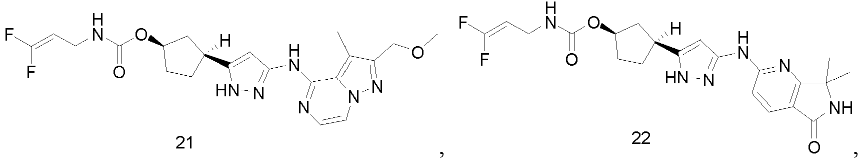

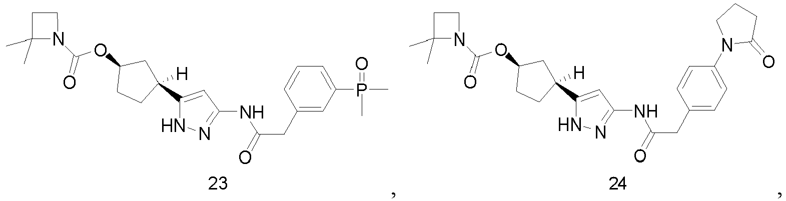

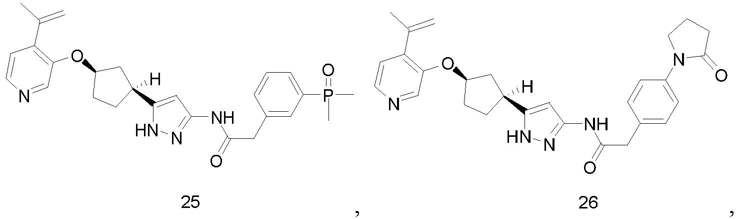

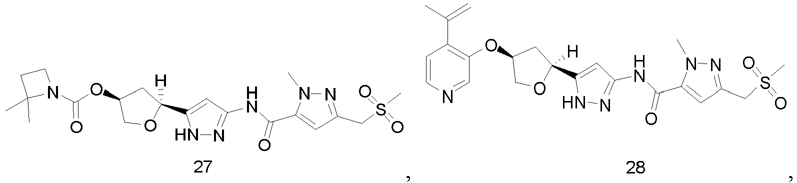

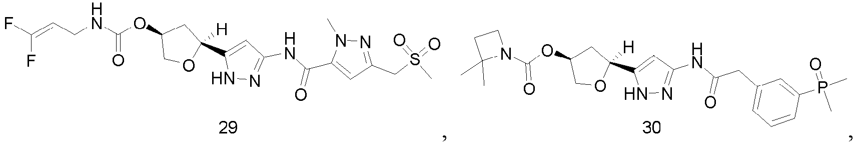

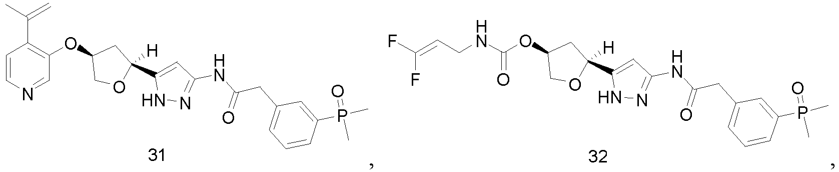

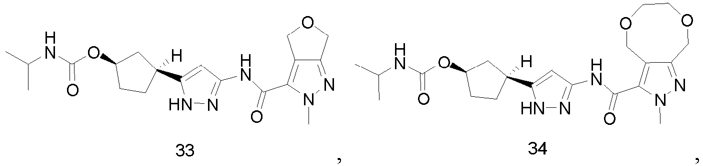

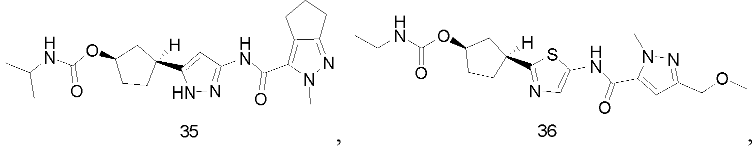

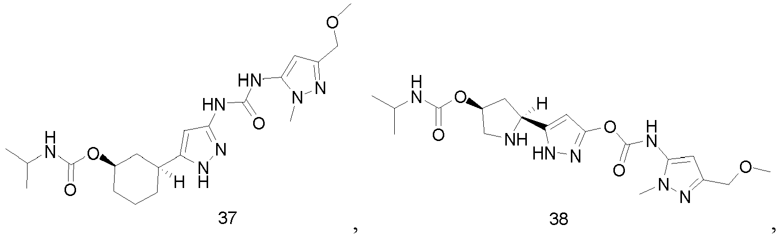

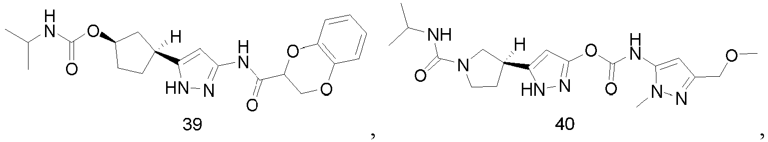

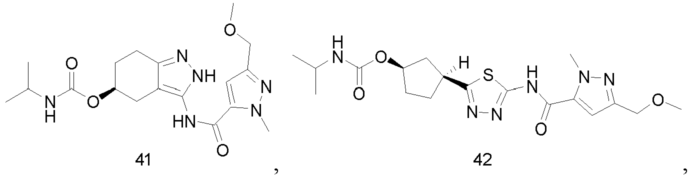

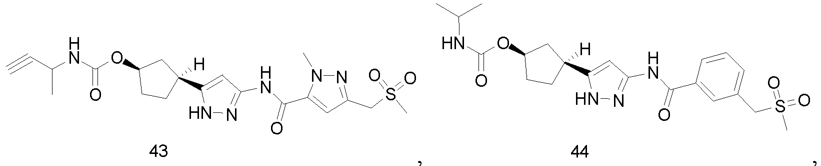

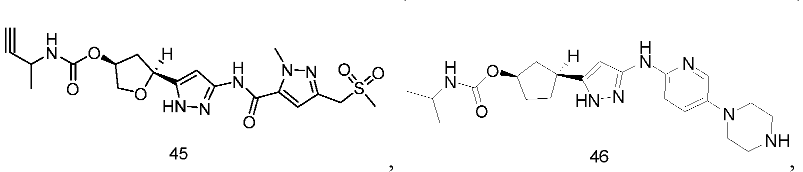

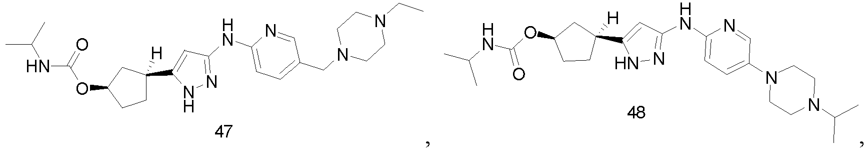

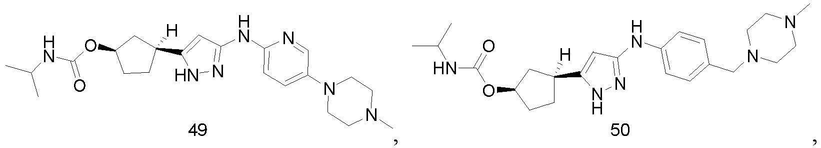

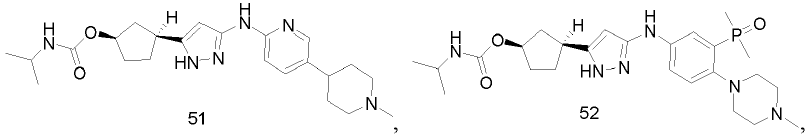

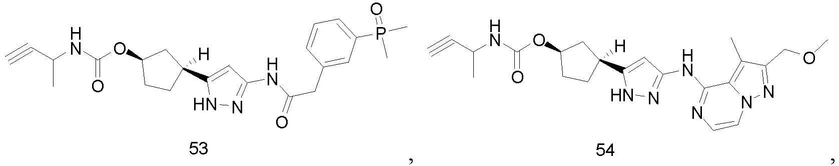

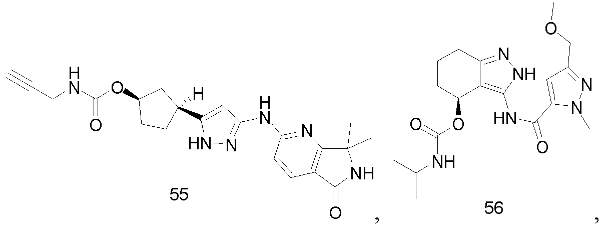

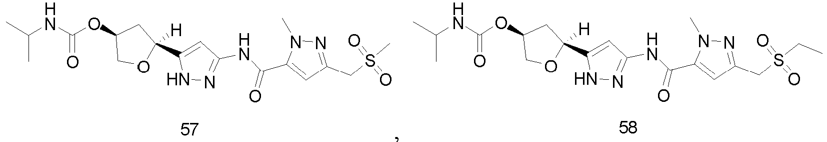

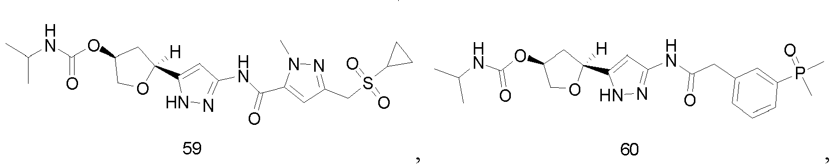

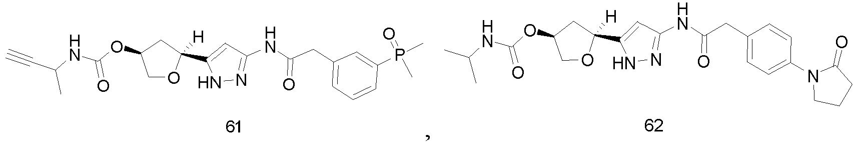

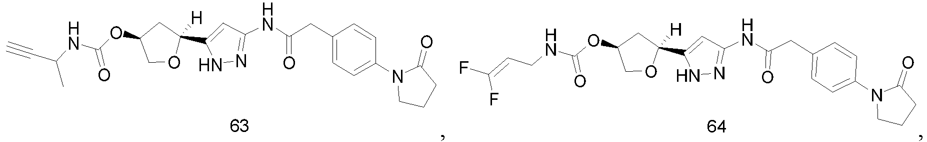

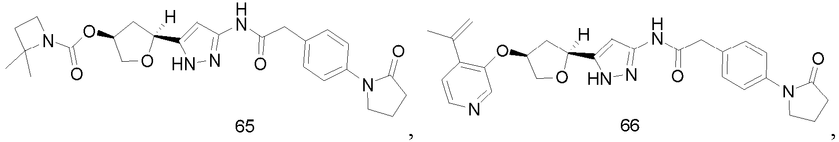

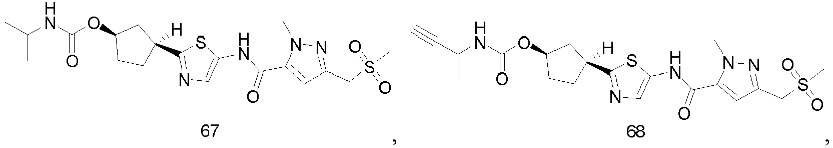

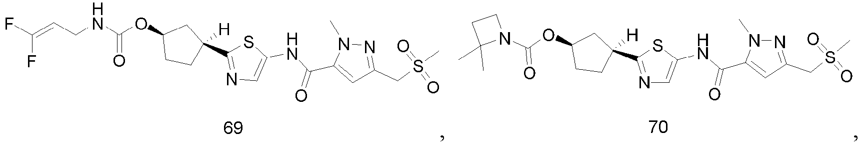

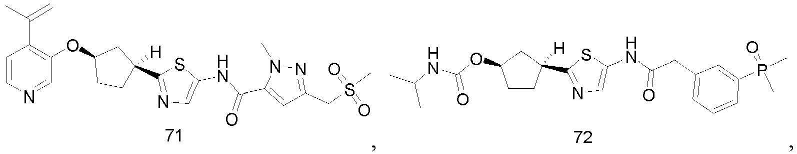

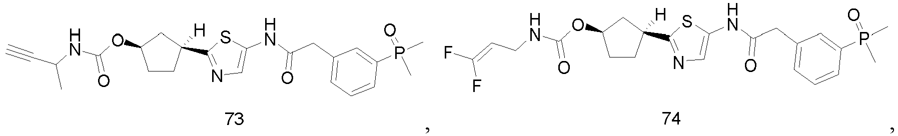

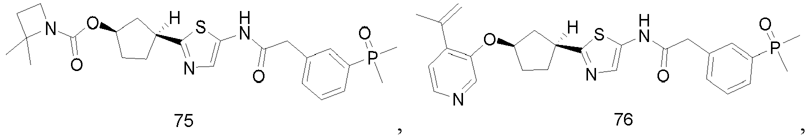

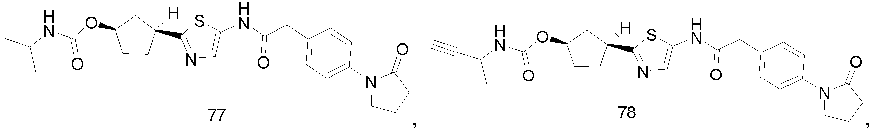

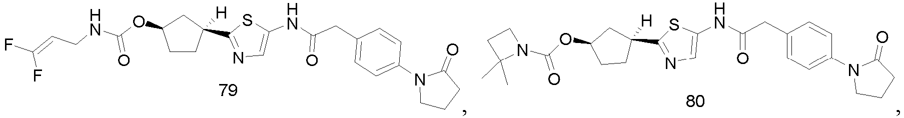

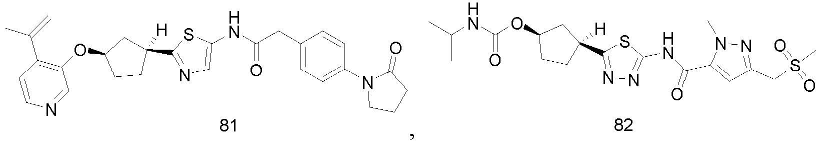

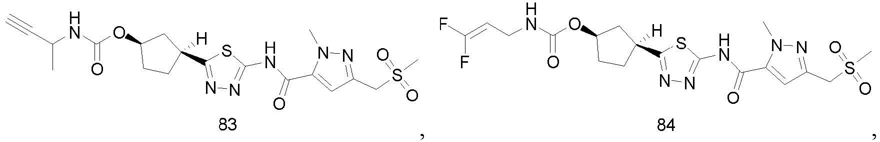

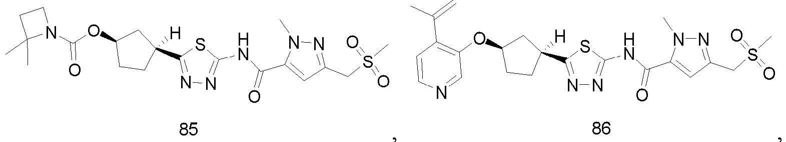

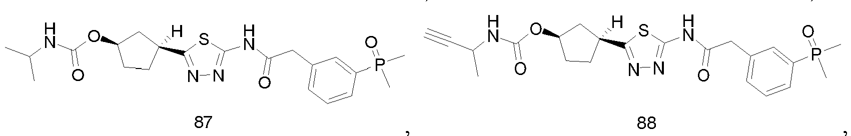

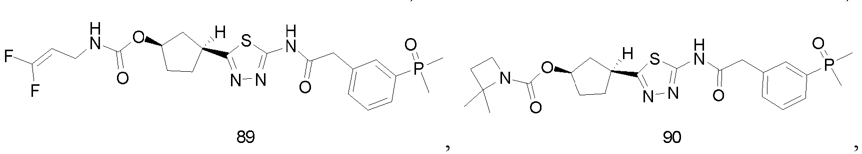

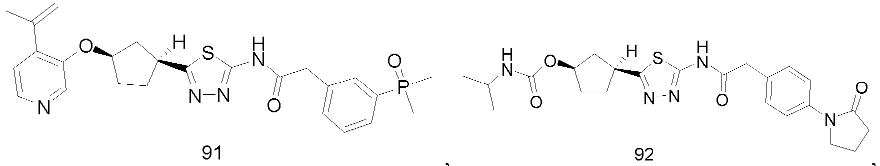

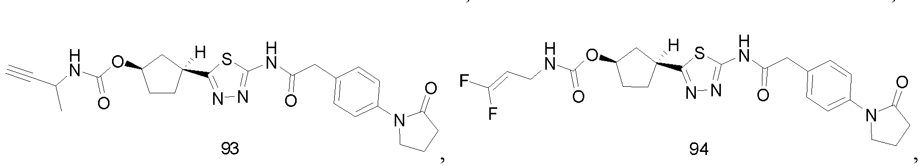

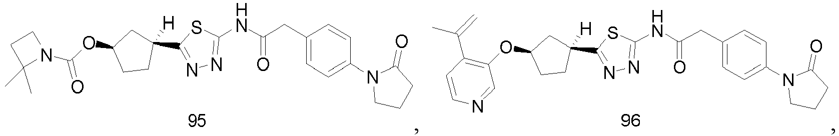

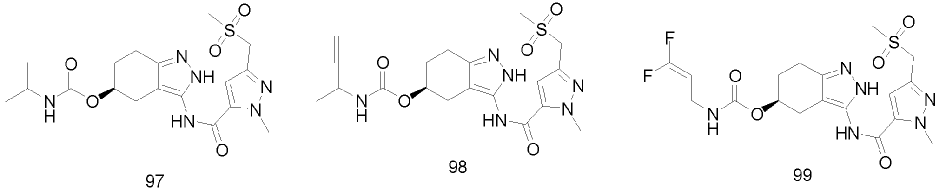

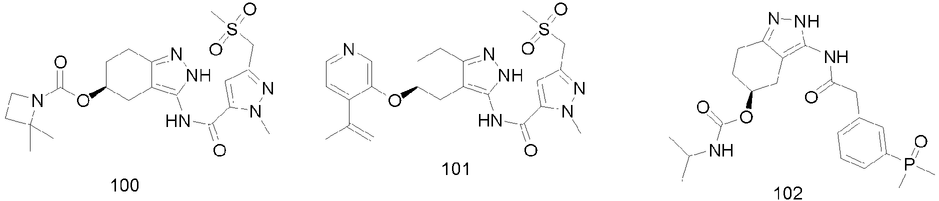

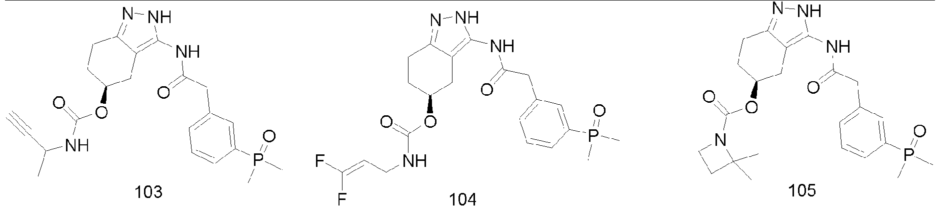

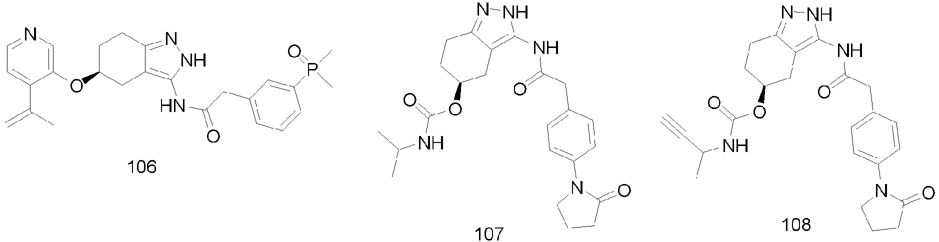

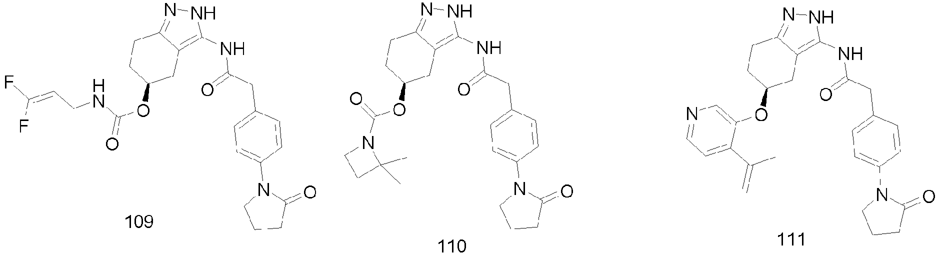

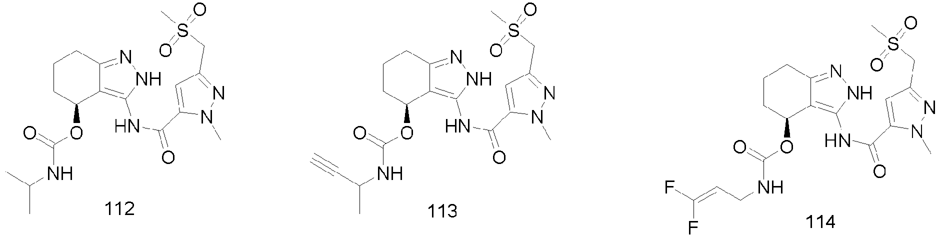

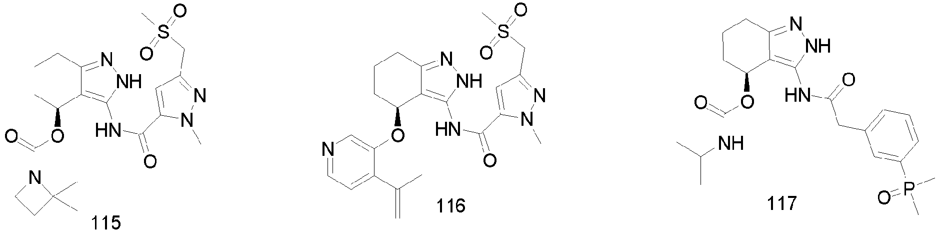

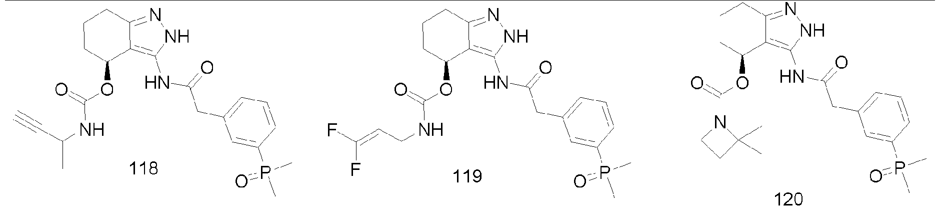

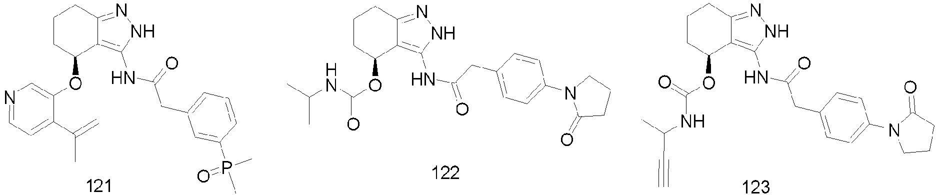

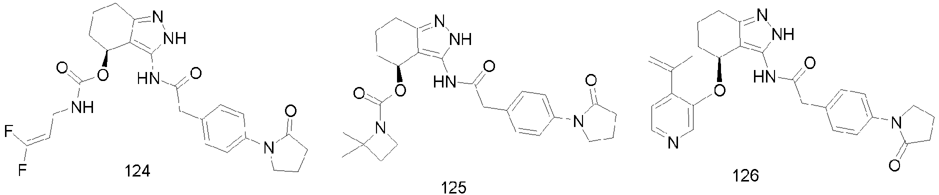

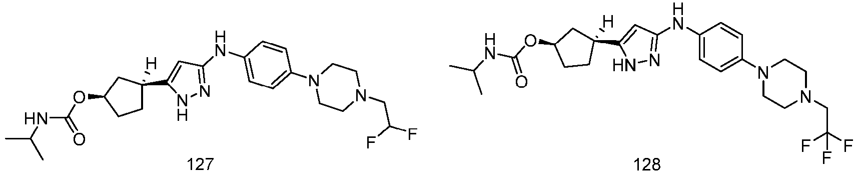

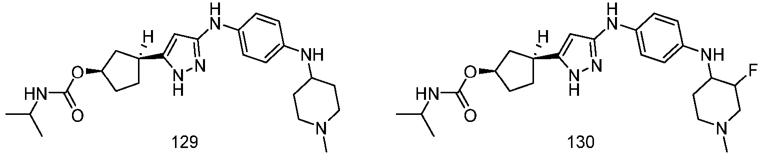

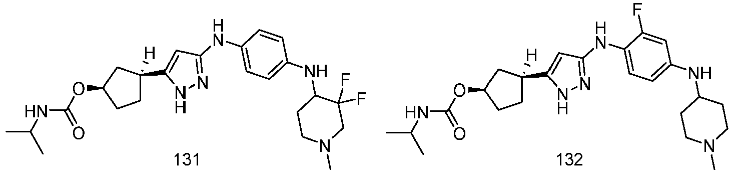

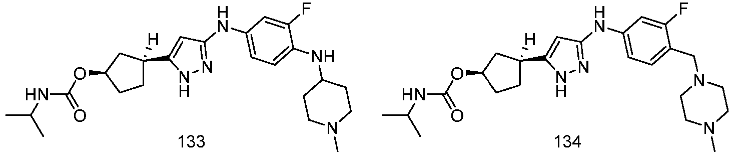

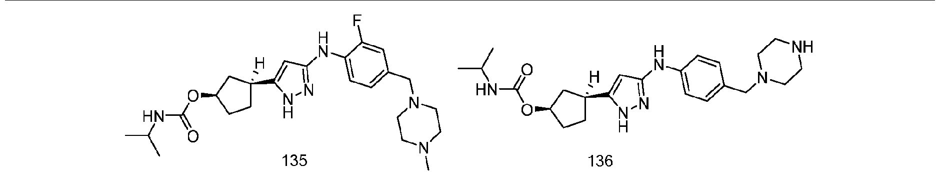

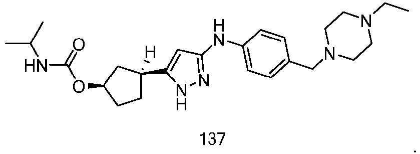

9. A pharmaceutical composition, comprising the compound or the pharmaceutically acceptable salt, the stereoisomer, or the deuteride thereof according to any one of claims 1-7 and a pharmaceutically acceptable carrier.

10. Use of the compound or the pharmaceutically acceptable salt, the stereoisomer, or the deuteride thereof according to any one of claims 1-7 or the pharmaceutical composition according to claim 7 in preparing a medicament.

11. The use according to claim 8, wherein the medicament is used for preventing or treating a related disease mediated by CDK2.

12. The use according to claim 8, wherein the medicament is used for preventing or treating abnormal cell growth in a subject.

13. The use according to claim 11, wherein the abnormal cell growth is a cancer selected from the group consisting of breast cancer, ovarian cancer, bladder cancer, uterine cancer, cervical cancer, prostate cancer, lung cancer, esophageal cancer, head and neck cancer, colorectal cancer, renal cancer, liver cancer, pancreatic cancer, gastric cancer, thyroid cancer, skin cancer, esophageal cancer, lymphoma, sarcoma, multiple myeloma, or a solid tumor.
